# EUROPEAN PATENT APPLICATION

(11) **EP 3 789 486 A1**
(43) Date of publication of application: **10.03.2021**
(21) Application number: 19797057.7
(22) Date of filing: 30.04.2019
(51) Int. Cl.: C12N 5/0783, C12N 15/12, C12N 15/62, A61K 35/17, A61P 35/00

(54) **IMMUNE EFFECTOR CELL AND USE THEREOF**

(30) Priority: 03.05.2018 CN 201810415367; 06.09.2018 CN 201811038855; 27.11.2018 CN 201811431644
(71) Applicant: Carsgen Therapeutics Co., Ltd., Shanghai 200231 (CN); Shanghai Cancer Institute, Shanghai 200032 (CN)
(72) Inventor: JIANG, Hua, Shanghai 200032 (CN); WANG, Huamao, Shanghai 200231 (CN); LI, Zonghai, Shanghai 200231 (CN)
(74) Representative: dompatent von Kreisler Selting Werner - Partnerschaft von Patent- und Rechtsanwälten mbB
(86) International application number: PCT/CN2019/085322
(87) International publication number: WO 2019/210863

(57) **Abstract**

A geneticall engineered cell. The cell expresses an exogenous receptor binding to an antigen, and expresses increased RUNX3 or exogenous RUNX3. Also provided are a use of the cell and a method for treating tumors.

## Description

### Technical field

The present invention belongs to the field of immunotherapy. In particular, the present invention relates to cells expressing an exogenous receptor binding to an antigen and expressing an increased level of RUNX3 or exogenous RUNX3, particularly immune effector cells.

### Background

Chimeric antigen receptor (CAR) is an artificial recombinant receptor, usually containing the antigen recognition domain of a monoclonal antibody located in the extracellular region, the transmembrane region, and the intracellular activation signaling domain of immune response cells.

However, due to the complexity of the microenvironment of organisms, especially solid tumors, drug candidates exhibiting excellent effects *in vitro* often fail to exhibit corresponding effects *in vivo.*

### Summary of the invention

The purpose of this disclosure is to provide a genetically engineered immune effector cell to increase the residence and killing ability of the immune effector cell in tumor tissues, thereby increasing the anti-tumor activity.

In one aspect, a genetically engineered cell is provided herein, wherein the cell expresses an exogenous receptor binding to an antigen and expresses an increased level of RUNX3 or exogenous RUNX3. In some embodiments, the cell is an immune effector cell. In some embodiments, the immune effector cell is a T cell. In some embodiments, the RUNX3 is a full-length human RUNX3 or a fragment of human RUNX3 having the same function as the full-length human RUNX3. In some embodiments, the RUNX3 is at least 90% identical to the sequence as shown in SEQ ID NO: 20. In some embodiments, the RUNX3 is constitutively expressed. In some embodiments, the RUNX3 is inducibly expressed. In some embodiments, the antigen is a tumor antigen or a pathogen antigen, preferably a tumor antigen. In some embodiments, the tumor antigen is a solid tumor antigen. In some embodiments, the tumor antigen is GPC3 or claudin 18.2. In some embodiments, the receptor is a chimeric receptor selected from the group consisting of chimeric antigen receptor (CAR), modified T cell (antigen) receptor (TCR), T cell fusion protein (TFP), T Cell antigen coupler (TAC) or a combination thereof. In some embodiments, the receptor is a chimeric antigen receptor. In some embodiments, the intracellular domain of the chimeric antigen receptor comprises the intracellular costimulatory signaling domain of CD137. In some embodiments, the extracellular domain of the receptor has an amino acid sequence that is at least 80%, 85%, 90%, 95%, 96%, 97%, 98%, or 99% identical to SEQ ID NO: 42 or SEQ ID NO: 22. In some embodiments, the intracellular domain of the receptor contains has an amino acid sequence that is at least 80%, 85%, 90%, 95%, 96%, 97%, 98%, or 99% identical to SEQ ID NO: 47. In some embodiments, the intracellular domain of the receptor also has an amino acid sequence that is at least 90% identical to SEQ ID NO: 46 or SEQ ID NO: 49, or comprises an amino acid sequence that is at least 90% identical to SEQ ID NO: 46 or SEQ ID NO: 49. In some embodiments, the cell comprises a nucleic acid sequence that is at least 80%, 85%, 90%, 95%, 96%, 97%, 98%, or 99% identical to SEQ ID NO: 17, 19, 53, 54, 55, or 56. In some embodiments, the cell also expresses an exogenous cytokine receptor-binding protein, or an exogenous cytokine or a polypeptide thereof. In some embodiments, the cell also expresses an exogenous cytokine. In some embodiments, the exogenous cytokine receptor-binding protein can specifically bind to the corresponding cytokine receptor and enhance activities of the receptor. In some embodiments, the exogenous cytokine or polypeptide thereof can specifically bind to the corresponding cytokine receptor and enhance activities of the receptor. In some embodiments, the cytokine has an amino acid sequence that is at least 80%, 85%, 90%, 95%, 96%, 97%, 98%, or 99% identical to the sequence as shown in SEQ ID NO: 39, 41 or 36. In some embodiments, the expression level of RUNX3 is increased by at least 10%, 20%, 30%, 40%, 50%, 60%, 70%, 80%, 90%, or 100% as compared with the wild type. In some embodiments, the increase in the expression level of the RUNX3 as compared with the wild type is sufficient to increase the retention of the cell in non-lymphatic sites. In some embodiments, the cytokine is constitutively expressed. In some embodiments, the cytokine is inducibly expressed. In some embodiments, the cell expresses an exogenous RUNX3. In some embodiments, the receptor and/or RUNX3 are expressed by using a viral vector; and preferably, the viral vector includes: a lentiviral vector, retroviral vector or adenoviral vector.

In another aspect of the present invention, an expression construct is provided herein, wherein the expression construct comprises sequentially connected expression cassette 1 of an antigen-binding receptor and expression cassette 2 of RUNX3, wherein the expression cassettes are optionally connected by tandem fragments selected from the group consisting of F2A, PA2, T2A, and E2A. In some embodiments, the expression cassette of the antigen-binding receptor comprises a nucleic acid sequence encoding the sequence as shown in SEQ ID NO: 57, 58, 59, 60, 61, or 62, and the expression cassette of RUNX3 has a nucleic acid sequence encoding the sequence as shown in SEQ ID NO: 20. In some embodiments, the expression construct further comprises expression cassette 3 comprising a nucleic acid sequence encoding a cytokine, and the sequence of the cytokine is an amino acid sequence which is at least 80%, 85%, 90%, 95%, 96%, 97%, 98%, or 99% identical to the sequence as shown in SEQ ID NO: 36, 39, or 41.

In another aspect, an expression vector is provided herein, comprising the expression construct described herein.

In another aspect, a virus is provided herein, comprising the expression vector described herein.

In another aspect, a method is provided herein for increasing the viability of an immune effector cell expressing a chimeric receptor in an individual by increasing the expression level of RUNX3 in the immune effector cell. In another aspect, a method is provided herein for increasing the viability of an immune effector cell expressing a chimeric receptor in an individual, wherein the expression level of RUNX3 in the immunity effector cell is increased by expressing exogenous RUNX3 in the immune effector cell. In some embodiments, the RUNX3 is a full-length human RUNX3 or a fragment of human RUNX3 having the same function as the full-length human RUNX3. In some embodiments, the RUNX3 has at least 90% identity with the sequence as shown in SEQ ID NO: 20. In some embodiments, the RUNX3 is constitutively expressed. In some embodiments, the RUNX3 is inducibly expressed. In some embodiments, the immune effector cell also co-expresses a cytokine. In some embodiments, the individual is also administered with a cytokine. In some embodiments, the immune effector cell is a T cell. In some embodiments, the chimeric receptor is a chimeric antigen receptor. In some embodiments, the individual is also administered with a chemotherapeutic drug.

In another aspect, the use of the cell, expression construct or virus described herein is provided herein for preparing a medicament for inhibiting a tumor or inhibiting a pathogen.

In another aspect, a pharmaceutical composition is provided herein for inhibiting a tumor or inhibiting a pathogen, the pharmaceutical composition comprising the cell described herein and a pharmaceutically acceptable carrier or excipient.

In another aspect, a kit or a pharmaceutical kit for treating a tumor or pathogen infection, the kit comprising the cell or pharmaceutical composition described herein.

In another aspect, the use of a immune effector cell and a chemotherapeutic drug is provided herein for preparing a medicament for treating a tumor, wherein the immune effector cell expresses an increased level of RUNX3 or exogenous RUNX3 and a chimeric antigen receptor targeting a tumor antigen. In some embodiments, the chemotherapeutic agent includes sorafenib. In some embodiments, the tumor antigen is GPC3 or claudin 18.2. In some embodiments, the immune effector cell is a T cell.

In another aspect, a method is provided herein for treating a tumor in a patient in need thereof, comprising providing the patient with the cell as described herein.

It should be understood that, within the scope of the disclosure herein, the above technical features described herein and the technical features specifically described in the following (such as the examples) can be combined with each other, and various specific combinations of these technical features should be regarded as being specifically disclosed.

### Description of drawings

Figure 1A is a map of plasmid PRRLSIN-hu9F2-28Z;
Figure 1B is a map of plasmid PRRLSIN-hu9F2-BBZ;
Figure 1C is a map of plasmid MSCV-hu8E5-2I-mBBZ;
Figure 1D is a map of plasmid pUC57-RUNX3-V2;
Figure 1E is a map of plasmid pRRLSIN-hu9F2-28Z-F2A-huRUNX3;
Figure IF is a map of plasmid pRRLSIN-hu9F2-BBZ-F2A-huRUNX3;
Figure 1G is a map of plasmid MSCV-hu8E5-2I-mBBZ-F2A-mRunX3;
Figure 1H is a map of plasmid pRRLSIN-hu9F2-BBZ-F2A-huRUNX3-NFAT-IL15;
Figure 1I is a map of plasmid pRRLSIN-hu9F2-BBZ-F2A-huRUNX3-NFAT-IL18;
Figure 1J is a map of plasmid pRRLSIN-hu9F2-BBZ-F2A-huRUNX3-NFAT-IL21;
Figure 1K is a map of plasmid pRRLSIN-hu9F2-BBZ(CD28TM)-F2A-RUNX3;
Figure 2 shows the phenotype detection of CAR-T cells *in vitro;*
Figure 3A shows the results of *in vitro* killing toxicity test targeting GPC3 positive cells of CAR T cells expressing RUNX3; and Figure 3B shows the results of *in vitro* killing toxicity test targeting GPC3 positive cells of CAR T cells expressing RUNX3 and a cytokine;
Figure 4 shows the results of *in vitro* killing toxicity test of CAR T cells expressing RUNX3 and targeting claudin 18.2 positive cells;
Figure 5 shows the *in vivo* treatment experiment of CAR T cells expressing RUNX3 on the small-load subcutaneous xenograft tumor model of B-NDG mice bearing PLC/PRF/5 hepatocarcinoma cells: Figure 5A shows the test results of the volume of the transplanted tumor, Figure 5B shows the test results of the weight of the transplanted tumor; and Figure 5C shows the test results of the weight of the mouse;
Figure 6 shows the *in vivo* treatment experiment of CAR T cells expressing RUNX3 on the heavy-load subcutaneous xenograft tumor model of NPG mice bearing PLC/PRF/5 hepatocarcinoma cells: Figure 6A shows the test results of the volume of the transplanted tumor, Figure 6B shows the test results of the weight of the transplanted tumor; and Figure 6C shows the test results of the weight of the mouse;
Figure 7 shows the anti-tumor treatment experiment of CAR T cells expressing a cytokine in combination with RUNX3 on the subcutaneously transplanted tumor of GPC3-positive cells: Figure 7A shows the test results of the volume of the transplanted tumor, Figure 7B shows the test results of the weight of the transplanted tumor; and Figure 7C shows the test results of the weight of the mouse;
Figure 8 shows the *in vivo* treatment experiment of CAR T cells expressing RUNX3 on the subcutaneously transplanted tumor model of mice bearing PANC02-A2 pancreatic cancer cells;
Figure 9 shows the *in vivo* treatment experiment of CAR T cells expressing RUNX3 in combination with sorafenib on the subcutaneously transplanted tumor model of mice bearing PLC/PRF/5 liver cancer cells: Figure 9A shows the test results of the volume of the transplanted tumor, Figure 9B shows the test results of the weight of the transplanted tumor, and Figure 9C shows the test results of the weight of the mouse;
Figure 10 shows the detection results of CAR T cells secreting cytokines IL-2, TNF-α, and IFN-γ;
Figure 11 shows changes in the tumor volume of Hepa1-6-GPC3 subcutaneously transplanted tumor;
Figure 12 shows the results from comparing the weight of subcutaneously transplanted Hepa1-6-GPC3 tumors after administering different CAR-T cells;
Figure 13 shows the results of changes in the body weight of mice during the treatment of subcutaneously transplanted Hepa1-6-GPC3 tumors by different CAR-T cells;
Figure 14 shows the comparison of expression levels of RUNX3 in different immune effector cells.

### Modes for carrying out the invention

Various aspects described herein can exist in a range format. It should be understood that the description in range format is only for convenience and brevity, and should not be regarded as an unchangeable limitation on the scope described herein. Therefore, the description of a range should be considered as specifically disclosing all possible subranges and individual values within the range. For example, the description of a range, such as from 1 to 6, should be considered as specifically disclosing subranges, such as 1 to 3, 1 to 4, 1 to 5, 2 to 4, 2 to 6, 3 to 6, etc., and individual values within the range, such as 1, 2, 2.7, 3, 4, 5, 5.3, and 6. For another example, a range, such as 95-99% identity, includes a range with 95%, 96%, 97%, 98%, or 99% identity, and includes a sub-range, such as 96-99%, 96-98%, 96-97%, 97-99%, 97-98% and 98-99% identity. This applies regardless of the width of the range.

Based on the present disclosure, a skilled person should understand that many changes or modifications can be made in the disclosed specific embodiments and the same or similar results can still be obtained without departing from the spirit and scope described herein. The scope of the present invention is not limited to the specific embodiments described herein (which are only intended to exemplify various aspects described herein), and it should be considered that functionally equivalent methods and components are still included within the stated range described herein.

Unless specifically defined, all technical and scientific terms used herein have meanings commonly understood by a skilled person in the field of gene therapy, biochemistry, genetics, and molecular biology. All methods and materials similar or equivalent to those described herein can be used in the practice or tests described herein. These techniques, such as methods and materials are described in literature, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al. , 2001, Cold Spring Harbor, New York: Cold Spring Harbor Laboratory Press); Oligonucleotide Synthesis (MJ Gaited., 1984); Mullis et al. US Pat. No. 4,683,195; Nucleic Acid Hybridization (BD Harries & SJ Higginseds. 1984); Transcription And Translation (BD Hames & SJ Higginseds. 1984); Culture Of Animal Cells (RI Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), especially Vols. 154 and 155 (Wuetal. eds.) and Vol.185, "Gene Expression Technology" (D. Goeddel, ed.); Gene Transfer Vectors For Mammalian Cells (JH Miller and MP Caloseds., 1987, Cold Spring Harbor Laboratory); Immunochemical Methods In Cell And Molecular Biology (M ayer and Walker, eds., Academic Press, London, 1987); Hand book Of Experimental Immunology, Volume I-IV (DM Weir and CC Blackwell, eds., 1986); and Manipulating the Mouse Embryo (Cold Spring Harbor Laboratory Press, Cold Spring Harbor, NY, 1986).

All publications, patent applications, patents and other references mentioned herein are incorporated herein by reference in their entirety. In case of conflict, the present specification shall prevail. In addition, unless otherwise specified, the materials, methods, and examples listed in the present specification are only illustrative and not intended to be limiting.

As used herein, the term "engineered" and other grammatical forms thereof can refer to one or more changes in nucleic acid, such as a nucleic acid within the genome of an organism. The term "engineered" can refer to changes, additions and/or deletions of genes. Engineered cells can also refer to cells with added, deleted and/or changed genes.

The term "genetically engineered cell" as used herein refers to a cell modified by means of genetic engineering. In some embodiments, the cell is an immune effector cell. In some embodiments, the cell is a T cell. In some embodiments, the genetically engineered cell described herein refers to a cell expressing an exogenous receptor that specifically binds to a target antigen. In some embodiments, the genetically engineered cell described herein refers to a cell that expresses an exogenous receptor specifically binding to a target antigen and expresses an increased level of RUNX3 or exogenous RUNX3. In some embodiments, the genetically engineered cell described herein may also be a T cell co-expressing a chimeric antigen receptor that specifically binds to a tumor antigen, an elevated level of RUNX3, or exogenous RUNX3 and cytokines (such as IL-15. IL-18, IL-21, etc.).

The term "immune effector cell" refers to a cell participating in an immune response and producing immune effects, such as a T cell, B cell, natural killer (NK) cells natural killer T (NKT) cell, mast cell, and bone marrow-derived phagocyte. In some embodiments, the immune effector cell is a T cell, NK cell, NKT cell. In some embodiments, the T cell can be an autologous T cell, xenogeneic T cell, or allogeneic T cell. In some specific embodiments, the NK cell may be an allogeneic NK cell.

As used herein, the term "immune effector function" refers to the function or response of an immune effector cell, such as inducing, enhancing, or regulating the activation of the immune system and the generation of immune responses.

RUNX3 (Runt-related transcription factor 3), also known as osteogenic-related transcription factor 3, comprises natural full-length RUNX3 and fragments of RUNX3 having RUNX3 function. The natural full-length RUNX3 gene is located on chromosome 1p36, 67 kb in a full length, and comprises two promoters P1 and P2 and six exons. The gene has two large conserved CpG islands, one is located at the start of exon 6, and the other is located near exon 2.

In some embodiments, the genetically engineered cell described herein expresses an increased level of RUNX3. In some embodiments, the increased expression level is relative to wild type. For this purpose, "wild-type" refers to a cell that has not been subject to the measures described in the present invention to increase RUNX3. It is worth noting that the wild-type is not limited to a naturally-occurring cell, such as an immune effector cell in an individual that has not been genetically engineered, but it can also be, for example, a cell that has been genetically engineered. For example, in some embodiments, the wild-type cell is a cell that expresses an exogenous receptor binding to an antigen. In some embodiments, the wild-type cell is a CAR T cell. In some embodiments, the wild-type cell is an immune effector cell in an individual's PBMCs. In some embodiments, the expression level of RUNX3 is increased by regulating the upstream genes of RUNX3.

In some embodiments, the expression level of RUNX3 is increased by transferring exogenous RUNX3 gene into the immunoengineered cells. In some embodiments, the expression level of RUNX3 is increased by at least 10%, 20%, 30%, 40%, 50%, 60%, 70%, 80%, 90%, or 100% relative to wild type. In some embodiments, the increase in the expression level of RUNX3 relative to the wild type is sufficient to increase the retention of the cells in non-lymphatic sites. In some embodiments, the increase in the expression level of RUNX3 relative to the wild type is sufficient to increase the retention of the cells in the tumor tissue. In some embodiments, when administered to an individual, the increase in the expression level of RUNX3 relative to wild-type is sufficient to increase the retention of the cells in non-lymphatic sites of the individual. In some embodiments, when administered to an individual, the increase in the expression level of RUNX3 relative to wild-type is sufficient to increase the retention of the cells in the tumor tissue of the individual. In some embodiments, when administered to an individual, the increase in the expression level of RUNX3 relative to wild-type is sufficient to increase inhibitory effects of the cells on tumor growth in the individual. In some embodiments, when administered to an individual, the increase in the expression level of RUNX3 relative to wild-type is sufficient to increase killing effects of the cells on tumor cells in the individual.

In some embodiments, a method is provided herein for increasing or improving the viability of an immune effector cell expressing a chimeric receptor in an individual, comprising administering the immune effector cell expressing the chimeric receptor in combination with exogenous RUNX3 to the individual. In some embodiments, when administered to an individual, the amount of exogenous RUNX3 is sufficient to increase the retention of the cell in a non-lymphatic site of the individual. In some embodiments, when administered to an individual, the amount of exogenous RUNX3 is sufficient to increase the retention of the cell in the tumor tissue of the individual. In some embodiments, when administered to an individual, the amount of exogenous RUNX3 is sufficient to increase inhibitory effects of the cell on tumor growth in the individual. In some embodiments, when administered to an individual, the amount of exogenous RUNX3 is sufficient to increase killing effects of the cell on tumor cells in the individual.

In some embodiments, a method is provided herein for treating cancer, comprising administering an immune effector cell expressing a chimeric receptor in combination with exogenous RUNX3 to an individual in need thereof. In some embodiments, when administered to an individual, the amount of exogenous RUNX3 is sufficient to increase the retention of the cell in a non-lymphatic site of the individual. In some embodiments, when administered to an individual, the amount of exogenous RUNX3 is sufficient to increase the retention of the cell in the tumor tissue of the individual. In some embodiments, when administered to an individual, the amount of exogenous RUNX3 is sufficient to increase inhibitory effects of the cell on tumor growth in the individual. In some embodiments, when administered to an individual, the amount of exogenous RUNX3 is sufficient to increase killing effects of the cell on tumor cells in the individual.

"Consitutive expression", also known as continuous expression, refers to the continuous expression of genes in cells under almost all physiological conditions. The term "inducible expression" refers to the expression under certain conditions, such as when T cells bind to an antigen.

The terms "therapeutically effective amount" and "effective amount" are used interchangeably herein and refer to the amount of a compound, preparation, substance, or composition which is effective to achieve specific biological results, such as but not limited to an amount or dosage sufficient to promote T cell responses. When indicating "immunologically effective amount", "anti-tumor effective amount", "tumor-inhibiting effective amount" or "therapeutically effective amount", the precise administration dose of the immune effector cells or therapeutic agents described herein can be determined by a physician in consideration of the individual's age, weight, tumor size, degree of metastasis, and the condition of the patient (subject). An effective amount of immune effector cells refers to, but is not limited to, the number of immune effector cells which can increase, enhance or prolong the anti-tumor activity of the immune effector cells; increase the number of anti-tumor immune effector cells or activated immune effector cells; and promote IFN-γ secretion, tumor regression, tumor shrinkage and tumor necrosis.

The term "promoter" as used herein is a DNA sequence recognized by a synthetic mechanism of a cell or an introduced synthetic mechanism required to initiate the specific transcription of a polynucleotide sequence.

A typical eukaryotic promoter consists of a minimal promoter and other cis elements. The minimal promoter is essentially a TATA box region, where RNA polymerase II (polII), TATA binding protein (TBP) and TBP-related factor (TAF) can be combined to initiate transcription. It has been found that such sequence elements (e.g., enhancers) increase the overall expression level of adjacent genes, generally in a location and/or orientation-independent manner.

NFAT (Nuclear factor of activated T cells) is a nuclear factor of activated T cells. In some specific embodiments, NFAT plays an important role in the transcription and expression of cytokines during T cell activation. In some embodiments, RUNX3 is inducibly expressed using an inducible promoter. In some embodiments, the inducible promoter is NFAT promoter. In some embodiments, the encoding sequence of RUNX3 is placed under the regulation of the minimal promoter containing NFAT binding motif. In some specific embodiments, the IL2 minimal promoter containing 6 NFAT binding motifs is a promoter composed of 6 NFAT binding sites and IL2 minimal promoter in tandem.

In some embodiments, the antigen-binding receptor described herein refers to a chimeric receptor. "Chimeric receptor" as used herein refers to a fusion molecule formed by linking DNA fragments or cDNAs corresponding to proteins from different sources using gene recombination technology. A chimeric receptor generally includes an extracellular domain, transmembrane domain, and intracellular domain. The chimeric receptor that can be used in the present invention includes but not limited to: chimeric antigen receptor (CAR), modified T cell (antigen) receptor (TCR), T cell fusion protein (TFP), T cell antigen coupler (TAC).

As used herein, "chimeric antigen receptor" or "CAR" refers to a group of polypeptides that, when present in immune effector cells, render the cells with specificity against target cells (usually cancer cells) and generate intracellular signals. CAR usually includes at least one extracellular antigen binding domain (also named as extracellular region), transmembrane domain (also named as transmembrane region), and cytoplasmic signaling domain (also named herein as "intracellular signaling domain" or "intracellular region") which includes functional signaling domains derived from stimulatory molecules and/or costimulatory molecules as defined below. In certain aspects, groups of polypeptides are bound to each other. The group of polypeptides includes a dimerization switch that can couple polypeptides to each other in the presence of a dimerization molecule, for example, for coupling an antigen-binding domain to an intracellular signal transduction domain. In one aspect, the stimulatory molecule is the ζ chain binding to T cell receptor complex. In one aspect, the cytoplasmic signaling domain further comprises one or more functional signaling domains derived from at least one costimulatory molecule as defined below. In one aspect, the costimulatory molecule is selected from the costimulatory molecules described herein, such as 4-1BB (i.e., CD137), CD27 and/or CD28. In one aspect, the CAR comprises a chimeric fusion protein comprising an extracellular antigen binding domain, transmembrane domain and intracellular signaling domain comprising a functional signaling domain derived from a stimulatory molecule. In one aspect, the CAR comprises a chimeric fusion protein comprising an extracellular antigen-binding domain, transmembrane domain and a functional signaling domain derived from a co-stimulatory molecule and an intracellular signaling domain derived from a functional signaling domain of a stimulatory molecule. In one aspect, the CAR comprises a chimeric fusion protein comprising an extracellular antigen-binding domain, transmembrane domain, and comprises two functional signaling domains derived from one or more costimulatory molecules.

"Transmembrane domain" as used herein refers to a cell membrane-spanning region in a protein sequence, and may include one or more additional amino acids adjacent to the transmembrane region, for example, one or more amino acids associated with the extracellular region of the protein, from which the transmembrane region is derived (for example, 1, 2, 3, 4, 5, 6, 7, 8, 9, 10 up to 15 amino acids in the extracellular region) and/or one or more additional amino acids associated with the extracellular region of the protein, from which the transmembrane protein is derived (e.g., 1, 2, 3, 4, 5, 6, 7, 8, 9, 10 up to 15 amino acids in the intracellular region). In one aspect, the transmembrane domain is a domain related to one of the other domains of the chimeric receptor. For example, in one embodiment, the transmembrane domain may be derived from the same protein, from which the signaling domain, co-stimulatory domain or hinge domain is derived. In some cases, the transmembrane domain can be selected or modified by amino acid substitutions to prevent such domains from binding to transmembrane domains of the same or different surface membrane proteins, for example, to minimize the interaction with other members of the receptor complex. In one aspect, the transmembrane domain is capable of being subjected to homodimerization with another chimeric receptor on the surface of the cell expressing the chimeric receptor. The transmembrane domain can be derived from natural or recombinant sources. When the source is natural source, the domain can be derived from any membrane-bound protein or transmembrane protein. In one aspect, the transmembrane domain is capable of transmitting a signal to the intracellular domain whenever the chimeric receptor binds to the target. The transmembrane domain, which can be specifically used in the present invention, may include at least the following transmembrane domains: for example, α, β or ζ chains of T cell receptors, CD28, CD27, CD3ε, CD45, CD4, CD5, CD8, CD9, CD16, CD22, CD33, CD37, CD64, CD80, CD86, CD134, CD137, CD154. In some embodiments, the transmembrane domain may include at least the following transmembrane regions: for example, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2Rβ, IL2Rγ, IL7Rα, ITGA1, VLA1, CD49a, ITGA4, IA4, CD49D, ITGA6, VLA 6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO) -3), BLAME (SLAMF8), SELPLG (CD162), LTBR, PAG/Cbp, NKG2D, NKG2C.

In some cases, the transmembrane domain can be connected to the extracellular region of a CAR, such as the antigen binding domain of the CAR via a hinge (for example, a hinge from a human protein). Optionally, short oligopeptide or polypeptide linkers between 2 and 10 amino acids in length can form a bond between the transmembrane domain of the CAR and the cytoplasmic region. Glycine-serine dimer provides a particularly suitable linker.

As used herein, "cytoplasmic domain" (also named as intracellular region) includes intracellular signaling domain. The intracellular signaling domain is responsible for the activation of immune effector functions of an immune cell into which the chimeric receptor has been introduced. The immune effector function of an immune cell can be, for example, cytolytic activity or auxiliary activity, including secretion of cytokines. Therefore, the term "intracellular signaling domain" refers to a part of a protein that transduces immune effector function signals and guides cells to perform specific functions. Although the entire intracellular signaling domain can usually be used, in many cases it is not necessary to use the entire chain. When the truncated part of the intracellular signaling domain is used, such a truncated part can be used instead of the complete chain, as long as it transduces the immune effector function signal. Therefore, the term intracellular signaling domain means that a truncated portion of the intracellular signaling domain sufficient to transduce immune effector function signals is included.

It is well known that the signal generated by TCR alone is not sufficient to fully activate T cells, and secondary and/or costimulatory signals are also required. Therefore, T cell activation can be considered as being mediated by two different kinds of cytoplasmic signaling sequences: those that trigger antigen-dependent primary activation by TCR (primary intracellular signaling domains) and those that act in an antigen-independent manner to provide secondary or costimulatory signals (secondary cytoplasmic domains, such as costimulatory domains).

The term "stimulatory molecule" refers to a molecule expressed by immune cells (e.g., T cells, NK cells, B cells) to provide cytoplasmic signal transduction sequences that modulate the activation of immune cells used in at least some aspects of immune cell signaling pathways in a stimulating manner. In one aspect, the signal is a primary signal initiated by, for example, the binding of TCR/CD3 complex and MHC antigen peptide complex, and mediates T cell responses, including, but not limited to, proliferation, activation, differentiation, and the like. The primary cytoplasmic signaling sequence (also named as "primary signaling domain") that acts in a stimulating manner may contain signaling motif which is named as immunoreceptor tyrosine-based activation motif (ITAM). In particular, examples of ITAM-containing cytoplasmic signaling sequences used herein include, but are not limited to, those derived from CD3ζ, common FcRγ (FCER1G), FcγRIIa, FcRβ (FcEpsilon R1b), CD3γ, CD3δ, CD3ε, CD79a, CD79b, DAP10 and DAP12. The intracellular signaling domain in any of the CARs described herein includes intracellular signaling sequences, such as the primary signaling sequence of CD3-ζ. In the specific CARs described herein, the primary signaling sequence of CDS-ζ is equivalent residues from human or non-human species, such as mouse, rodent, monkey, ape, etc.

The term "costimulatory molecule" refers to a homologous binding partner on T cells, which specifically binds a costimulatory ligand, thereby mediating the costimulatory response of T cells, such as but not limited to proliferation. Co-stimulatory molecules are cell surface molecules other than antigen receptors or ligands thereof, which promote an effective immune response. Co-stimulatory molecules include but are not limited to MHC class I molecules, BTLA and Toll ligand receptors, and OX40, CD27, CD28, CDS, ICAM-1, LFA-1 (CD11a/CD18), ICOS (CD278) and 4- 1BB (CD137). Further examples of such costimulatory molecules include CDS, ICAM-1, GITR, BAFFR, HVEM (LIGHTR), SLAMF7, NKp80 (KLRF1), NKp44, NKp30, NKp46, CD160, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1 CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand specifically binding to CD83.

The costimulatory intracellular signaling domain can be the intracellular part of a costimulatory molecule. The costimulatory molecules can be represented by the following proteins: TNF receptor protein, immunoglobulin-like protein, cytokine receptor, integrin, signaling lymphocyte activation molecule (SLAM protein), and NK cell receptor. Examples of such molecules include CD27, CD28, 4-1BB (CD137), OX40, GITR, CD30, CD40, ICOS, BAFFR, HVEM, ICAM-1, antigen-1 (LFA-1) associated with lymphocyte function, CD2, CDS, CD7, CD287, LIGHT, NKG2C, NKG2D, SLAMF7, NKp80, NKp30, NKp44, NKp46, CD160, B7-H3 and ligands specifically binding to CD83, etc.

The intracellular signaling domain may include all intracellular part or all of the natural intracellular signaling domain of the molecule, or a functional fragment or derivative thereof.

The term "4-1BB" (CD137) refers to a member of TNFR superfamily with the amino acid sequence provided in GenBank Accession No.AAA62478.2, or equivalent residues from non-human species, such as mice, rodents, monkeys, apes, etc.; and "4-1BB costimulatory domain" is defined as the amino acid residues 214-255 of GenBank Accession No.AAA62478.2, or the equivalent residues from non-human species, such as mouse, rodent, monkey, ape, etc. In one aspect, the "4-1BB costimulatory domain" is equivalent residues from humans or from non-human species, such as mice, rodents, monkeys, apes, and the like.

The term "T cell receptor (TCR)" is a characteristic mark on the surface of all T cells, which binds to CD3 by non-covalent bonds to form a TCR-CD3 complex. TCR is responsible for recognizing antigens bound to major histocompatibility complex molecules. TCR is a heterodimer composed of two different peptide chains, α and β chains, each of which can be divided into several parts, variable region (V region), constant region (C region), transmembrane region and cytoplasmic region, characterized in that the cytoplasmic region is very short. TCR molecules belong to the immunoglobulin superfamily, and their antigen specificity exists in the V region; each of V regions (Vα, Vβ) has three hypervariable regions CDR1, CDR2, and CDR3, with CDR3 having the largest variation, which directly determines the antigen-binding specificity of TCR. When TCR recognizes the MHC-antigen peptide complex, CDR1 and CDR2 recognize and bind to the side wall of the antigen binding groove of the MHC molecule, and CDR3 directly binds to the antigen peptide. TCR is divided into two categories: TCR1 and TCR2; TCR1 is composed of two chains, γ and δ, and TCR2 is composed of two chains, α and β.

The term "T cell fusion protein (TFP)" includes recombinant polypeptides derived from various polypeptides that constitute TCR, which can bind to the surface antigens of target cells, interact with other polypeptides of the complete TCR complex and usually co-localized on the surface of T cells. TFP consists of a TCR subunit and an antigen binding domain consisting of a human or humanized antibody domain, wherein the TCR subunit includes at least part of the TCR extracellular domain, transmembrane domain, and the stimulation domain of the internal signal domain of the TCR intracellular domain; the TCR subunit and the antibody domain are effectively connected, wherein the extracellular, transmembrane and intracellular signal domains of the TCR subunit are derived from CD3ε or CD3γ, and the TFP integrates into the TCR expressed on T cells.

The term "T cell antigen coupler (TAC)" includes three functional domains: 1. tumor-targeting domain, including single-chain antibodies, designed ankyrin repeat protein (DARPin) or other targeting groups; 2. extracellular domain, a single-chain antibody binding to CD3, so that TAC receptor and TCR receptor are close; 3. transmembrane region and intracellular region of CD4 co-receptor, wherein the intracellular region is connected to the protein kinase LCK to catalyze the phosphorylation of immunoreceptor tyrosine activation motifs (ITAM) of the TCR complex as the initial step of T cell activation.

The term "antibody" refers to a protein or polypeptide sequence derived from an immunoglobulin molecule specifically binding to an antigen. Antibodies can be of polyclonal or monoclonal, multi-chain or single-chain, or whole immunoglobulins, and can be derived from natural sources or recombinant sources. The antibody may be a tetramer of immunoglobulin molecules.

The term "antibody fragment" refers to at least a portion of an antibody that retains the ability to specifically interact with an epitope of an antigen (e.g., through binding, steric hindrance, stabilization/destabilization, spatial distribution). Examples of antibody fragments include, but are not limited to, Fab, Fab', F(ab')₂, Fv fragments, scFv, disulfide-linked Fv (sdFv), Fd fragments composed of VH and CHI domains, linear antibodies, single domain antibodies (such as sdAb), multispecific antibodies formed by antibody fragments (such as bivalent fragments including two Fab fragments connected by disulfide bonds in the hinge region) and isolated CDRs or other epitope binding fragments of antibodies.

The term "scFv" refers to a fusion protein comprising at least one antibody fragment comprising light chain variable region and at least one antibody fragment comprising heavy chain variable region, wherein the light chain and heavy chain variable regions are contiguous (for example, via a synthetic linker, such as a short flexible polypeptide linker), and can be expressed as a single-chain polypeptide, and wherein the scFv retains the specificity of the intact antibody from which it is derived. Unless specified, as used herein, scFv may have the VL and VH variable regions in any order (for example, relative to the N-terminus and C-terminus of the polypeptide), and the scFv may include VL-linker-VH or may include VH-linker-VL.

The term "antibody heavy chain" refers to the larger of the two polypeptide chains which is present in the antibody molecule in its naturally occurring configuration and usually determines the type of antibody.

The term "antibody light chain" refers to the smaller of the two polypeptide chains which is present in the antibody molecule in its naturally occurring configuration. κ(k) and λ(l) light chains refer to the two main isotypes of antibody light chains.

The term "recombinant antibody" refers to an antibody produced using recombinant DNA technology, such as an antibody expressed by a phage or yeast expression system. The term should also be interpreted as referring to antibodies that have been produced by synthesizing a DNA molecule encoding the antibody (and wherein the DNA molecule expresses the antibody protein) or the amino acid sequence of the specified antibody, wherein the DNA or amino acid sequence has been obtained by recombinant DNA or amino acid sequence technology which is available and well-known in the art.

The term "antigen" or "Ag" refers to a molecule that causes an immune response. The immune response may involve the production of antibodies or the activation of cells with specific immunity or both. A skilled person should understand that any macromolecule including virtually all proteins or peptides can serve as an antigen. In addition, the antigen can be derived from recombinant or genomic DNA. When the term is used herein, a skilled person should understand that any DNA that includes a nucleotide sequence or part of a nucleotide sequence encoding a protein that causes an immune response can encode an "antigen." In addition, a skilled person should understand that the antigen need not be encoded only by the full-length nucleotide sequence of the gene. It is obvious that the present invention includes but is not limited to the use of partial nucleotide sequences of more than one gene, and these nucleotide sequences are arranged in different combinations to encode polypeptides that elicit a desired immune response. Moreover, a skilled person should understand that antigens need not be encoded by "genes" at all. It is obvious that the antigen can be synthetically produced, or it can be derived from a biological sample, or it can be a macromolecule other than a polypeptide. Such biological samples may include, but are not limited to tissue samples, tumor samples, cells or fluids containing other biological components.

"Tumor antigen" refers to an antigen that is newly emerged or overexpressed during the occurrence and development of hyperproliferative diseases. In certain aspects, the hyperproliferative disorders described herein refer to tumors.

The tumor antigens described herein can be solid tumor antigens or hematoma antigens.

The tumor antigens described herein include but are not limited to: Thyroid Stimulating Hormone Receptor (TSHR); CD171; CS-1; C-type lectin-like molecule-1; Ganglioside GD3; Tn antigen; CD19; CD20; CD 22 ; CD30; CD70; CD123; CD138; CD33; CD44; CD44v7/8; CD38; CD44v6; B7H3 (CD276), B7H6; KIT (CD117); Interleukin 13 receptor subunit α (IL-13Rα); Interleukin 11 receptor α (IL-11Rα); Prostate Stem Cell Antigen (PSCA); Prostate Specific Membrane Antigen (PSMA); Carcinoembryonic Antigen (CEA); NY-ESO-1; HIV-1 Gag; MART-1; gp100; Tyrosine Enzyme; Mesothelin; EpCAM; Protease Serine 21 (PRSS21); Vascular Endothelial Growth Factor Receptor, Vascular Endothelial Growth Factor Receptor 2 (VEGFR2); Lewis (Y) Antigen; CD24; Platelet Derived Growth Factor Receptor β (PDGFR) -β); stage-specific embryonic antigen-4 (SSEA-4); cell surface-associated mucin 1 (MUC1), MUC6; epidermal growth factor receptor family and its mutants (EGFR, EGFR2, ERBB3, ERBB4, EGFRvIII)); Neural cell adhesion molecule (NCAM); Carbonic anhydrase IX (CAIX); LMP2; Ephrin A receptor 2 (EphA2); Fucosyl GM1; Sialyl Lewis adhesion molecule (sLe); Ganglioside GM3Galp(1-4)bDGlcp(1-1)Cer; TGS5; high molecular weight melanoma-associated antigen (HMWMAA); o-acetyl GD2 ganglioside (OAcGD2); folate receptor; tumor vascular endothelium Marker 1 (TEM1/CD248); Tumor vascular endothelial marker 7 related (TEM7R); Claudin 6, Claudin 18.2, Claudin 18.1; ASGPR1; CDH16; 5T4; 8H9; αvβ6 integrin; B cell maturation antigen (BCMA); CA9; kappa light chain; CSPG4; EGP2, EGP40; FAP; FAR; FBP; embryonic AchR; HLA-A1, HLA-A2; MAGEA1, MAGE3; KDR; MCSP; NKG2D ligand; PSC1; ROR1 ; Sp17; SURVIVIN; TAG72; TEM1; Fibronectin; Tenascin; Carcinoembryonic variant of tumor necrosis zone; G protein-coupled receptor class C group 5-member D (GPRC5D); X chromosome open reading frame 61 (CXORF61); CD97; CD179a; Anaplastic Lymphoma Kinase (ALK); Polysialic acid; Placenta specific 1 (PLAC1); the hexose part of globoH glycoceramide (GloboH); breast differentiation antigen (NY-BR-1); uroplakin 2 (UPK2); hepatitis A virus cell receptor 1 (HAVCR1); adrenergic receptor β3 (ADRB3); pannexin 3 (PANX3); G protein coupled receptor 20 (GPR20); lymphocyte antigen 6 complex locus K9 (LY6K); olfactory receptor 51E2 (OR51E2); TCRγ alternating reading frame protein (TARP); Wilms tumor protein (WT1); ETS translocation variant gene 6 (ETV6-AML); Sperm protein 17 (SPA17); X antigen family member 1A (XAGE1); Angiopoietin binds to cell surface receptor 2 (Tie2); Melanoma cancer testis antigen-1 (MAD-CT-1); Melanoma cancer testis antigen-2 (MAD-CT-2); Fos-related antigen 1; p53 mutant; human telomerase reverse transcriptase (hTERT); sarcoma translocation breakpoint; melanoma inhibitor of apoptosis (ML-IAP); ERG (transmembrane protease serine 2 (TMPRSS2) ETS fusion gene); N-acetylglucosaminyl transferase V (NA17); Pairing box protein Pax-3 (PAX3); Androgen receptor; Cyclin B1; V-myc avian myeloidosis virus oncogene neuroblastoma-derived homolog (MYCN); Ras homolog Family member C (RhoC); Cytochrome P450 1B1 (CYP1B1); CCCTC binding factor (zinc finger protein)-like (BORIS); Squamous cell carcinoma antigen 3 (SART3) recognized by T cells; Paired box protein Pax-5 (PAX5); proacrosin binding protein sp32 (OYTES1); lymphocyte-specific protein tyrosine kinase (LCK); A kinase anchoring protein 4 (AKAP-4); synovial sarcoma X breakpoint 2 (SSX2); CD79a; CD79b ; CD72; Leukocyte-associated immunoglobulin-like receptor 1 (LAIR1); IgA receptor Fc fragment (FCAR); Leukocyte immunoglobulin-like receptor subfamily member 2 (LILRA2); CD300 molecular-like family member f (CD300LF) ; C-type lectin domain family 12 member A (CLEC12A); bone marrow stromal cell antigen 2 (BST2); mucin-like hormone receptor-like 2 (EMR2) containing EGF-like module; lymphocyte antigen 75 (LY75); phosphatidyl Inositol proteoglycan-3 (GPC3); Fc receptor-like 5 (FCRL5); immunoglobulin lambda-like polypeptide 1 (IGLL1).

The pathogen antigen is selected from: virus, bacteria, fungus, protozoa, or parasite antigen; and virus antigen is selected from: cytomegalovirus antigen, Epstein-Barr virus antigen, human immunodeficiency virus antigen, or influenza virus antigen.

"Tumor" refers to a broad category of disorders in which hyperproliferative cell growth occurs *in vitro* (e.g., transformed cells) or *in vivo.* Conditions that can be treated or prevented by the methods described herein include, for example, various neoplasms, including benign or malignant tumors, various hyperplasias, etc. Specific examples of cancer include but are not limited to: breast cancer, prostate cancer, leukemia, lymphoma, nasopharyngeal cancer, colon cancer, rectal cancer, renal cell carcinoma, liver cancer, non-small cell lung cancer, small intestine cancer, esophageal cancer, melanoma, bone cancer, pancreatic cancer, skin cancer, head and neck cancer, uterine cancer, ovarian cancer, stomach cancer, testicular cancer, fallopian tube cancer, endometrial cancer, cervical cancer, vaginal cancer, thyroid cancer, parathyroid cancer, adrenal gland cancer, soft tissue sarcoma, urethral cancer, penile cancer, bladder cancer, ureter cancer, renal pelvis cancer, central nervous system (CNS) tumor, spine tumor, glioma, pituitary adenoma, astrocytoma, a combination and metastatic foci thereof.

The term "transfected" or "transformed" or "transduced" refers to a process by which exogenous nucleic acid is transferred or introduced into a host cell. A "transfected" or "transformed" or "transduced" cell is a cell that has been transfected, transformed, or transduced with exogenous nucleic acid. The cell includes the cell of the primary subject and a progeny thereof.

The term "specifically binds" refers to an antibody or ligand binding to a protein of a binding partner (e.g., tumor antigen) present in a sample, but the antibody or ligand does not substantially recognize or bind to other molecules in the sample.

The term "refractory" refers to a disease, such as a tumor, which does not respond to a treatment. In embodiments, the refractory tumor may be resistant to a treatment before or at the beginning of the treatment. In other embodiments, a refractory tumor can become resistant during treatment. A refractory tumor is also named as a resistant tumor. In the present invention, a refractory cancer includes, but are not limited to, a cancer which is not sensitive to radiotherapy, relapses after radiotherapy, not sensitive to chemotherapy, relapses after chemotherapy, not sensitive to CAR-T treatment, or relapses after CAR-T treatment. The treatment regimens described herein can be used fot the refractory or recurrent malignancies.

As used herein, "relapsed" means that signs and symptoms before the effective treatment re-appear in a patient after a period of improvement, for example, after an effective tumor treatment.

The terms "individual" and "subject" have the same meaning herein, and can be humans and animals from other species.

The term "enhancement" means that the response of a subject or tumor cells to to the treatment disclosed herein is improved. For example, an enhanced response may include 5%, 10%, 15%, 20%, 25%, 30%, 35%, 40%, 45%, 50%, 55%, 60%, 65%, 70%, 75%, 80%, 85%, 90%, 95% or 98% or higher of improvement in response. As used herein, "enhancement" can also refer to increase in the number of subjects responding to treatments such as immune effector cell therapy. For example, an enhanced response can refer to the total percentage of subjects responding to treatment, where the percentages are 5%, 10%, 15%, 20%, 25%, 30%, 35%, 40%, 45%, 50%, 55 %, 60%, 65%, 70%, 75%, 80%, 85%, 90%, 95% or 98% more.

In one aspect, the treatment is determined based on clinical results; the increase, enhancement or extension of the anti-tumor activity of T cells; compared with the number before treatment, the increase in the number of anti-tumor T cells or activated T cells, the promotion of IFN-γ secretion, or a combination thereof. In another aspect, the clinical results are tumor regression; tumor shrinkage; tumor necrosis; anti-tumor response through the immune system; tumor enlargement, recurrence or spread, or a combination thereof. In another aspect, the therapeutic effect is predicted by the presence of T cells, the presence of genetic markers indicative of T cell inflammation, promotion of IFN-γ secretion, or a combination thereof.

The immune effector cells as disclosed herein can be administered to an individual via various routes, including, for example, orally or parenterally, such as intravenous, intramuscular, subcutaneous, intraorbital, intrasaccular, intraperitoneal, intrarectal, intracisternal, intratumoral, intravasal, intradermal route, or passive or promoted absorption through the skin using, for example, skin patches or transdermal iontophoresis, respectively.

When practicing the methods described herein, the total amount of agent to be administered can be administered to the subject as a single dose as a bolus injection or by infusion over a relatively short period of time, or can be administered using a graded treatment regimen, wherein multiple doses are administered over an extended time of period. A skilled person will know that the amount of the composition for treating pathological conditions in a subject depends on many factors, including the age and general health of the subject, as well as the route of administration and the number of treatments to be administered. Taking these factors into account, a technician will adjust the specific dosage as needed. In general, phase I and phase II clinical trials are initially used to determine the formulation of the composition and the route and frequency of administration.

As used herein, "GPC3" or "Glypican 3" is a member of Glypican family, which plays an important role in regulating cell growth and differentiation. Abnormal expression of GPC3 is closely related to the occurrence and development of a variety of tumors, such as liver cancer, lung cancer, breast cancer, ovarian cancer, kidney cancer, thyroid cancer, gastric cancer, colorectal cancer, and so on.

In the present invention, immune effector cells target tumors positively expressing GPC3. In a specific embodiment, the tumor includes, but is not limited to, breast cancer, glioma, liver cancer, gastric cancer, lung cancer, esophageal cancer, head and neck cancer, bladder cancer, ovarian cancer, cervical cancer, kidney cancer, pancreatic cancer, cervical cancer, liposarcoma, melanoma, adrenal carcinoma, schwannoma, malignant fibrous histiocytoma, esophageal cancer. A skilled person will know that some tumor cells, such as liver cancer cells, are not sensitive to many drugs. Therefore, some drugs may sometimes have poor or no effect *in vivo,* even if they are effective *in vitro.* Therefore, in a preferred embodiment, the GPC3-positive tumors or GPC-positive tumors described herein are liver cancer, gastric cancer, lung cancer, and esophageal cancer. In some embodiments, in the PLC/PRF/5 liver cancer cell small-load subcutaneous xenograft model, there are significant tumor-suppressing effects in the 9F2-BBZ(CD28TM)-RUNX3 CART group and 9F2-BBZ-RUNX3 CART group, and compared with 9F2-28Z CART group and 9F2-BBZ CART group, the tumor suppressing effects are better. In the PLC/PRF/5 liver cancer cell heavy-load subcutaneous xenograft model, there are significant anti-tumor effects in the 9F2-28Z-RUNX3 CART group and 9F2-BBZ-RUNX3 CART group, and compared with the 9F2-28Z CART group and 9F2-BBZ CART group, the anti-tumor effects are significantly improved.

When CAR-T cells co-expressing exogenous RUNX3 are used in a subject, the corresponding species can be selected. For example, when used in mice, mouse-derived RUNX3 is used, and elements for constructing a CAR, such as transmembrane domain and intracelluar domain can also be of murine origin. When the subject is a human, human-derived RUNX3 and human-derived CAR elements are preferred.

In some embodiments, the sequence of a CAR used may be as shown in SEQ ID NO: 57, 58, or 59.

In some embodiments, the nucleic acid sequences for expressing CARs targeting GPC3 and exogenous RUNX3 described herein can be, for example, the nucleic acid sequences as shown in SEQ ID NOs: 19, 17, and 53. The term "CLD18" refers to claudin 18 (CLD18), Genbank accession number: splice variant 1 (CLD18A1): NP_057453, NM016369, and splice variant 2 (CLD18A2): NM_001002026, NP_001002026, which is a intrinsic transmembrane protein with a molecular weight of about 27,9/27,72 kD. Claudin is an internal membrane protein located in the tight junction of epithelium and endothelium, a network of interconnected chains of particles in the tissue membrane tightly connected between adjacent cells. In tight junctions, occludin and claudin are the most important transmembrane protein components, A primary barrier is produced to prevent and control the paracellular transport of solutes and restrict the lateral diffusion of membrane lipids and proteins to maintain cell polaritym due to their strong intercellular adhesion properties. The proteins that form tight junctions are critically involved in the structure of tissue epithelial tissues.

CLD18A1 (Claudin 18A1) is selectively expressed in the epithelium of normal lung and stomach, while CLD18A2 (Claudin 18A2) is only expressed in gastric cells. Moreover, CLD18A2 is limited to differentiated short-lived gastric epithelial cells, but does not exist in the gastric stem cell area. Both variants are strongly expressed in several types of cancer, including tumors of the stomach, esophagus, pancreas, and lung, as well as human cancer cell lines. The expression thereof is mainly in the adenocarcinoma subtypes of these indications.

The term "CLD18" includes any variants (including CLD18A1 and CLD18A2), conformations, isoforms, and species homologs of CLD18 that are naturally expressed by cells or expressed by cells transfected with the CLD18 gene. Preferably, "CLD18" refers to human CLD18, particularly CLD18A2 (SEQ ID NO: 51) and/or CLD18A1 (SEQ ID NO: 52), more preferably CLD18A2.

In some embodiments, CAR T cells expressing RUNX3 and targeting CLD18A2 have significant toxic killing effects on PANC02-A2 which is positive for claudin 18.2, but have almost no killing effects on PANC02 cells which are negative for claudin 18.2. In some embodiments, CAR T cells expressing RUNX3 and targeting CLD18A2 have significant anti-tumor effects on the subcutaneous xenograft model of pancreatic cancer cell PANC02-A2 positively expressing claudin 18.2, and compared with CLD18A2-CAR T cells, the tumor-suppressing effects are better.

When CAR-T cells co-expressing exogenous RUNX3 are used in a subject, the corresponding species can be selected. For example, when used in mice, mouse-derived RUNX3 or fragments thereof are used, and elements for constructing CAR, such as transmembrane domains, intracellular domains, etc. can also be of murine origin. When the subject is a human, human-derived RUNX3 or fragments thereof and human-derived CAR elements are preferred.

In some embodiments, the sequence of the used CAR may be as shown in SEQ ID NO: 60, 61, or 62.

In some specific embodiments, the nucleic acid sequence expressing the CAR targeting CLD18A2 and exogenous RUNX3 as described herein may be the nucleic acid sequence as shown in SEQ ID NO: 54, 55, 56. Cytokines are soluble proteins produced by cells induced by immunogens or other stimulants. In some embodiments, cytokines refer to, for example, IL15, IL18, and IL21. A cytokine polypeptide refers to a truncated fragment of a cytokine that has a similar function to a cytokine, such as truncated fragments of natural full-length IL15 that have similar biological functions as IL15., Cells can express exogenous cytokines or functional fragments thereof through conventional bioengineering methods.

Interleukin 15 (IL15 or IL-15), human IL15 has a 4-helix bundle structure, a molecular weight of 14-15kD, contains 114 amino acids, and is mainly synthesized in monocytes and dendritic cells. It is encoded by a single gene, located on chromosome 4q31, and contains 8 exons. As a soluble cytokine, IL-15 plays an important role in the replication and differentiation of NK cells, T cells and B cells. The process of tumor occurrence, development and metastasis depends on the lymphocyte-mediated immune response, and IL-15 enhances the differentiation and proliferation of T cells and the secretion of antibodies by B cells, which play a key role in enhancing the immune response to tumor cells.

IL-15 can interact with IL-15R (preferably from mammals, such as murine or human IL-15), preferably from mammals (e.g., murine or human), and has one of the following characteristics: (i) an amino acid sequence of naturally occurring mammalian IL-15 or fragments thereof, such as the amino acid sequence as shown in SEQ ID NO: 39 (human) or fragments thereof; (ii) an amino acid sequence substantially having at least 85%, 90%, 95%, 98%, 99% homology with the amino acid sequence as shown in SEQ ID NO: 39 (human) or a fragment thereof; (iii) an amino acid sequence encoded by a nucleotide sequence of naturally occurring mammalian IL-15 or fragments thereof (such as SEQ ID NO: 38 (human) or a fragment thereof); (iv) an amino acid sequence encoded by a nucleotide sequence having, for example, at least 85%, 90%, 95% , 98%, 99% homology with the nucleotide sequence as shown in SEQ ID NO: 38 (human) or a fragment thereof; (v) an amino acid sequence encoded by a nucleotide sequence degenerate from the naturally occurring IL-15 nucleotide sequence or a fragment thereof (such as, SEQ ID NO: 38 (human) or a fragment thereof); or (vi) a nucleotide sequence that hybridizes to one of the aforementioned nucleotide sequences under stringent conditions, such as high stringency conditions.

"Enhancement in IL-15R activity" should be understood to mean that the IL-15R-binding protein of the present disclosure enhances any one or more activities of naturally occurring IL-15R, including but not limited to stimulating the proliferation, cytotoxicity or maturation of NK cells; stimulating the proliferation or differentiation of B cells and T cells; stimulating the production and affinity maturation of antibodies in B cells; stimulating the cytotoxicity of CD8+ T cells; stimulating the production of interferon γ in T cells and NK cells; inhibiting the activation and maturation of dendritic cells (DC); inhibiting the release of inflammatory mediators from mast cells; enhancing the phagocytosis of macrophages; inhibiting the production or survival of TReg cells; and stimulating the proliferation of bone marrow progenitor cells.

In some embodiments, for liver cancer cells with high expression of GPC3, when the effector target ratio is 3:1 and 1:1, the RUNX3-CAR T cells (CAR T cells expressing RUNX3) expressing IL15 in combination are better than the cells in the control group (CAR T cells only expressing RUNX3). In some embodiments, the tumor-inhibiting effects of the RUNX3-CAR T cells expressing IL15 in combination are better than those of the cells in the control group (CAR T cells only expressing RUNX3), in which 1 of the 5 mice showed tumor regression.

Interleukin 18 (IL-18 or IL18) is a synonym of IL-18 polypeptide, interleukin-18 polypeptide, IFN-γ inducible factor or interferon-y inducible factor, which refers to a protein (preferably from mammals, such as murine or human) interacting (e.g. binding to) with IL-18R (NM_003855.3, NM_001282399.1) (preferably from mammals, such as murine or human IL-18), and has one of the following characteristics: (i) an amino acid sequence of naturally occurring mammalian IL-18 or fragments thereof, such as the amino acid sequence as shown in SEQ ID NO: 41 (human) or fragments thereof; (ii) an amino acid sequence substantially having at least 85%, 90%, 95%, 98%, 99% homology with the amino acid sequence as shown in SEQ ID NO: 41 (human) or a fragment thereof; (iii) an amino acid sequence encoded by a nucleotide sequence of naturally occurring mammalian IL-18 or fragments thereof (such as SEQ ID NO: 40 (human) or a fragment thereof); (iv) an amino acid sequence encoded by a nucleotide sequence having, for example, at least 85%, 90%, 95% , 98%, 99% homology with the nucleotide sequence as shown in SEQ ID NO: 40 (human) or a fragment thereof; (v) an amino acid sequence encoded by a nucleotide sequence degenerate from the naturally occurring IL-18 nucleotide sequence or a fragment thereof (such as, SEQ ID NO: 40 (human) or a fragment thereof); or (vi) a nucleotide sequence that hybridizes to one of the aforementioned nucleotide sequences under stringent conditions, such as high stringency conditions. Throughout this specification, the term IL-18 interchangeably includes pro-IL-18 (the precursor of mature IL-18 before protease cleavage) and mature IL-18 (after protease cleavage), unless specifying pro- or mature form.

"Enhancement in IL-18R activity" should be understood to mean that the IL-18R-binding protein of the present disclosure enhances any one or more activities of naturally occurring IL-18R, including but not limited to stimulating the proliferation, cytotoxicity or maturation of NK cells; stimulating the proliferation or differentiation of B cells and T cells; stimulating the production and affinity maturation of antibodies in B cells; stimulating the cytotoxicity of CD8+ T cells; stimulating the production of interferon γ in T cells and NK cells; inhibiting the activation and maturation of dendritic cells (DC); inhibiting the release of inflammatory mediators from mast cells; enhancing the phagocytosis of macrophages; inhibiting the production or survival of TReg cells; and stimulating the proliferation of bone marrow progenitor cells.

In some embodiments, for liver cancer cells with high or moderate expression of GPC3, when the effector target ratio is 3:1 and 1:1, the RUNX3-CAR T cells (CAR T cells expressing RUNX3) expressing IL18 in combination are better than the cells in the control group (CAR T cells only expressing RUNX3). In some embodiments, the tumor-inhibiting effects of the RUNX3-CAR T cells expressing IL18 in combination are better than those of the cells in the control group (CAR T cells only expressing RUNX3).

"Interleukin 21 (IL-21 or IL21)" is a type I cytokine, which is produced by activated CD4+ T cells, NKT cells, Tfh cells and Th17 cells, of a higher homology with IL-2, IL-4, and IL-15, and belongs to the γc family member. hIL-21 (human IL21) is located on the long arm of chromosome 4 (4q26-27), transcribes a mature mRNA consisting of 642 nucleotides, encodes a protein precursor consisting of 162 amino acids, of which the first 31 amino acids are signal peptides, and the latter 131 amino acids constitute mature IL 21 with a four-helical domain and a molecular weight of 15KD. The 5' regulatory region of IL-21 contains three T cell activating nuclear factor (NF AT) binding sites, and the activity of IL-21 promoter is produced by the action of calcium ionophores on cells. There are two DNaseI hypersensitive sites in IL-21, both of which are conserved in humans and mice. One of them is located in the IL-21 promoter region and is related to TCR-mediated IL-21 transcription. hIL-21 can specifically bind to human interleukin 21 receptor (hIL-21R), activate JAK/STAT and other signal transmission pathways, and exhibit complex biological effects. It can regulate differentiation, apoptosis of B cell, produce subtupes of antibody, promote T cell-mediated acquired immunity, enhance the cytotoxicity of NK cells and the ability to produce IFN γ, and mediate the transition between active immunity and passive immunity. rhIL-21 plays an important role in allergic reactions, inflammatory reactions, autoimmune reactions and anti-tumor clinical applications.

The term "IL-21" or "IL-21 polypeptide" as used herein refers to a protein (preferably from a mammal, such as a mouse or a human) capable of interacting (for example, binding to) with IL-21R (NM_021798.3) (preferably from a mammal, such as murine or human IL-21), and has one of the following characteristics: (i) an amino acid sequence of naturally occurring mammalian IL-21 or fragments thereof, such as the amino acid sequence as shown in SEQ ID NO: 36 (human) or fragments thereof; (ii) an amino acid sequence substantially having at least 85%, 90%, 95%, 98%, 99% homology with the amino acid sequence as shown in SEQ ID NO: 36 (human) or a fragment thereof; (iii) an amino acid sequence encoded by a nucleotide sequence of naturally occurring mammalian IL-21 or fragments thereof (such as SEQ ID NO: 35 (human) or a fragment thereof); (iv) an amino acid sequence encoded by a nucleotide sequence having, for example, at least 85%, 90%, 95% , 98%, 99% homology with the nucleotide sequence as shown in SEQ ID NO: 35 (human) or a fragment thereof; (v) an amino acid sequence encoded by a nucleotide sequence degenerate from the naturally occurring IL-21 nucleotide sequence or a fragment thereof (such as, SEQ ID NO: 35 (human) or a fragment thereof); or (vi) a nucleotide sequence that hybridizes to one of the aforementioned nucleotide sequences under stringent conditions, such as high stringency conditions.

"Enhancement in IL-21R activity" should be understood to mean that the IL-21R-binding protein of the present disclosure enhances any one or more activities of naturally occurring IL-21R, including but not limited to stimulating the proliferation, cytotoxicity or maturation of NK cells; stimulating the proliferation or differentiation of B cells and T cells; stimulating the production and affinity maturation of antibodies in B cells; stimulating the cytotoxicity of CD8+ T cells; stimulating the production of interferon γ in T cells and NK cells; inhibiting the activation and maturation of dendritic cells (DC); inhibiting the release of inflammatory mediators from mast cells; enhancing the phagocytosis of macrophages; inhibiting the production or survival of TReg cells; and stimulating the proliferation of bone marrow progenitor cells.

In some embodiments, for liver cancer cells with high or moderate expression of GPC3, when the effector target ratio is 3:1 and 1:1, the RUNX3-CAR T cells (CAR T cells expressing RUNX3) expressing IL21 in combination are better than the cells in the control group (CAR T cells only expressing RUNX3). In some embodiments, the tumor-inhibiting effects of the RUNX3-CAR T cells expressing IL21 in combination are better than those of the cells in the control group (CAR T cells only expressing RUNX3).

In some embodiments, the immune effector cells of the present invention are used in combination with chemotherapy drugs. In some embodiments, the chemotherapeutic drug may be Sorafenib.

Sorafenib is the first oral chemotherapy drug approved for treating advanced liver cancer, the chemical name of which is 4-(4-3-[4-chloro-3-(trifluoromethyl)phenyl]ureidophenoxy)-2-methylpyridine-2-carboxyl-4-tol uenesulfonate, the molecular formula of which is C21H16C1F3N403 • C7H8O3S, and the molecular weight of which is 637.03. Sorafenib is poorly soluble in water, slightly soluble in ethanol, and soluble in polyvinylglycerol 400 (PEG400). As a multi-enzyme inhibitor, Sorafenib can directly inhibit the proliferation of tumor cells by inhibiting multiple kinases in MAPK pathway in tumor cells (such as Raf1, BRaf). At the same time, Sorafenib can also act on platelet-derived growth factor receptor-β (PDGFR-β), vascular endothelial growth factor receptor 2, 3 (VEGFR-2,-3) to inhibit tumor growth by inhibiting angiogenesis in the tumor. The sorafenib described herein can be safely administered orally or parenterally by itself, or in a composition with pharmaceutically acceptable carriers, excipients and other additives (such as tablets, sustained-release preparations, capsules, injections, solutions). When orally administered, the composition can be formulated into tablets, dragees or capsules. To prepare the oral composition, lactose or starch can be used as a carrier, and gelatin, sodium carboxymethyl cellulose, methyl cellulose polyvinylpyrrolidone, etc. are suitable binding agents or granulating agents. Starch or microcrystalline cellulose can be used as the disintegrant, and talc, colloidal silica, glyceryl stearate, calcium or magnesium stearate, etc. are often used as suitable anti-adhesive agents and lubricants. For example, tablets can be prepared by compressing wet granules. The active ingredient and the carrier as well as one optional part of disintegrant form a mixture, the mixture and an aqueous, alcoholic or aqueous alcoholic solution of the binder are granulated in a suitable equipment, and then other disintegrants, lubricants and anti-sticking agents are added into the dried granules for tableting the mixture. For increasing the solubility, heterocyclic derivatives can be isolated and prepared into pharmaceutically acceptable organic acids, preferably methanesulfonic acid, fumaric acid, etc., to facilitate administration in a form of injection. The dosage can vary depending on the subject, the method of administration, symptoms and other factors.

In the present invention, the immune effector cells and sorafenib are given in no particular order; the sorafenib can be administered firstly and then the immune effector cells; it can also be administered simultaneously; and the immune effector cells can also be administered firstly and then sorafenib. In certain embodiments, the immune effector cell therapy is administered 1 hour, 2 hours, 3 hours, 4 hours, 5 hours, 6 hours, 7 hours, 8 hours, 9 hours, 10 hours, 11 hours , 12 hours, 1 day, 2 days, 3 days, 4 days, 5 days, 6 days, 7 days, 8 days, 9 days, 10 days, 11 days, 12 days, 13 days, 14 days, 15 days, 16 Days, 17 days, 18 days, 19 days, 20 days, 21 days, 22 days, 23 days, 24 days, 25 days, 26 days, 27 days, 28 days, 29 days, 1 month or any combination thereof before the administration of sorafenib. In certain embodiments, immune effector cell therapy is administered 1 hour, 2 hours, 3 hours, 4 hours, 5 hours, 6 hours, 7 hours, 8 hours, 9 hours, 10 hours, 11 hours, 12 hours, 1 day, 2 days, 3 days, 4 days, 5 days, 6 days, 7 days, 8 days, 9 days, 10 days, 11 days, 12 days, 13 days, 14 days, 15 days, 16 Days, 17 days, 18 days, 19 days, 20 days, 21 days, 22 days, 23 days, 24 days, 25 days, 26 days, 27 days, 28 days, 29 days, 1 month or any combination thereof after the administration of sorafenib.

In some embodiments, in the group of 9F2-BBZ-RUNX3 CAR T cell therapy in combination with sorafenib, the tumor-inhibiting effects are the most significant, wherein 1 out of 5 mice exhibits tumor regression, and the weight of the mice is not significant varied.

The chimeric antigen receptor polypeptides described herein can be sequentially linked as follows:
extracellular antigen binding region-CD8 transmembrane region-4-1BB-CD3ζ,
extracellular antigen binding region-CD28a-CD28b-CD3ζ,
extracellular antigen binding region-CD28a-CD28b-4-1BB-CD3ζ,

And combinations thereof, where CD28a in the relevant chimeric antigen receptor protein represents the transmembrane region of CD28 molecule, and CD28b represents the intracellular signal region of CD28 molecule.

The present invention also includes a nucleic acid encoding the chimeric antigen receptor. The present invention also relates to variants of the aforementioned polynucleotides, which encode polypeptides having the same amino acid sequence as the present invention or polypeptide fragments, analogs and derivatives.

The present invention also provides a vector containing the nucleic acid of the chimeric antigen receptor. The invention also includes viruses comprising the vectors described above. The viruses of the invention include packaged infectious viruses as well as viruses to be packaged that contain the necessary components for packaging into infectious viruses. Other viruses known in the art that can be used to transduce exogenous genes into immune effector cells and their corresponding plasmid vectors are also useful in the present invention.

The present invention also provides a chimeric antigen-modified immune effector cell, which is transduced with a nucleic acid encoding the chimeric antigen receptor or is transduced with the aforementioned recombinant plasmid containing the nucleic acid, or the virus containing the plasmid. Conventional nucleic acid transduction methods in the art, including non-viral and viral transduction methods, can be used in the present invention. Non-viral transduction methods include electroporation and transposon methods. Recently, nucleofector nuclear transfection instrument developed by Amaxa can directly introduce foreign genes into nucleus to achieve highly efficient transduction of target genes. In addition, compared with conventional electroporation, the transduction efficiency of transposon system based on Sleeping Beauty system or PiggyBac transposon was significantly improved. The combination of nucleofector transfection instrument and SB Sleeping Beauty transposon system has been reported [Davies JK., et al. Combining CD19 redirection and alloanergization to generate tumor-specific human T cells for allogeneic cell therapy of B-cell malignancies. Cancer Res, 2010, 70(10): OF1-10.], and high transduction efficiency and site-directed integration of target genes can be achieved by this method. In one embodiment of the invention, the transduction method of a T lymphocyte modified by a chimeric antigen receptor gene is a transduction method based on a virus such as a retrovirus or a lentivirus. The method has the advantages of high transduction efficiency and stable expression of exogenous gene, and the time for in vitro culturing T lymphocytes to clinical level can be shorten. The transduced nucleic acid is expressed on the surface of the transgenic T lymphocytes by transcription, translation. *In vitro* cytotoxicity assay performed on various cultured tumor cells demonstrated that the immune effector cells modified by the chimeric antigen described herein have highly specific tumor cell killing effects (also known as cytotoxicity), and can effectively survive in tumor tissue. Therefore, the nucleic acid encoding a chimeric antigen receptor protein described herein, a plasmid comprising the nucleic acid, a virus comprising the plasmid, and a transgenic immune effector cells transfected with the nucleic acid, plasmid or virus described above can be effectively used in tumor immunotherapy.

In addition to the chimeric antigen receptor described above, the immune cells modified by the chimeric antigen described herein may also express another chimeric antigen receptor, which does not contain CD3ζ, but contains intracellular signaling domain of CD28 and intracellular signal domain of CD137, or a combination of both.

The immune cells modified by the chimeric antigen described herein can be used in the preparation of a pharmaceutical composition or diagnostic reagent. In addition to an effective amount of the antibody, immunological conjugate, or immune cell, the composition may further comprise a pharmaceutically acceptable carrier. The term "pharmaceutically acceptable" means that when the molecular entities and compositions are properly administered to animals or humans, they do not cause adverse, allergic or other untoward reactions.

Specific examples of some of the substances which may be used as pharmaceutically acceptable carriers or components thereof are sugars, such as lactose, dextrose and sucrose; starches, such as corn starch and potato starch; cellulose and its derivatives, such as carboxymethylcellulose sodium, ethylcellulose and methylcellulose; gum tragacanth; malt; gelatin; talc; solid lubricants such as stearic acid and magnesium stearate; calcium sulfate; vegetable oils, such as peanut oil, cottonseed oil, sesame oil, olive oil, corn oil and cocoa butter; polyhydric alcohols such as propylene glycol, glycerin, sorbitol, mannitol and polyethylene glycol; alginic acid; emulsifiers such as Tween®; wetting agents such as sodium lauryl sulfate; coloring agents; flavoring agents; tablets, stabilizers; antioxidants; preservatives; pyrogen-free water; isotonic saline solutions; and phosphate buffers and the like.

The composition of the present invention can be prepared into various dosage forms as needed, and the dosage to be administered to a patient can be determined by a physician according to factors, such as type, age, body weight, and general disease condition of a patient, mode of administration, and the like. For example, injection or other treatment may be used.

The invention also provides a kit containing the immune effector cells of the invention. The kit can be used to treat or prevent cancer and pathogen infection. In one embodiment, the kit may include a therapeutic or prophylactic composition containing an effective amount of immune effector cells comprising one or more unit dosage forms. In some embodiments, the kit includes a sterile container that may contain a therapeutic or prophylactic composition; and such a container may be a box, ampoule, bottle, vial, tube, bag, blister pack, or other suitable container known in the art. Such containers can be made of plastic, glass, laminated paper, metal foil or other materials suitable for holding medicines. In some embodiments, the immune effector cells of the present invention may be provided with instructions for administering the immune effector cells to subjects who are at risk of cancer and pathogen infection. The instruction will generally include information about the use of the composition to treat or prevent cancer and pathogen infection. In some embodiments, the kit may include about 1 × 10⁴ cells to about 1 × 10⁶ cells. In some embodiments, the kit may include at least about 1 × 10⁵ cells, at least about 1 × 10⁶ cells, at least about 1 × 10⁷ cells, at least about 4 × 10⁷ cells, at least about 5 × 10⁷ cells, at least about 6 × 10⁷ cells, at least about 6 × 10⁷ cells, 8 × 10⁷ cells, at least about 9 × 10⁷ cells, at least about 1 × 10⁸ cells, at least about 2 × 108 cells, at least about 3 × 10⁸ cells, at least about 4 × 10⁸ cells, at least about 5 × 10⁸ cells, at least about 6 × 10⁸ cells, at least about 6 × 10⁸ cells, at least about 8 × 10⁸ cells, at least about 9 × 10⁸ cells, at least about 1 × 10⁹ cells, at least about 2 × 10⁹ cells, at least about 3 × 10⁹ cells, at least about 4 × 10⁹ cells, at least about 5 × 10⁹ cells, at least about 6 × 10⁹ cells, at least about 8 × 10⁹ cells, at least about 9 × 10⁹ cells, at least about 1 × 10¹⁰ cells, at least about 2 × 10¹⁰ cells, at least about 3 × 10¹⁰ cells, at least about 4 × 10¹⁰ cells, at least about 5 × 10¹⁰ cells, at least about 6 × 10¹⁰ cells, at least about 9 × 10¹⁰ cells, at least about 9 × 10¹⁰ cells, at least about 1 × 10¹¹ cells, at least about 2 × 10¹¹ cells, at least about 3 × 10¹¹ cells, at least about 4 × 10¹¹ cells, at least about 5 × 10¹¹ cells, at least about 8 × 10¹¹ cells, at least about 9 × 10¹¹ cells, or at least about 1 × 10¹² cells. For example, about 5 x 10¹⁰ cells can be included in the kit. In another example, the kit may include 3 × 10⁶ cells; and the cells may be expanded to about 5 × 10¹⁰ cells and administered to a subject.

In some embodiments, the kit may include allogeneic cells. In some embodiments, the kit can include cells including genomic modifications. In some embodiments, the kit may contain "ready-to-use" cells. In some embodiments, the kit can include cells that can be expanded for clinical use. In some cases, the kit may contain contents for research purposes.

In some embodiments, the instruction includes at least one of the following: a description of the therapeutic agent; a dosage regimen and administration for treating or preventing tumors, pathogen infections, immune diseases, or allogeneic transplantation or symptoms thereof; preventive measures, warnings, contraindications, overdose information, adverse reactions, animal pharmacology, clinical research, and/or cited literature. The instruction can be printed directly on the container (if any), or as a label on the container, or as a separate paper, booklet, card or folder in the container or provided in the container. In some embodiments, the instruction provides methods for administering the immune effector cells of the present invention for treating or preventing tumors, pathogen infections, immune diseases, or allogeneic transplantation or symptoms thereof. In some cases, the instruction provides a method for administering the immune effector cells of the present invention before, after, or simultaneously with the administration of a chemotherapeutic agent.

In some embodiments, the kit of the present invention also contains chemotherapeutic drugs. In some embodiments, sorafenib is included in the kit of the present invention.

Advantages described herein:
1. The immune effector cells modified by the chimeric antigen receptor described herein can effectively increase the survival and function of the immune effector cells in the tumor;
2. The immune effector cells modified by the chimeric antigen receptor described herein can also achieve better cell killing on solid tumors, and compared with the immune effector cells modified by the chimeric antigen receptor not co-expressing RUNX3, they have better killing effects on solid tumor cells *in vivo* and expansion performance *in vitro;*
3. The chimeric antigen receptor cells which co-express RUNX3 and target tumor antigens as described herein express cytokines, such as IL15 or IL18 or IL21, thereby further improving the anti-tumor ability;
4. The chimeric antigen receptor cells which co-express RUNX3 and target tumor antigens described herein are combined with sorafenib to have synergistic anti-tumor effects;

The present invention will be further explained below in conjunction with specific embodiments. It should be understood that these examples are only used to illustrate the present invention and not to limit the scope described herein. The experimental methods without specific conditions in the following examples usually follow the conventional conditions as described in for example, J. Sambrook et al., Molecular Cloning Experiment Guide, Third Edition, Science Press, 2002, or the conditions suggested by the manufacturer.

Exemplary antigen receptors described herein, including CARs, and methods for engineering and introducing the receptors into cells, may refer to, conetnts disclosed in for example, Chinese Patent Application Publication Nos. CN107058354A, CN107460201A, CN105194661A, CN105315375A, CN105713881A, CN106146666A, CN106519037A, CN106554414A, CN105331585A, CN106397593A, CN106467573A, CN104140974A, International Patent Application Publication No. WO2018006882A1, WO2015172339A8, WO2018/018958A1.

### Example 1. Construction of T cells expressing chimeric antigen receptors

### 1. Construction of PRRLSIN-hu9F2-28Z, PRRLSIN-hu9F2-BBZ, MSCV-hu8E5-2I-mBBZ plasmid and lentivirus

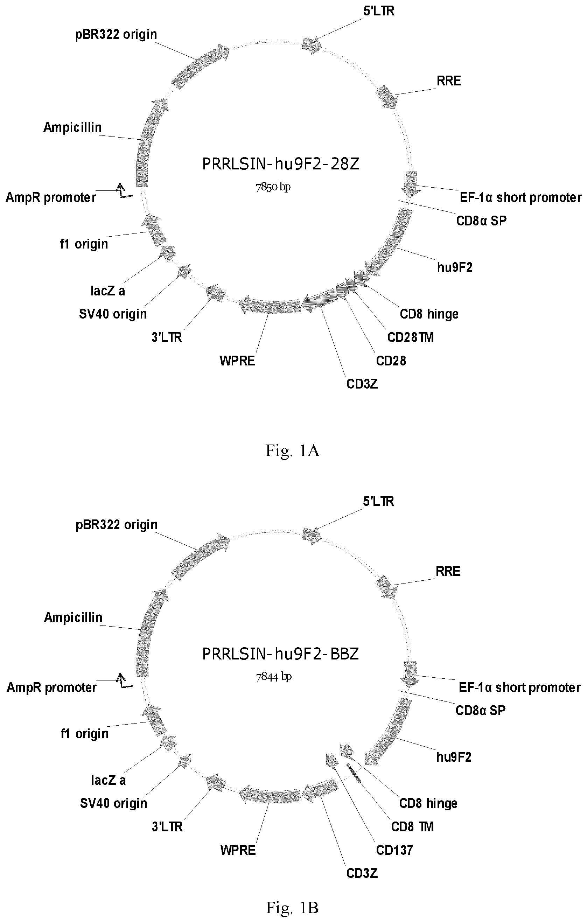

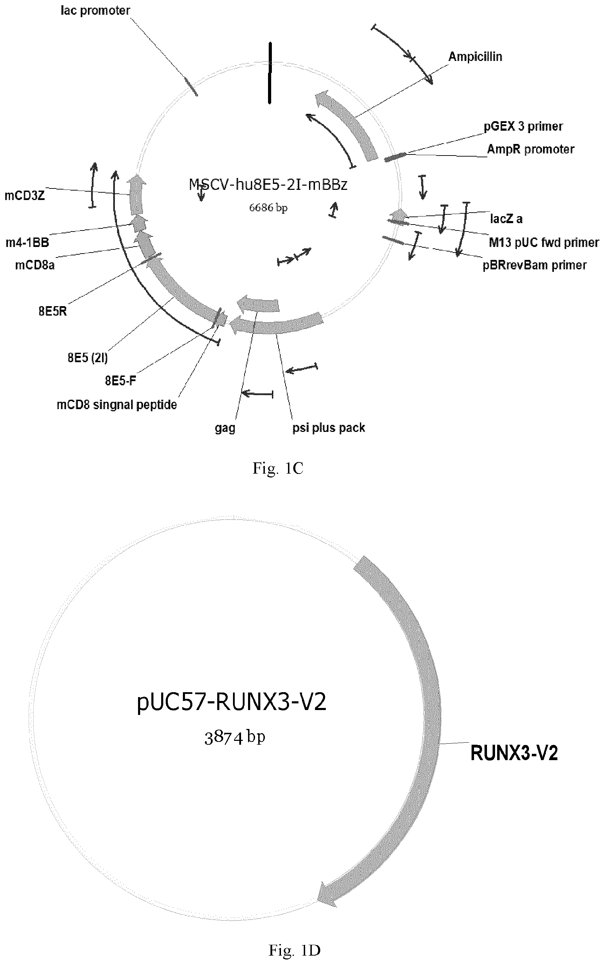

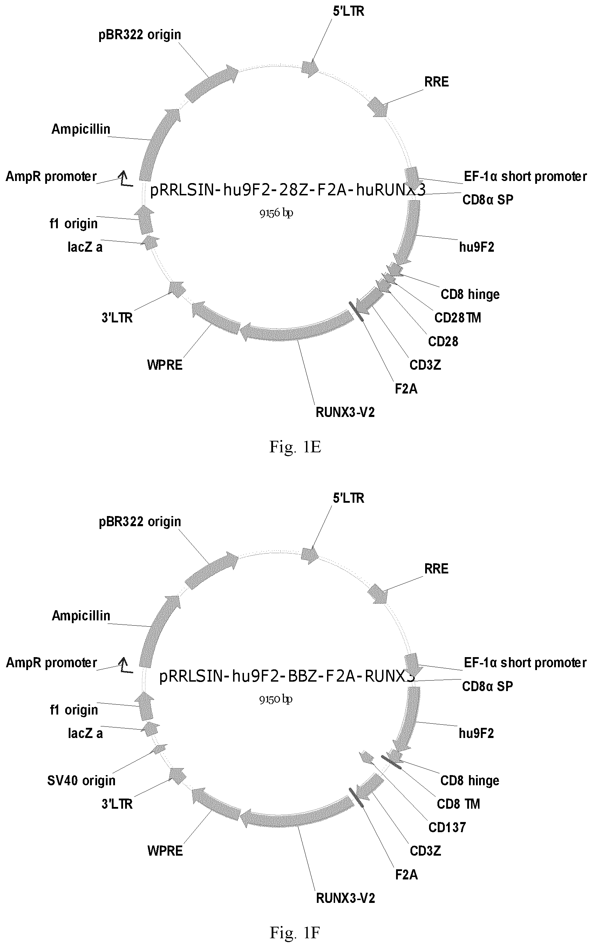

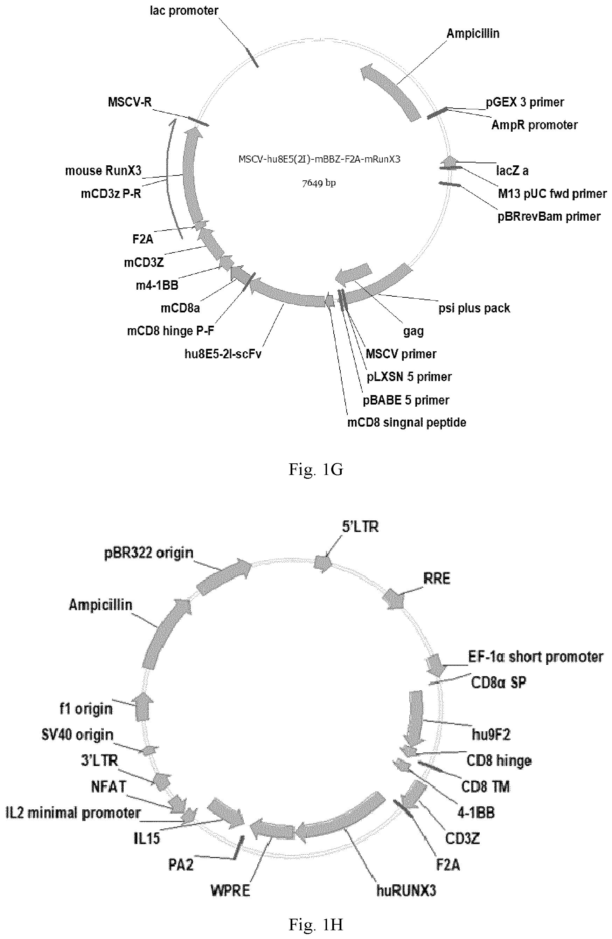

pRRLSIN-cPPT.EF-1α was used as a vector to construct lentiviral plasmids, PRRLSIN-hu9F2-28Z (see Figure 1A for the plasmid map) and PRRLSIN-hu9F2-BBZ (see Figure 1B for the plasmid map) expressing the second-generation of chimeric antigen receptor of the humanized antibody hu9F2 by conventional molecular biology methods.

The hu9F2-28Z sequence (SEQ ID NO: 57) was composed of CD8α signal peptide (nucleotide sequence as shown in SEQ ID NO: 1, amino acid sequence as shown in SEQ ID NO: 43), hu9F2 scFV (nucleotide sequence as shown in SEQ ID NO: 2, amino acid sequence as shown in SEQ ID NO: 42), CD8 hinge (nucleotide sequence as shown in SEQ ID NO: 3, amino acid sequence as shown in SEQ ID NO: 44), CD28 transmembrane domain (nucleotide sequence as shown in SEQ ID NO: 4, amino acid sequence as shown in SEQ ID NO: 45), CD28 intracellular domain (nucleotide sequence as shown in SEQ ID NO: 5, amino acid sequence as shown in SEQ ID NO: 46) and intracellular segment CD3ξ of CD3 (nucleotide sequence as shown in SEQ ID NO: 6, amino acid sequence as shown in SEQ ID NO: 47). PRRLSIN-hu9F2-28Z was transfected into 293T cells to package lentivirus so as to obtain lentivirus hu9F2-28Z.

The sequence of hu9F2-BBZ (SEQ ID NO: 58) consisted of CD8α signal peptide, hu9F2 scFV, CD8 hinge, CD8 transmembrane domain (nucleotide sequence as shown in SEQ ID NO: 7, amino acid sequence as shown in SEQ ID NO: 48), CD137 intracellular signaling domain (nucleotide sequence as shown in SEQ ID NO: 8 and amino acid sequence as shown in SEQ ID NO: 49) and intracellular segment CD3ξ of CD3. PRRLSIN-hu9F2-BBZ was transfected into 293T cells to package lentivirus so as to obtain lentivirus hu9F2-BBZ.

A retroviral plasmid MSCV-hu8E5-2I-mBBZ (see Figure 1C for the plasmid map) expressing the second-generation of chimeric antigen receptor was constructed by using MSCV.pBABE 5 as a vector. The sequence of hu8E5-2I-mBBZ consisted of mouse CD8α signal peptide (nucleotide sequence as shown in SEQ ID NO: 27, amino acid sequence as shown in SEQ ID NO: 28), scFv targeting claudin 18.2 (nucleotide sequence as shown in SEQ ID NO: 21, amino acid sequence as shown in SEQ ID NO: 22), mouse CD8 hinge and transmembrane region (nucleotide sequence as shown in SEQ ID NO: 29, amino acid sequence as shown in SEQ ID NO: 30), murine 4-1BB intracellular signaling domain (nucleotide sequence as shown in SEQ ID NO: 31, amino acid sequence as shown in SEQ ID NO: 32) and intracellular segment CD3ζ of mouse CD3 (nucleotide sequence as shown in SEQ ID NO: 33, amino acid sequence as shown in SEQ ID NO: 34). MSCV-hu8E5-2I-mBBZ was transfected into 293T cells to packaging retrovirus so as to obtain retrovirus hu8E5-2I-mBBZ.

### 2. Construction of pRRLSIN-hu9F2-28Z-F2A-huRUNX3, pRRLSIN-hu9F2-BBZ-F2A-huRUNX3, pRRLSIN-hu9F2-BBZ-F2A-huRUNX3-NFAT-IL15, pRRLSIN-hu9F2-BBZ-F2A-huRUNX3-NFAT-IL18, pRRLSIN-hu9F2-BBZ-F2A-huRUNX3-NFAT-IL21, MSCV-hu8E5-2I- mBBZ-F2A-mRunX3 plasmids

### 2.1 PCR-cloning target genes

pRRLSIN-hu9F2-28Z plasmid was used as a template, and primers GC33-28bbz-LF (SEQ ID NO: 9) & CD3Z-F2A-R (SEQ ID NO: 10) were used in amplification to obtain gene fragment F1 (SEQ ID NO: 11) (Hu9F2-28Z, with homology arm and part of F2A sequence);

pUC57-RUNX3-V2 plasmid (Figure ID) was used as a template, primers F2A-RUNX3-F (SEQ ID NO: 12) & RUNX-SalI-R (SEQ ID NO: 13) were used to amplify gene fragment F2 (SEQ ID NO: 14) (RUNX3 with a homology arm); and gene fragment F2 was used as a template, and primers F2A-F (SEQ ID NO: 15) & RUNX-SalI-R (SEQ ID NO: 13) were used to amplify gene fragment F3 (SEQ ID NO : 16) (F2A-RUNX3 with homology arm);

Fragments F1 and F3 were used as templates for conducting PCR-bridge to splice the full length, and primers GC33-28bbz-LF (SEQ ID NO: 9) & RUNX-SalI-R (SEQ ID NO: 13) were used in amplification to obtain the full-length gene fragment hu9F2- 28Z-F2A-huRUNX3 (SEQ ID NO: 17) (Figure IE);

pRRLSIN-hu9F2-BBZ plasmid was used as a template, and primers GC33-28bbz-LF (SEQ ID NO: 9) & CD3Z-F2A-R (SEQ ID NO: 10) were used to amplify gene fragment F4 (SEQ ID NO: 18) (hu9F2- BBZ, with the homology arm and part of F2A sequence);

Fragments F4 and F3 were used as templates for conducting PCR-bridge to splice the full length, and primers GC33-28bbz-LF (SEQ ID NO: 9) & RUNX-SalI-R (SEQ ID NO: 13) were used in amplification to obtain the full-length gene fragment hu9F2- BBZ-F2A-huRUNX3 (SEQ ID NO: 19 (Figure IF).

### 2.2 Obtaining vectors and fragments by enzyme-digestion

Vector pRRLSIN-hu9F2-28Z was double-digested with restriction enzymes MluI & SalI to obtain the linearized vector pRRLSIN-MluI&SalI.

Homologous recombinase was used to circularize the vector pRRLSIN-MluI&SalI and the fragment hu9F2-28Z-F2A-huRUNX3 to form the plasmid pRRLSIN-hu9F2-28Z-F2A-huRUNX3. 293T cells were transfected to package lentivirus, so as to obtain lentivirus hu9F2-28Z-RUNX3.

Homologous recombinase was used to circularize the vector pRRLSIN-MluI&SalI and the fragment hu9F2-BBZ-F2A-huRUNX3 to form the plasmid pRRLSIN-hu9F2-BBZ-F2A-huRUNX3. 293T cells were transfected to package lentivirus, so as to obtain lentivirus hu9F2-BBZ-RUNX3.

F2A-mRunx3 sequence was inserted into the MSCV-hu8E5-2I-mBBZ plasmid, and the second-generation of chimeric antigen receptor targeting Claudin 18.2 and the corresponding retroviral plasmid MSCV-hu8E5-2I-mBBZ-F2A-mRunX3 (Figure G) were constructed. F2A-mRunX3 was composed of F2A (nucleotide sequence as shown in SEQ ID NO: 23, amino acid sequence as shown in SEQ ID NO: 24), mouse RUNX3 (nucleotide sequence as shown in SEQ ID NO: 25, amino acid sequence as shown in SEQ ID NO: 26). MSCV-hu8E5-2I-mBBZ-F2A-mRunX3 was transfected into 293T cells to package retrovirus, so as to obtain retrovirus hu8E5-2I-mBBZ-RUNX3.

6 consecutive NFAT (SEQ ID NO: 37) and IL15 (nucleotide sequence as shown in SEQ ID NO: 38, amino acid sequence as shown in SEQ ID NO: 39) were inserted into pRRLSIN-hu9F2-BBZ-F2A-huRUNX3 plasmid to construct the plasmid hu9F2-BBZ-RUNX3-IL15 (see Figure 1H for the plasmid map). 293T cells were transfected to package lentivirus, so as to obtain lentivirus hu9F2-BBZ-RUNX3-IL15.

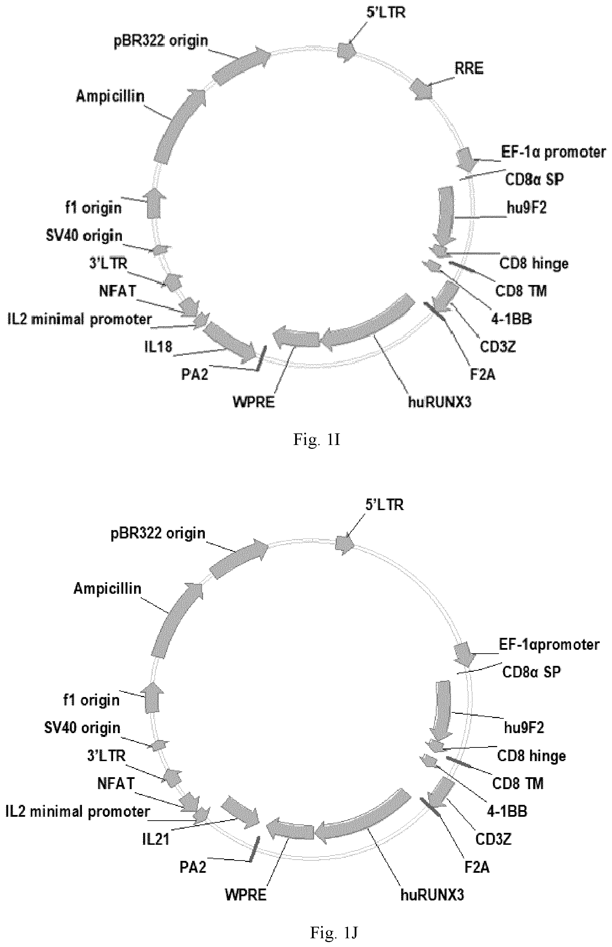

6 consecutive NFAT (SEQ ID NO: 37) and IL18 (nucleotide sequence as shown in SEQ ID NO: 40, amino acid sequence as shown in SEQ ID NO: 41) were inserted into pRRLSIN-hu9F2-BBZ-F2A-huRUNX3 plasmid to construct the plasmid hu9F2-BBZ-huRUNX3-IL18 (see Figure 1I for the plasmid map). 293T cells were transfected to package lentivirus, so as to obtain lentivirus hu9F2-BBZ-huRUNX3-IL18.

6 consecutive NFAT (SEQ ID NO: 37) and IL21 (nucleotide sequence as shown in SEQ ID NO: 35, amino acid sequence as shown in SEQ ID NO: 36) were inserted into pRRLSIN-hu9F2-BBZ-F2A-huRUNX3 plasmid to construct the plasmid pRRLSIN-hu9F2-BBZ-F2A-huRUNX3-NFAT-IL21 (see Figure 1J for the plasmid map). 293T cells were transfected to package lentivirus, so as to obtain lentivirus hu9F2-BBZ-huRUNX3 -IL21.

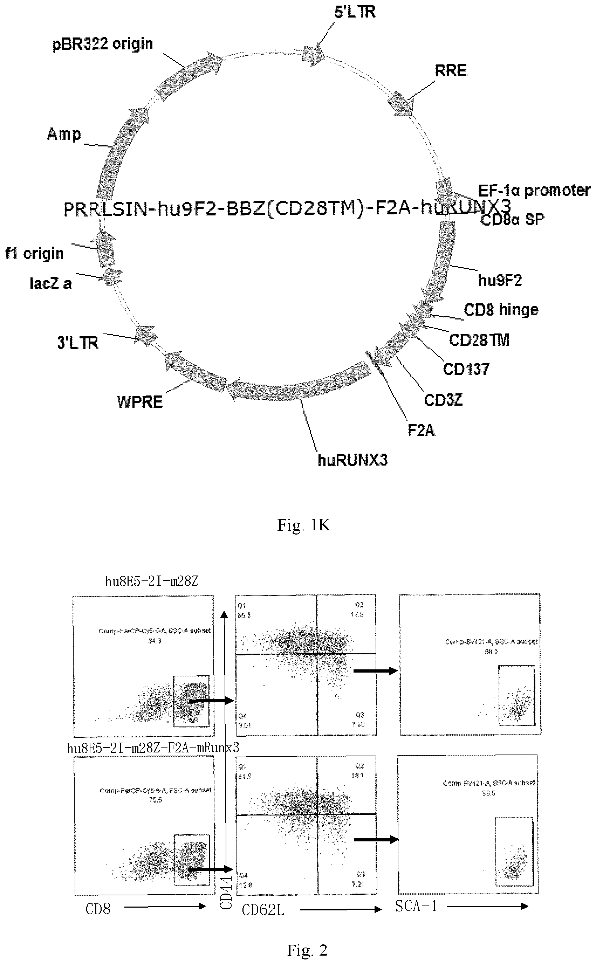

pRRLSIN-cPPT.EF-1α was used as a vector to construct a lentiviral plasmid, PRRLSIN-hu9F2-BBZ(CD28TM)-F2A-huRUNX3 (see Figure 1K for the plasmid map) by the same method as said above. The sequence of hu9F2-BBZ(CD28TM)-F2A-huRUNX3 consisted of CD8α signal peptide, hu9F2 scFV, CD8 hinge, CD28 transmembrane domain, CD137 intracellular signal domain, intracellular segment CD3ξ of CD3 and F2A (nucleotide sequence as shown in SEQ ID NO: 23, amino acid sequence as shown in SEQ ID NO: 24), human RunX3 (nucleotide sequence as shown in SEQ ID NO: 50, amino acid sequence as shown in SEQ ID NO: 20). PRRLSIN-hu9F2-BBZ(CD28TM)-F2A-huRUNX3 was transfected into 293T cells to package lentivirus, so as to obtain lentivirus hu9F2-BBZ(CD28TM)-RUNX3.

### 2.3 Activation of T cells:

Human PBMCs were taken and cultured in AIM-V medium with 2% human AB type serum, 500 U/mL recombinant human IL-2, and CD3/CD28 antibody combined with magnetic beads for activation for 48 h. The activated T cells were infected by the obtained lentiviruses hu9F2-28Z, hu9F2-BBZ, hu9F2-28Z-RUNX3, hu9F2-BBZ-RUNX3, hu9F2-BBZ-RUNX3-IL15, hu9F2-BBZ-huRUNX3-IL18, hu9F2-BBZ-huRUNX3 -IL21, hu9F2-BBZ(CD28TM)-RUNX3, respectively to obtain 9F2-28Z CART cells, 9F2-BBZ CART cells, 9F2-28Z-RUNX3 CART cells, 9F2-BBZ-RUNX3 CART cells, 9F2-BBZ-RUNX3-NFAT-IL15 CART cells, 9F2-BBZ-RUNX3-NFAT-IL18 CART cells, 9F2-BBZ-RUNX3-NFAT-IL21 CART cells, 9F2-BBZ (CD28TM)-RUNX3 CART cells.

Spleen T lymphocytes of C57BL/6 mice were taken, the purified mouse CD3⁺ T lymphocytes were added to Dynabeads Mouse T-activator CD3/CD28 at a volume ratio of 1:1, washed once with PBS, activated, and cultured in an incubator. The medium was RPMI 1640 complete medium with 10% FBS serum. Mouse spleen T lymphocytes activated for 24 hours were inoculated into a 12-well plate coated with recombinant human fibrin fragments, and retroviruses hu8E5-2I-mBBZ and hu8E5-2I-mBBZ-RUNX3 were added respectively for infection for 12 hours, and then cultured and expanded to the required number, so as to obtain hu8E5-2I-mBBZ CAR T cells and hu8E5-2I-mBBZ-mRunX3 CAR T cells.

### Example 2. In vitro detection of CAR-T cell phenotype

According to different cell phenotypes, memory T cells can be divided into two types: central memory T cells (Tcm) and effector memory T cells (Tem). Central memory T cells are similar to naive T cells and highly express the lymphatic homing molecule L-selectin (CD62L). Memory stem cell-like T cells are a group of CD8+ T cells, which have low expression of hyaluronan receptor CD44 and high expression of CD62L and stem cell antigen Sca-1.

hu8E5-2I-mBBZ CART cells and hu8E5-2I-mBBZ-RunX3 CAR-T cells infected for four days were taken for Tcm (central memory T cells) and Tscm (memory stem cell-like T cells) detection, Flow cytometry detection was performed using antibody staining and the results are shown in Figure 2.

It can be seen from the flow cytometry results in Figure 2 that, in CD8-positive cells of different CAR-T cells, there is no significant difference in the proportions of Tcm expressing CD62L and CD44, as well as Tscm expressing low expression of CD44 and high expression of CD62L and Sca-1.

### Example 3. In vitro killing toxicity test targeting GPC3 positive cells

CytoTox 96 non-radioactive cytotoxicity detection kit (Promega) was used. The specific method refers to the CytoTox 96 non-radioactive cytotoxicity detection kit instructions.

### 1. CAR T cells expressing RUNX3

Effector cells: Untransduced (UTD) T cells, 9F2-28Z CART cells, 9F2-BBZ CART cells, 9F2-28Z-RUNX3 CART cells, 9F2-BBZ-RUNX3 CART cells were inoculated in 96-well plates according to the effector target ratio of 3: 1, 1: 1 or 1: 3.

Target cells: 50 µL of 2×10⁵/mL (GPC3 expression positive) Huh7 and PLC/PRF/5 cells, (GPC3 expression negative) SK-HEP-1 cells were inoculated in the corresponding 96-well plates, respectively.

5 replicate wells were set for each group. The culture plates were placed in a cell culture box and incubated for 18 hours.

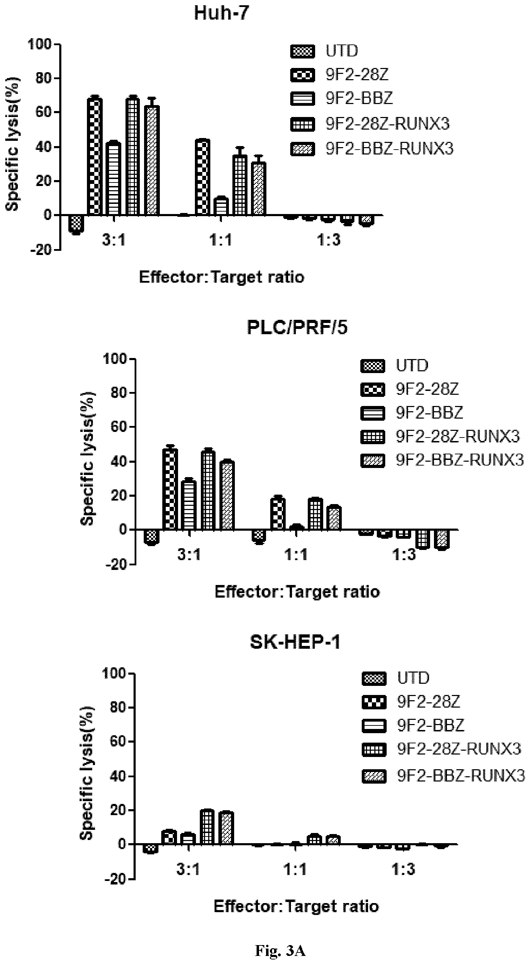

Each experimental group and each control group were set as follows: experimental group: each target cell + T lymphocytes expressing different chimeric antigen receptors; Control group 1: maximum release of LDH by target cell; Control group 2: spontaneous release of LDH by target cell; Control group 3: spontaneous release of LDH by effector cells. The calculation formula is: % cytotoxicity = [(experimental group - spontaneous release by effector cell - spontaneous release by target cell) / (maximum release by target cell - spontaneous release by target cell)] ^{∗} 100. The experimental results are shown in Figure 3A.

Compared with the UTD in the control group, the above CAR-T exhibited significant toxic killing effects on Huh7 and PLC/PRF/5 cells with positive GPC3 expression at an effector target ratio of 3:1 and 1:1, and at an effector target ratio of 1:3, there was basically no killing effect. There was almost no killing effect on SK-HEP-1 cells negative for GPC3 expression. The killing effects of 9F2-28Z-RUNX3 CART group and 9F2-BBZ-RUNX3 CART group were comparable to thoes of 9F2-28Z CART group; but both were significantly better than those of 9F2-BBZ CART group.

### 2. CAR T cells expressing RUNX3 in combination with cytokines

Effector cells: Untransduced (UTD) T cells, 9F2-BBZ CART cells, 9F2-BBZ-RUNX3 CART cells, 9F2-BBZ-RUNX3-NFAT-IL15 CART cells, 9F2-BBZ-RUNX3-NFAT-IL18 CART cells, 9F2-BBZ-RUNX3-NFAT-IL21 CART cells were inoculated in 96-well plates according to the effector target ratio of 3: 1, 1: 1 or 1: 3.

Target cells: 50 µL of 2×10⁵/mL (GPC3 expression positive) Huh7 and PLC/PRF/5 cells, (GPC3 expression negative) SK-HEP-1 cells were inoculated in the corresponding 96-well plates, respectively.

5 replicate wells were set for each group. The culture plates were placed in a cell culture box and incubated for 18 hours.

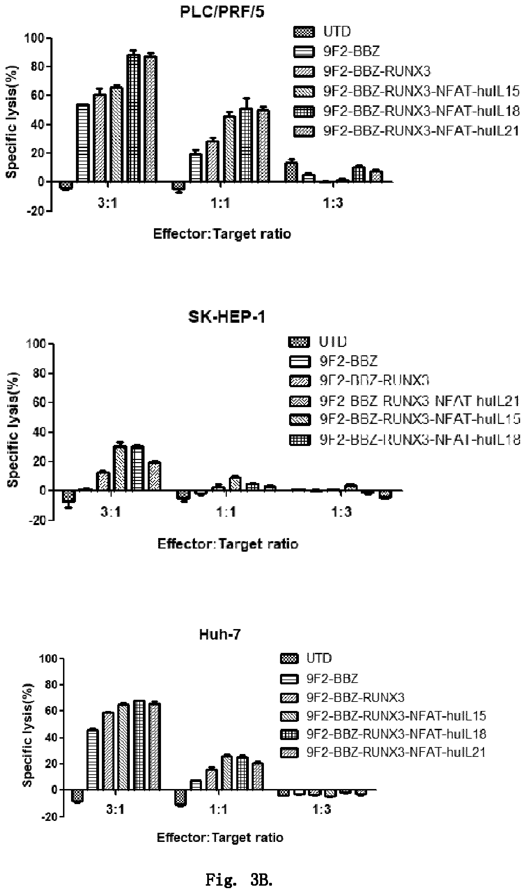

Each experimental group and each control group were set as follows: experimental group: each target cell + T lymphocytes expressing different chimeric antigen receptors; Control group 1: maximum release of LDH by target cell; Control group 2: spontaneous release of LDH by target cell; Control group 3: spontaneous release of LDH by effector cells. The calculation formula is: % cytotoxicity = [(experimental group - spontaneous release by effector cell - spontaneous release by target cell) / (maximum release by target cell - spontaneous release by target cell)] ^{∗} 100. The experimental results are shown in Figure 3B.

Compared with UTD in the control group, for huh7 with high expression of GPC3, the CAR T cell group expressing IL15, IL18 and IL21 in combination is better than 9F2-BBZ-RUNX3 CAR T cell group, when the effector target ratio is 3:1 and 1:1; and for PLC/PRF/5 with moderate expression of GPC3, the CAR-T cell group expressing IL-18 and IL-21 in combination is better than other cell groups, when the effector target ratio is 3:1 and 1:1.

### Example4. In vitro killing toxicity test targeting claudin18.2 positive cells

The specific method may refer to Example 3.

Effector cells: Untransduced (UTD) T cells, hu8E5-2I-mBBZ CART cells, hu8E5-2I-mBBZ-mRunX3 CAR-T cells were inoculated in 96-well plate according to the effector target ratio of 3: 1, 1: 1 or 1: 3.

Target cells: 50 µL of 2×10⁵/mL mouse pancreatic cancer cell line (claudin18.2 expression positive) and PANC02 (claudin18.2 expression negative) cells were inoculated in the corresponding 96-well plates, respectively.

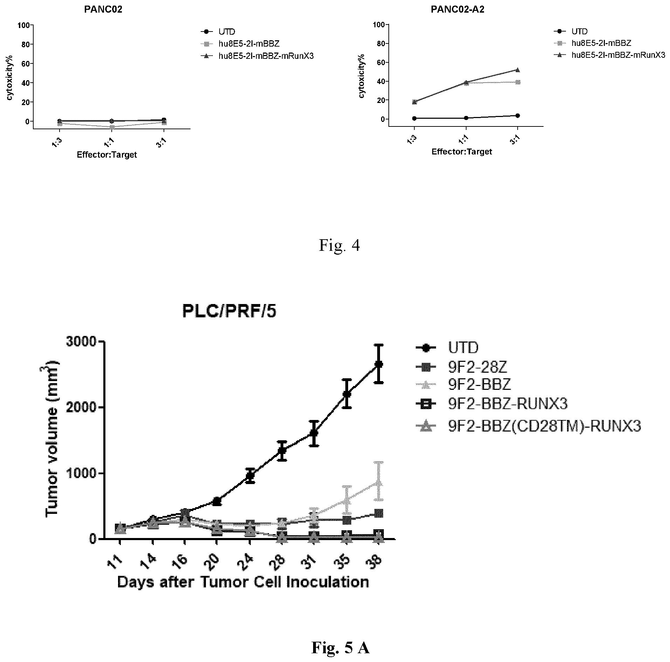

The results of the experiment were shown in Figure 4. Compared with hu8E5-2I-mBBZ CART cells, CAR-T cells expressing RUNX3 exhibited significant toxic killing effects on PANC02-A2 with positive expression of claudin 18.2 at an effector target ratios of 3:1 and 1:1, but almost no killing effects on PANC02 cells with negative expression of claudin 18.2.

### Example 5. Anti-tumor treatment experiment on subcutaneous xenograft of GPC3 positive cells

### 1. PLC/PRF/5 liver cancer cell small-load subcutaneous xenograft model

1) Experimental groups: B-NDG mice (BIOCYTOGEN) of 6-8 weeks old were randomly divided into 5 groups (n = 5), namely Untransduced (UTD), control group of 9F2-28Z CART cell, control group of 9F2-BBZ CART cell, treatment group of 9F2-BBZ(CD28TM)-RUNX3 CART cell, treatment group of 9F2-BBZ-RUNX3 CART cell.
2) Inoculation of subcutaneous xenograft: PLC/PRF/5 cells in the logarithmic growth phase and in good growth condition were collected by Trypsin digestion and washed once with normal saline. The cell density was adjusted to 1.5×10⁷/mL and 200 µL of cell suspension was injected into the subcutaneous part of the right abdomen of B-NDG mice using a syringe, that is, each mouse was inoculated with 3 × 10⁶ tumor cells, and the inoculation day was the 0^{th} day.
3) Reinfusion of CAR-T cells: When the average tumor volume was about 158 mm³, that is, on the 11^{th} day after tumor inoculation, 2.5×10⁶ CAR-T cells or untransduced T cell control were injected.

The changes in the tumor volume were recorded. The calculation formula of tumor volume is: tumor volume = (tumor length × tumor width²) / 2, and the result is shown in Figure 5A.

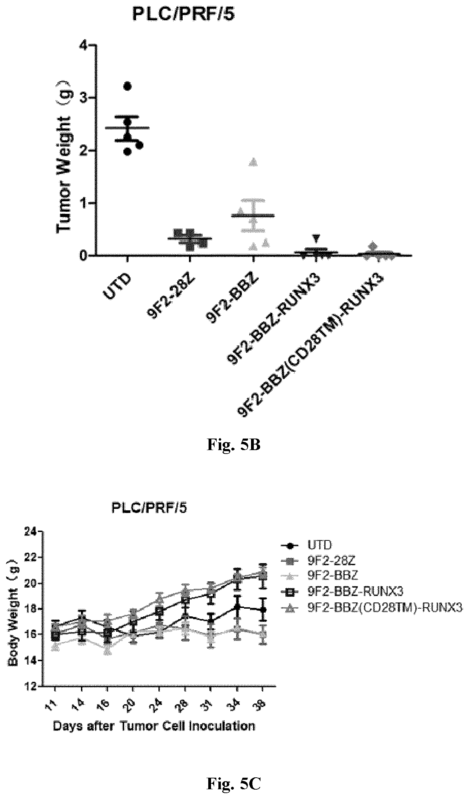

The mice were euthanized, the subcutaneous tumor was stripped, and the tumor weight was weighed. The results are shown in Figure 5B.

The tumor inhibiting rate was calculated with reference to the control group. 27 days after CAR T injection, compared with the UTD control group, each group showed obvious tumor-inhibiting effects. The inhibiting rates were: 9F2-28Z CART group: 85.09%, 9F2-BBZ CART group : 66.78%, 9F2-BBZ-RUNX3 CART group: 97.71% (4 out of 5 mice showed tumor regression), 9F2-BBZ(CD28TM)-RUNX3 CART group: 98.83% (4 out of 5 mice showed tumor regression).

After administration of CAR-T cells, the curve of changes the in body weight of mice in each group is shown in Figure 5C. Compared with UTD, the body weight of mice in the 9F2-28Z and 9F2-BBZ groups was slightly lower, and the weight of mice in 9F2-BBZ(CD28TM)-RUNX3 and 9F2-BBZ-RUNX3 treatment groups showed a growth advantage.

### 2. PLC/PRF/5 liver cancer cell heavy-load subcutaneous xenograft model

1) Experimental groups: NPG (Vitonda) mice of 6-8 weeks old are randomly divided into 5 groups (n = 5), namely Untransduced (UTD), control group of 9F2-28Z CART cell, control group of 9F2-BBZ CART cell, treatment group of 9F2-28Z-RUNX3 CART cell, and treatment group of 9F2-BBZ-RUNX3 CART cell.
2) Inoculation of subcutaneous xenograft: PLC/PRF/5 cells in the logarithmic growth phase and in good growth condition were collected by Trypsin digestion and washed once with normal saline. The cell density was adjusted to 1.5×10⁷/mL and 200 µL of cell suspension was injected into the subcutaneous part of the right abdomen of NPG mice using a syringe, that is, each mouse was inoculated with 3 × 10⁶ tumor cells, and the inoculation day was the 0^{th} day.
3) Reinfusion of CAR-T cells: When the average tumor volume was about 260 mm³, that is, on the 14^{th} day after tumor inoculation, 2.0×10⁶ CAR-T cells or untransduced T cell control were injected.

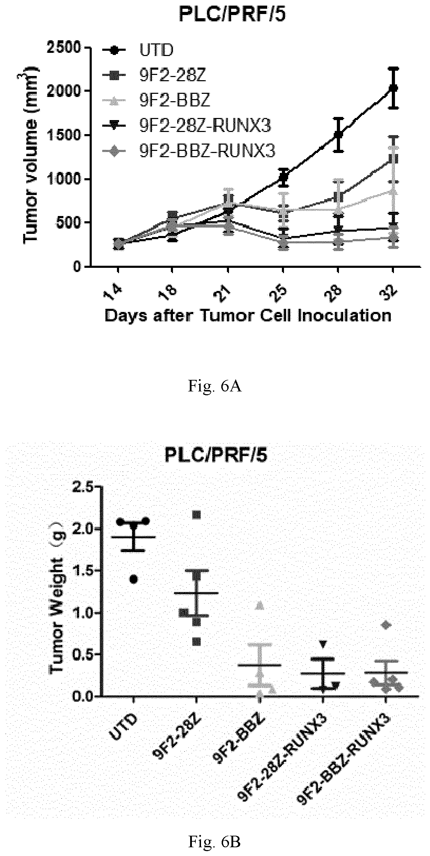

The changes in the volume of mouse subcutaneous tumors were measured and recorded twice a week. The calculation formula of tumor volume is: tumor volume = (tumor length × tumor width²) / 2, and the result is shown in Figure 6A.

As showed in 6B. The tumor inhibiting rate was calculated with reference to the control group. On 18 days after CAR T injection, compared with the UTD control group, each group showed obvious tumor-inhibiting effects. The inhibiting rates were: 9F2-28Z CART group: 40.50%, 9F2-BBZ CART group: 56.95%, 9F2-28Z-RUNX3 CART group: 77.69%, 9F2-BBZ-RUNX3 CART group: 83.68%.

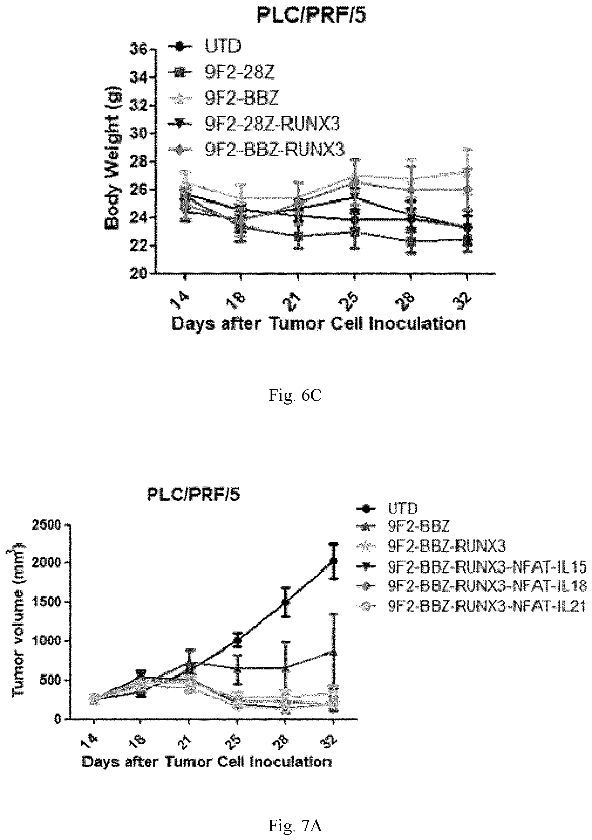

After administration of CAR-T cells, the curve of changes the in body weight of mice in each group is shown in Figure 6C. Compared with UTD, the body weight of mice in the 9F2-28Z group was slightly lower, and the weight of mice in 9F2-BBZ(CD28TM)-RUNX3 and 9F2-BBZ-RUNX3 treatment groups showed a growth advantage.

### Example 6. Anti-tumor therapy experiment in combination with cytokine on subcutaneous xenograft of GPC3 positive cells

### PLC/PRF/5 subcutaneous xenograft model

1) Experimental groups: NPG mice of 6-8 weeks old are randomly divided into 6 groups (n = 5), namely Untransduced (UTD), control group of 9F2-BBZ CART cells, treatment group of 9F2-BBZ-RUNX3 CART cells, treatment group of 9F2-BBZ-RUNX3-NFAT-IL15 CART cells, treatment group of 9F2-BBZ-RUNX3-NFAT-IL18 CART cells, treatment group of 9F2-BBZ-RUNX3- NFAT-IL21 CART cells.
2) Inoculation of subcutaneous xenograft: PLC/PRF/5 cells in the logarithmic growth phase and in good growth condition were collected by Trypsin digestion and washed once with normal saline. The cell density was adjusted to 1.5×10⁷/mL and 200 µL of cell suspension was injected into the subcutaneous part of the right abdomen of NPG mice using a syringe, that is, each mouse was inoculated with 3 × 10⁶ tumor cells, and the inoculation day was the 0^{th} day.
3) Injection of CAR T: On D14 days after subcutaneous inoculation of tumor cells, the average tumor volume was about 260 mm³. CAR T cells were injected, and the injection dose: 2.0×10⁶/animal.

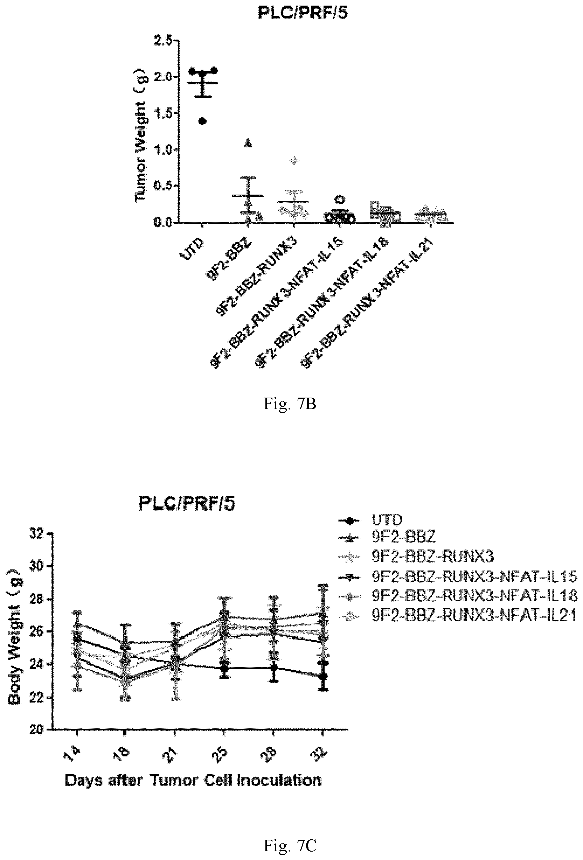

On D18 days after the injection of CAR T, the tumor volume in the UTD group reached 2000 mm³, and the mice were euthanized. Figures 7A and 7B showed that, compared with the second-generation of CAR T, CAR T carrying RUNX3 and cytokines exhibited certain therapeutic advantages. Figure 7B showed the weight of the tumor removed from the mouse. Compared with the UTD control group, the tumor inhibiting rate of each group was: 9F2-BBZ: 56.95%, 9F2-BBZ-RUNX3: 83.68%, 9F2-BBZ-RUNX3-NFAT-IL15: 90.25% (1 out of 5 mice showed tumor regression), 9F2-BBZ-RUNX3-NFAT-IL18: 90.64%, 9F2-BBZ-RUNX3-NFAT-IL21: 90.34%.

It shows that in the CAR T cell groups carrying both of RUNX3 and the cytokine IL15, IL18 or IL21 exhibited the best anti-tumor effects. In the CAR T cell group carrying both of RUNX3 and the cytokine IL15, 1 out of 5 mice showed tumor regression; the anti-tumor effects of CAR T cell group only carrying RUNX3 were the second.

Figure 7C showed that, compared with UTD, the body weight of mice in each treatment group exhibited a certain growth advantage, which may benefit from the better tumor inhibiting effects of CAR T.

### Example 7. Anti-tumor therapy targeting subcutaneous xenograft of claudin18.2 positive cells

### PANC02-A2 subcutaneous xenograft model

1) Experimental groups: C57BL/6 mice of 6-8 weeks old are randomly divided into several groups (n = 5-6), namely Untransduced (UTD) T cells, treatment group of hu8E5-2I-mBBZ CART cells, treatment group of 8E5-2I-mBBZ-mRunX3 CAR-T (or named as mBBZ-mRunX3) cells.
2) Inoculation of subcutaneous xenograft: PANC02-A2 cells in the logarithmic growth phase and in good growth condition were collected by Trypsin digestion and washed once with normal saline. The cell density was adjusted to 6×10⁶/mL and 200 µL of cell suspension was injected into the subcutaneous part of mouse right axillary of C57BL/6 mice using a syringe, that is, each mouse was inoculated with 1.2×10⁶ tumor cells, and the inoculation day was the 0^{th} day.
3) Injection of CAR T: On D11 days after subcutaneous inoculation of tumor cells, the average tumor volume was about 150 mm³. CAR T cells were injected, and the injection dose: 5×10⁶/animal.

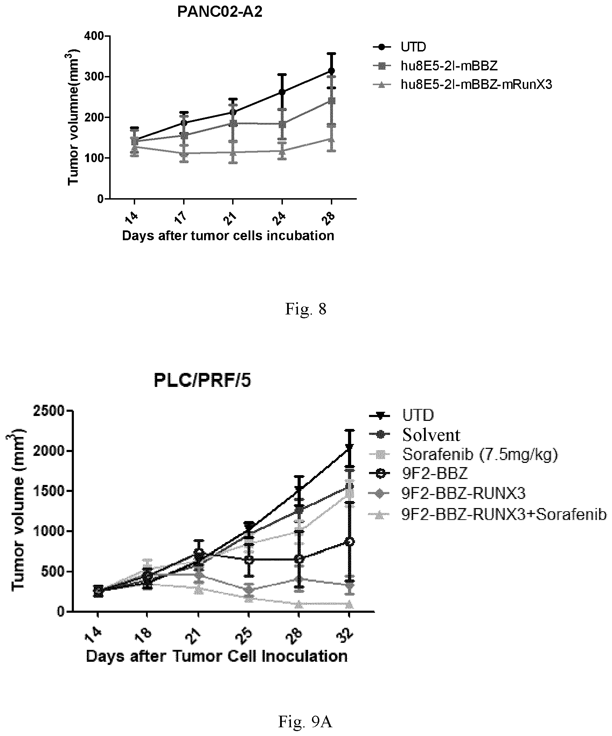

The curve of tumor volume was shown in Figure 8, showing that co-expression of RUNX3 showed better anti-tumor effects.

The tumor inhibiting rate was calculated for each group. 14 days after CAR T injection, compared with the UTD control group, each group showed obvious tumor-inhibiting effects. The inhibiting rates were: hu8E5-2I-mBBZ CART group: 38.08%, hu8E5-2I-mBBZ-mRunX3 CART group: 51.8%.

### Example 8. Anti-tumor therapy experiment in combination with Solafenib on subcutaneous xenograft of GPC3 positive cells

### PLC/PRF/5 subcutaneous xenograft model

1) Experimental groups: NPG mice of 6-8 weeks old are randomly divided into 6 groups (n = 5), namely Untransduced (UTD), solvent group, Sorafenib group, 9F2-BBZ CART cell group, 9F2-BBZ-RUNX3 CART cell group, combined treatment group of 9F2-BBZ-RUNX3 + Sorafenib.
2) Inoculation of subcutaneous xenograft: PLC/PRF/5 cells in the logarithmic growth phase and in good growth condition were collected by Trypsin digestion and washed once with normal saline. The cell density was adjusted to 1.5×10⁷/mL and 200 µL of cell suspension was injected into the subcutaneous part of the right abdomen of NPG mice using a syringe, that is, each mouse was inoculated with 3 × 10⁶ tumor cells, and the inoculation day was the 0^{th} day.
3) Injection of CAR T: On D14 days after subcutaneous inoculation of tumor cells, the average tumor volume was about 260 mm³. CAR T cells were injected, and the injection dose: 2.0×10⁶/animal. On D1-D14 days after subcutaneous inoculation of tumor cells, sorafenib was administered by gavage at a dose of 75 mg/kg, and the gavage volume was 200 ul, once a day, for 14 consecutive days, and the solvent was used as a negative control.

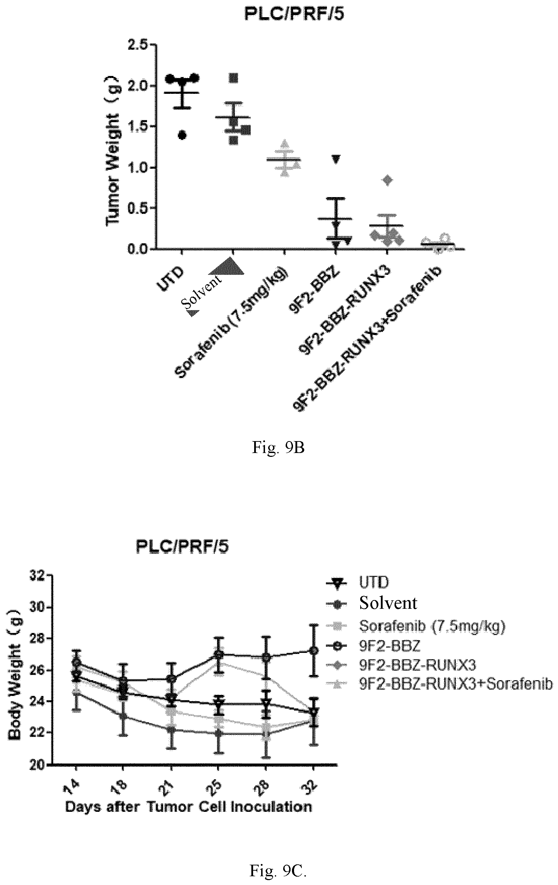

On D18 days after the injection of CAR T, the tumor volume in the UTD group reached 2000 mm³, and the mice were euthanized. Figures 9A and 9B showed that the treatment group of 9F2-BBZ-RUNX3 and treatment group of 9F2-BBZ-RUNX3 combined with Sorafenib exhibited obvious tumor killing effects, and Figure 9C showed the weight of the tumors removed from the mice. Compared with the UTD control group, the 9F2-BBZ-RUNX3 + Sorafenib combined treatment group exhibited the most significant tumor-inhibiting effects. The tumor inhibiting rates were: solvent group: 23.37%, Sorafenib treatment group: 27.38%, 9F2-BBZ: 56.95%, 9F2 -BBZ-RUNX3: 83.68%, 9F2-BBZ-RUNX3 + Sorafenib treatment group: 95.45% (1 out of 5 mice showed tumor regression).

Compared with UTD, the body weight of mice in the solvent group and Sorafenib treatment group decreased at the beginning of the treatment. The solvent group and Sorafenib may have certain toxic and side effects on the mice, however, the body weight was restored at the end of the treatment. Compared with UTD, the 9F2-BBZ-RUNX3 + Sorafenib combined treatment group exhibited no significant changes. The 9F2-BBZ and 9F2-BBZ-RUNX3 treatment groups exhibited certain growth advantages in the body weight, which may benefit from better tumor-inhibiting effects on the CAR T.

### Example 9. In vitro detection of cytokine

Untransduced (UTD) T cells, 9F2-28Z CART cells, 9F2-BBZ CAR T cells, 9F2-28Z-RUNX3 CAR T cells, 9F2-BBZ-RUNX3 CAR T and liver cancer cell lines Huh-7, PLC/PRF/5 , SK-HEP-1 cells were incubated for 24 hours at an effector target ratio of 1:1, then the supernatant was collected, and the secretion levels of cytokines IL-2, TNF, IFN-γ in the supernatant were detected through flow cytometry by CBA method.

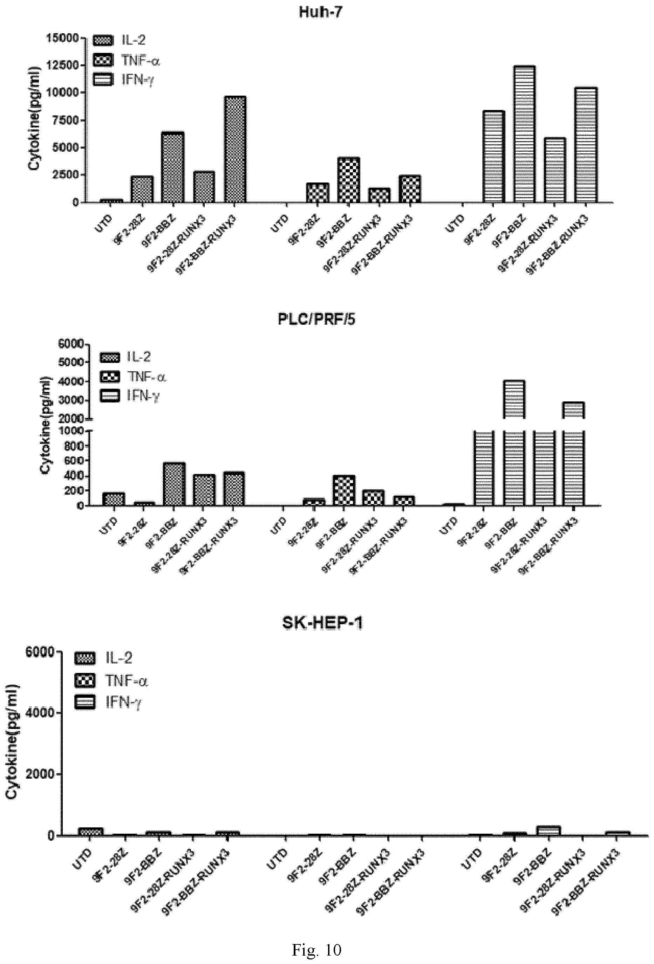

Figure 10 showed that the amount of IL-2, TNF, IFN-γ cytokines released from CAR T cells in the form of BBZ is better than that of CAR T cells in the form of 28Z (9F2-BBZ is better than 9F2-28Z; 9F2-BBZ-RUNX3 is better than 9F2-28Z-RUNX3). Moreover, the three cytokines IL-2, TNF and IFN-γ are highly released in GPC3-positive cells, wherein Huh-7 cells exhibited the highest release, and there was almost no release in GPC3-negative cells, SK-HEP-1 cells.

### Example 10. Anti-tumor therapy on Hepa1-6-GPC3 subcutaneous xenograft

### 1. Preparation of 9F2-mBBZ CAR T cells and 9F2-mBBZ-mRunX3 CAR T cells

9F2-mBBZ CAR T cells and 9F2-mBBZ-mRunX3 CAR-T cells were prepared according to Example 1.

MSCV.pBABE 5 was used as the vector to construct the retroviral plasmid MSCV-9F2-mBBZ. The 9F2-mBBZ sequence consists of mouse CD8α signal peptide (nucleotide sequence as shown in SEQ ID NO: 27, amino acid sequence as shown in SEQ ID NO: 28), scFv (nucleotide sequence as shown in SEQ ID NO: 2), mouse CD8 hinge and transmembrane region (nucleotide sequence as shown in SEQ ID NO: 29, amino acid sequence as shown in SEQ ID NO: 30) and murine 4-1BB intracellular signaling domain (nucleotide sequence as shown in SEQ ID NO: 31, the amino acid sequence as shown in SEQ ID NO: 32) and the intracellular segment CD3ζ of mouse CD3 (nucleotide sequence as shown in SEQ ID NO: 33, amino acid sequence as shown in SEQ ID NO: 34). MSCV-9F2-mBBZ was transfected into 293T cells to package retrovirus, so as to obtain retrovirus 9F2-mBBZ.

F2A-mRunx3 sequence was inserted into the MSCV-9F2-mBBZ plasmid, and the retroviral plasmid MSCV-9F2-mBBZ-mRunX3 expressing CAR and RUNX3 was constructed. F2A-mRunX3 consisted of F2A (nucleotide sequence as shown in SEQ ID NO: 23, amino acid sequence as shown in SEQ ID NO: 24), mouse RUNX3 (nucleotide sequence as shown in SEQ ID NO: 25, amino acid sequence as shown in SEQ ID NO: 26). MSCV-9F2-mBBZ-mRunX3 was transfected into 293T cells to package retrovirus, so as to obtain retrovirus 9F2-mBBZ-mRunX3.

T cells from C57BL/6 mice were taken and activated, and infected with retroviruses 9F2-mBBZ and 9F2-mBBZ-mRunX3, respectively, so as to obtain 9F2-mBBZ CAR T cells and 9F2-mBBZ-mRunX3 CAR T cells.

### 2. Killing effects on Hepa1-6-GPC3 subcutaneous xenograft model

1) Experimental groups: C57BL/6 mice of 6-8 weeks old were randomly divided into groups (n = 5-6), namely Untransduced (UTD) T cells, treatment group of 9F2-mBBZ CAR T cells, 9F2-mBBZ-mRunX3 CAR-T (or named as mBBZ-mRunX3) cells.
2) Inoculation of subcutaneous xenograft: Hepa1-6-GPC3 cells in the logarithmic growth phase and in good growth condition were collected by Trypsin digestion and washed once with PBS. The cell density was adjusted to 5×10⁷/mL and 200 µL of cell suspension was injected into the subcutaneous part of mouse right axillary of C57BL/6 mice using a syringe, that is, each mouse was inoculated with 1×10⁷ tumor cells, and the inoculation day was the 0^{th} day.
3) Reinfusion of CAR-T cells: On D6 day after tumor inoculation, the average tumor volume was about 300 mm³. CAR-T cells were injected, and the injection dose: 1.5×10⁶/animal.

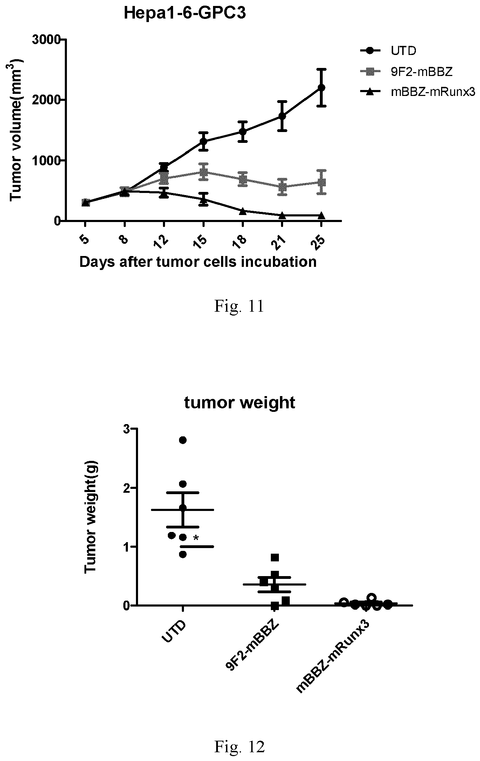

The changes in the volume of mouse subcutaneous tumors were measured and recorded twice a week. The calculation formula of tumor volume is: tumor volume = (tumor length × tumor width²) / 2, and the result is shown in Figure 11A.

The tumor-inhibiting rate was calculated with reference to the control group. On 20 days after CAR T injection, compared with the UTD control group, each group showed obvious tumor-inhibiting effects. The inhibiting rates were: 9F2-mBBZ CART group: 70.7%, 9F2-mBBZ-mRunX3 CART group: 95.8%.

The mice were euthanized, the subcutaneous tumor was removed, and the tumor weight was weighed. The results were shown in Figure 12.

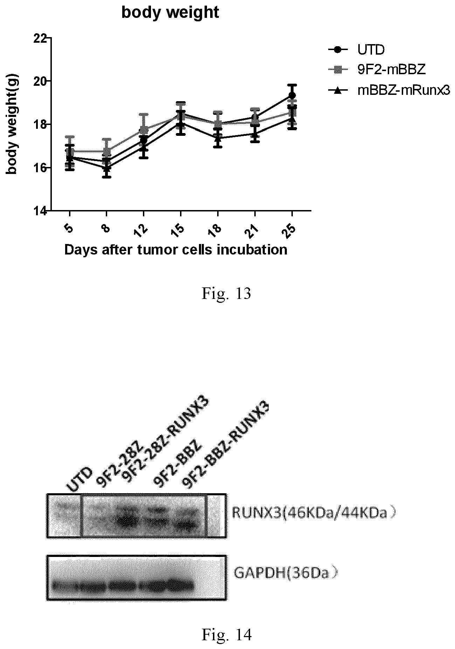

Changes in the body weight of the mice were monitored, and the results were shown in Figure 13. The body weight of the mice in the 9F2-BBZ group and 9F2-mBBZ-mRunX3 CART group was not significantly different from that of UTD.

### Example 11. Expression level of RUNX3 in immune effector cells

Cells were collected, and the cell protein was extracted. 20 µg of the corresponding cell protein was subjected to SDS-PAGE electrophoresis. 5% milk blocking solution was used at room temperature for 1 hour, and then the cells were incubated with anti-RUNX3 antibody and GAPDH antibody at 4°C overnight. After the incubation with the primary antibody was completed, the plate was washed with 0.1% PBST for three times, then incubated with goat anti-mouse IgG (H&L)-HRP antibody for 1 hour at room temperature. After the plate was washed, Super Signal West Pico Chemiluminescence Detection Kit was used for color imaging. As shown in Figure 14, compared with 9F2-28Z-RUNX3, 9F2-BBZ-RUNX3 and immune effector cells not transfected with RUNX3, the expression level of RUNX3 in the immune effector cells of the present invention was increased.

All documents mentioned in the present invention are cited as references in this application, as if each document is individually cited as a reference. In addition, it should be understood that after reading the above teachings described herein, a skilled person can make various changes or modifications to the present invention, and these equivalent forms also fall within the scope defined by the appended claims of the present application.

| SEQ ID NO: | Name of the sequence | Sequence |
|---|---|---|
| 1 | human CD8α signal peptide | atggccttaccagtgaccgccttgctcctgccgctggccttgctgctccacgccgccagg ccg |
| 2 | hu9F2 scFV | Gaggtgcagctggtgcagagcggcgccgaggtgaagaagcccggcgccagcgtga aggtgagcTgcaaggccagcggctacaccttcagcgactacgagatgcactgggtgc ggcaggcccccggcCagggcctggagtggatgggcgccatccaccccggcagcgg cgacaccgcctacaaccagcggTtcaagggccgggtgaccatcaccgccgacaaga gcaccagcaccgcctacatggagctgagcAgcctgcggagcgaggacaccgccgtg tactactgcgcccggttctacagctacgcctactggGgccagggcaccctggtgaccgt gagcgccggtggaggcggttcaggcggaggtggttctggcGgtggcggatcggaca tcgtgatgacccagacccccctgagcctgcccgtgacccccggcgagCccgccagca tcagctgccggagcagccagagcctggtgcacagcaacggcaacacctacctgCagt ggtacctgcagaagcccggccagagcccccagctgctgatctacaaggtgagcaacc ggTtcagcggcgtgcccgaccggttcagcggcagcggcagcggcaccgacttcacc ctgaagatcAgccgggtggaggccgaggacgtgggcgtgtactactgcagccagag catctacgtgccctacaccttcggccagggcaccaagctggagatcaaacgt |
| 3 | human CD8 hinge | Accacgacgccagcgccgcgaccaccaacaccggcgcccaccatcgcgtcgcagc ccctgtcccTgcgcccagaggcgtgccggccagcggcgggggcgcagtgcacac gagggggctggacttcgcctgtgat |
| 4 | human CD28 transmembrane domain | Ttttgggtgctggtggtggttggtggagtcctggcttgctatagcttgctagtaacagtgg cctttattattttctgggtg |
| 5 | human CD28 intracellular domain | Aggagtaagaggagcaggctcctgcacagtgactacatgaacatgactccccgccgc cccgggccaacccgcaagcattaccagccctatgccccaccacgcgacttcgcagcct atcgctcc |
| 6 | Intracellular segment CD3ξ of human CD3 | Agagtgaagttcagcaggagcgcagacgcccccgcgtaccagcagggccagaacc agctctataaCgagctcaatctaggacgaagagaggagtacgatgttttggacaagaga cgtggccgggaccctgAgatgggggggaaagccgcagagaaggaagaaccctcagg aaggcctgtacaatgaactgcagaaaGataagatggcggaggcctacagtgagattgg gatgaaaggcgagcgccggaggggcaaggggcaCgatggcctttaccagggtctca gtacagccaccaaggacacctacgacgcccttcacatgcaggccctgccccctcgc |
| 7 | human CD8 transmembrane domain | Atctacatctgggcgcccttggccgggacttgtggggtccttctcctgtcactggttatca |
| | | cc |
| 8 | Intracellular signaling domain of human CD137 | Aaacggggcagaaagaaactcctgtatatattcaaacaaccatttatgagaccagtaca aactactcaagaggaagatggctgtagctgccgatttccagaagaagaagaaggagga tgtgaactg |
| 9 | GC33-28bbz-LF | ATCCAGGCCTAAGCTTACGCGTCCTAGCGCTACCGGT CGCCA |
| 10 | CD3Z-F2A-R | TCAGAAGGTCAAAATTCAAAGTCTGTTTCACGCGAGG GGGCAGGGCCTGCATGTGAA |
| 11 | Gene fragment F 1 (hu9F2-28Z, with homology arm and part of F2A sequence) | ATCCAGGCCTAAGCTTACGCGTCCTAGCGCTACCGGT CGCCACCATGGCCTTACCAGTGACCGCCTTGCTCCTG CCGCTGGCCTTGCTGCTCCACGCCGCCAGGCCGGAGG TGCAGCTGGTGCAGAGCGGCGCCGAGGTGAAGAAGC CCGGCGCCAGCGTGAAGGTGAGCTGCAAGGCCAGCG GCTACACCTTCAGCGACTACGAGATGCACTGGGTGCG GCAGGCCCCCGGCCAGGGCCTGGAGTGGATGGGCGC CATCCACCCCGGCAGCGGCGACACCGCCTACAACCA GCGGTTCAAGGGCCGGGTGACCATCACCGCCGACAA GAGCACCAGCACCGCCTACATGGAGCTGAGCAGCCT GCGGAGCGAGGACACCGCCGTGTACTACTGCGCCCG GTTCTACAGCTACGCCTACTGGGGCCAGGGCACCCTG GTGACCGTGAGCGCCGGTGGAGGCGGTTCAGGCGGA GGTGGTTCTGGCGGTGGCGGATCGGACATCGTGATGA CCCAGACCCCCCTGAGCCTGCCCGTGACCCCCGGCGA GCCCGCCAGCATCAGCTGCCGGAGCAGCCAGAGCCT GGTGCACAGCAACGGCAACACCTACCTGCAGTGGTA CCTGCAGAAGCCCGGCCAGAGCCCCCAGCTGCTGATC TACAAGGTGAGCAACCGGTTCAGCGGCGTGCCCGAC CGGTTCAGCGGCAGCGGCAGCGGCACCGACTTCACCC TGAAGATCAGCCGGGTGGAGGCCGAGGACGTGGGCG TGTACTACTGCAGCCAGAGCATCTACGTGCCCTACAC CTTCGGCCAGGGCACCAAGCTGGAGATCAAACGTAC CACGACGCCAGCGCCGCGACCACCAACACCGGCGCC CACCATCGCGTCGCAGCCCCTGTCCCTGCGCCCAGAG GCGTGCCGGCCAGCGGCGGGGGGCGCAGTGCACACG AGGGGGCTGGACTTCGCCTGTGATTTTTGGGTGCTGG TGGTGGTTGGTGGAGTCCTGGCTTGCTATAGCTTGCT AGTAACAGTGGCCTTTATTATTTTCTGGGTGAGGAGT AAGAGGAGCAGGCTCCTGCACAGTGACTACATGAAC ATGACTCCCCGCCGCCCCGGGCCAACCCGCAAGCATT ACCAGCCCTATGCCCCACCACGCGACTTCGCAGCCTA TCGCTCCAGAGTGAAGTTCAGCAGGAGCGCAGACGC CCCCGCGTACCAGCAGGGCCAGAACCAGCTCTATAAC GAGCTCAATCTAGGACGAAGAGAGGAGTACGATGTT TTGGACAAGAGACGTGGCCGGGACCCTGAGATGGGG GGAAAGCCGCAGAGAAGGAAGAACCCTCAGGAAGG |
| | | CCTGTACAATGAACTGCAGAAAGATAAGATGGCGGA GGCCTACAGTGAGATTGGGATGAAAGGCGAGCGCCG GAGGGGCAAGGGGCACGATGGCCTTTACCAGGGTCT CAGTACAGCCACCAAGGACACCTACGACGCCCTTCAC ATGCAGGCCCTGCCCCCTCGCGTGAAACAGACTTTGA ATTTTGACCTTCTGA |
| 12 | F2A-RUNX3 -F | AGACGTTGAGTCCAACCCTGGGCCCATGCGTATTCCC GTAGACCCA |
| 13 | RUNX-SalI-R | ATCCAGAGGTTGATTGTCGACTCAGTAGGGCCGCCAC ACGGCCT |
| 14 | Gene fragment F2 (RUNX3, with part of F2A sequence and homology arm) | AGACGTTGAGTCCAACCCTGGGCCCATGCGTATTCCC GTAGACCCAAGCACCAGCCGCCGCTTCACACCTCCCT CCCCGGCCTTCCCCTGCGGCGGCGGCGGCAAGAT GGGCGAGAACAGCGGCGCGCTGAGCGCGCAGGCGGC CGTGGGGCCCGGAGGGCGCGCCCGGCCCGAGGTGCG CTCGATGGTGGACGTGCTGGCGGACCACGCAGGCGA GCTCGTGCGCACCGACAGCCCCAACTTCCTCTGCTCC GTGCTGCCCTCGCACTGGCGCTGCAACAAGACGCTGC CCGTCGCCTTCAAGGTGGTGGCATTGGGGGACGTGCC GGATGGTACGGTGGTGACTGTGATGGCAGGCAATGA CGAGAACTACTCCGCTGAGCTGCGCAATGCCTCGGCC GTCATGAAGAACCAGGTGGCCAGGTTCAACGACCTTC GCTTCGTGGGCCGCAGTGGGCGAGGGAAGAGTTTCA CCCTGACCATCACTGTGTTCACCAACCCCACCCAAGT GGCGACCTACCACCGAGCCATCAAGGTGACCGTGGA CGGACCCCGGGAGCCCAGACGGCACCGGCAGAAGCT GGAGGACCAGACCAAGCCGTTCCCTGACCGCTTTGGG GACCTGGAACGGCTGCGCATGCGGGTGACACCGAGC ACACCCAGCCCCCGAGGCTCACTCAGCACCACAAGCC ACTTCAGCAGCCAGCCCCAGACCCCAATCCAAGGCAC CTCGGAACTGAACCCATTCTCCGACCCCCGCCAGTTT GACCGCTCCTTCCCCACGCTGCCAACCCTCACGGAGA GCCGCTTCCCAGACCCCAGGATGCATTATCCCGGGGC CATGTCAGCTGCCTTCCCCTACAGCGCCACGCCCTCG GGCACGAGCATCAGCAGCCTCAGCGTGGCGGGCATG CCGGCCACCAGCCGCTTCCACCATACCTACCTCCCGC CACCCTACCCGGGGGCCCCGCAGAACCAGAGCGGGC CCTTCCAGGCCAACCCGTCCCCCTACCACCTCTACTA CGGGACATCCTCTGGCTCCTACCAGTTCTCCATGGTG GCCGGCAGCAGCAGTGGGGGCGACCGCTCACCTACC CGCATGCTGGCCTCTTGCACCAGCAGCGCTGCCTCTG |
| | | TCGCCGCCGGCAACCTCATGAACCCCAGCCTGGGCGG CCAGAGTGATGGCGTGGAGGCCGACGGCAGCCACAG CAACTCACCCACGGCCCTGAGCACGCCAGGCCGCATG GATGAGGCCGTGTGGCGGCCCTACTGAGTCGACAATC AACCTCTGGAT |
| 15 | F2A-F | AACAGACTTTGAATTTTGACCTTCTGAAGTTGGCAGG AGACGTTGAGTCCAACCCTGG |
| 16 | Gene fragment F3 (F2A-RUNX 3 with homology arm) | AACAGACTTTGAATTTTGACCTTCTGAAGTTGGCAGG AGACGTTGAGTCCAACCCTGGGCCCATGCGTATTCCC GTAGACCCAAGCACCAGCCGCCGCTTCACACCTCCCT CCCCGGCCTTCCCCTGCGGCGGCGGCGGCAAGAT GGGCGAGAACAGCGGCGCGCTGAGCGCGCAGGCGGC CGTGGGGCCCGGAGGGCGCGCCCGGCCCGAGGTGCG CTCGATGGTGGACGTGCTGGCGGACCACGCAGGCGA GCTCGTGCGCACCGACAGCCCCAACTTCCTCTGCTCC GTGCTGCCCTCGCACTGGCGCTGCAACAAGACGCTGC CCGTCGCCTTCAAGGTGGTGGCATTGGGGGACGTGCC GGATGGTACGGTGGTGACTGTGATGGCAGGCAATGA CGAGAACTACTCCGCTGAGCTGCGCAATGCCTCGGCC GTCATGAAGAACCAGGTGGCCAGGTTCAACGACCTTC GCTTCGTGGGCCGCAGTGGGCGAGGGAAGAGTTTCA CCCTGACCATCACTGTGTTCACCAACCCCACCCAAGT GGCGACCTACCACCGAGCCATCAAGGTGACCGTGGA CGGACCCCGGGAGCCCAGACGGCACCGGCAGAAGCT GGAGGACCAGACCAAGCCGTTCCCTGACCGCTTTGGG GACCTGGAACGGCTGCGCATGCGGGTGACACCGAGC ACACCCAGCCCCCGAGGCTCACTCAGCACCACAAGCC ACTTCAGCAGCCAGCCCCAGACCCCAATCCAAGGCAC CTCGGAACTGAACCCATTCTCCGACCCCCGCCAGTTT GACCGCTCCTTCCCCACGCTGCCAACCCTCACGGAGA GCCGCTTCCCAGACCCCAGGATGCATTATCCCGGGGC CATGTCAGCTGCCTTCCCCTACAGCGCCACGCCCTCG GGCACGAGCATCAGCAGCCTCAGCGTGGCGGGCATG CCGGCCACCAGCCGCTTCCACCATACCTACCTCCCGC CACCCTACCCGGGGGCCCCGCAGAACCAGAGCGGGC CCTTCCAGGCCAACCCGTCCCCCTACCACCTCTACTA CGGGACATCCTCTGGCTCCTACCAGTTCTCCATGGTG GCCGGCAGCAGCAGTGGGGGCGACCGCTCACCTACC CGCATGCTGGCCTCTTGCACCAGCAGCGCTGCCTCTG TCGCCGCCGGCAACCTCATGAACCCCAGCCTGGGCGG CCAGAGTGATGGCGTGGAGGCCGACGGCAGCCACAG CAACTCACCCACGGCCCTGAGCACGCCAGGCCGCATG GATGAGGCCGTGTGGCGGCCCTACTGAGTCGACAATC AACCTCTGGAT |
| 17 | Full length gene fragment hu9F2-28Z-F 2A-huRUNX 3 | ATCCAGGCCTAAGCTTACGCGTCCTAGCGCTACCGGT CGCCACCATGGCCTTACCAGTGACCGCCTTGCTCCTG CCGCTGGCCTTGCTGCTCCACGCCGCCAGGCCGGAGG TGCAGCTGGTGCAGAGCGGCGCCGAGGTGAAGAAGC CCGGCGCCAGCGTGAAGGTGAGCTGCAAGGCCAGCG GCTACACCTTCAGCGACTACGAGATGCACTGGGTGCG GCAGGCCCCCGGCCAGGGCCTGGAGTGGATGGGCGC CATCCACCCCGGCAGCGGCGACACCGCCTACAACCA GCGGTTCAAGGGCCGGGTGACCATCACCGCCGACAA GAGCACCAGCACCGCCTACATGGAGCTGAGCAGCCT GCGGAGCGAGGACACCGCCGTGTACTACTGCGCCCG GTTCTACAGCTACGCCTACTGGGGCCAGGGCACCCTG GTGACCGTGAGCGCCGGTGGAGGCGGTTCAGGCGGA GGTGGTTCTGGCGGTGGCGGATCGGACATCGTGATGA CCCAGACCCCCCTGAGCCTGCCCGTGACCCCCGGCGA GCCCGCCAGCATCAGCTGCCGGAGCAGCCAGAGCCT GGTGCACAGCAACGGCAACACCTACCTGCAGTGGTA CCTGCAGAAGCCCGGCCAGAGCCCCCAGCTGCTGATC TACAAGGTGAGCAACCGGTTCAGCGGCGTGCCCGAC CGGTTCAGCGGCAGCGGCAGCGGCACCGACTTCACCC TGAAGATCAGCCGGGTGGAGGCCGAGGACGTGGGCG TGTACTACTGCAGCCAGAGCATCTACGTGCCCTACAC CTTCGGCCAGGGCACCAAGCTGGAGATCAAACGTAC CACGACGCCAGCGCCGCGACCACCAACACCGGCGCC CACCATCGCGTCGCAGCCCCTGTCCCTGCGCCCAGAG GCGTGCCGGCCAGCGGCGGGGGGCGCAGTGCACACG AGGGGGCTGGACTTCGCCTGTGATTTTTGGGTGCTGG TGGTGGTTGGTGGAGTCCTGGCTTGCTATAGCTTGCT AGTAACAGTGGCCTTTATTATTTTCTGGGTGAGGAGT AAGAGGAGCAGGCTCCTGCACAGTGACTACATGAAC ATGACTCCCCGCCGCCCCGGGCCAACCCGCAAGCATT ACCAGCCCTATGCCCCACCACGCGACTTCGCAGCCTA TCGCTCCAGAGTGAAGTTCAGCAGGAGCGCAGACGC CCCCGCGTACCAGCAGGGCCAGAACCAGCTCTATAAC GAGCTCAATCTAGGACGAAGAGAGGAGTACGATGTT TTGGACAAGAGACGTGGCCGGGACCCTGAGATGGGG GGAAAGCCGCAGAGAAGGAAGAACCCTCAGGAAGGC CTGTACAATGAACTGCAGAAAGATAAGATGGCGGAG GCCTACAGTGAGATTGGGATGAAAGGCGAGCGCCGG AGGGGCAAGGGGCACGATGGCCTTTACCAGGGTCTC AGTACAGCCACCAAGGACACCTACGACGCCCTTCACA TGCAGGCCCTGCCCCCTCGCGTGAAACAGACTTTGAA TTTTGACCTTCTGAAGTTGGCAGGAGACGTTGAGTCC AACCCTGGGCCCATGCGTATTCCCGTAGACCCAAGCA CCAGCCGCCGCTTCACACCTCCCTCCCCGGCCTTCCCC TGCGGCGGCGGCGGCGGCAAGATGGGCGAGAACAGC GGCGCGCTGAGCGCGCAGGCGGCCGTGGGGCCCGGA GGGCGCGCCCGGCCCGAGGTGCGCTCGATGGTGGAC GTGCTGGCGGACCACGCAGGCGAGCTCGTGCGCACC GACAGCCCCAACTTCCTCTGCTCCGTGCTGCCCTCGC ACTGGCGCTGCAACAAGACGCTGCCCGTCGCCTTCAA |
| | | GGTGGTGGCATTGGGGGACGTGCCGGATGGTACGGT GGTGACTGTGATGGCAGGCAATGACGAGAACTACTC CGCTGAGCTGCGCAATGCCTCGGCCGTCATGAAGAAC CAGGTGGCCAGGTTCAACGACCTTCGCTTCGTGGGCC GCAGTGGGCGAGGGAAGAGTTTCACCCTGACCATCA CTGTGTTCACCAACCCCACCCAAGTGGCGACCTACCA CCGAGCCATCAAGGTGACCGTGGACGGACCCCGGGA GCCCAGACGGCACCGGCAGAAGCTGGAGGACCAGAC CAAGCCGTTCCCTGACCGCTTTGGGGACCTGGAACGG CTGCGCATGCGGGTGACACCGAGCACACCCAGCCCCC GAGGCTCACTCAGCACCACAAGCCACTTCAGCAGCCA GCCCCAGACCCCAATCCAAGGCACCTCGGAACTGAA CCCATTCTCCGACCCCCGCCAGTTTGACCGCTCCTTCC CCACGCTGCCAACCCTCACGGAGAGCCGCTTCCCAGA CCCCAGGATGCATTATCCCGGGGCCATGTCAGCTGCC TTCCCCTACAGCGCCACGCCCTCGGGCACGAGCATCA GCAGCCTCAGCGTGGCGGGCATGCCGGCCACCAGCC GCTTCCACCATACCTACCTCCCGCCACCCTACCCGGG GGCCCCGCAGAACCAGAGCGGGCCCTTCCAGGCCAA CCCGTCCCCCTACCACCTCTACTACGGACATCCTCT GGCTCCTACCAGTTCTCCATGGTGGCCGGCAGCAGCA GTGGGGGCGACCGCTCACCTACCCGCATGCTGGCCTC TTGCACCAGCAGCGCTGCCTCTGTCGCCGCCGGCAAC CTCATGAACCCCAGCCTGGGCGGCCAGAGTGATGGC GTGGAGGCCGACGGCAGCCACAGCAACTCACCCACG GCCCTGAGCACGCCAGGCCGCATGGATGAGGCCGTG TGGCGGCCCTACTGAGTCGACAATCAACCTCTGGAT |
| 18 | Gene fragment F4 (hu9F2-BBZ, , with homology arm and part of F2A sequence) | ATCCAGGCCTAAGCTTACGCGTCCTAGCGCTACCGGT CGCCACCATGGCCTTACCAGTGACCGCCTTGCTCCTG CCGCTGGCCTTGCTGCTCCACGCCGCCAGGCCGGAGG TGCAGCTGGTGCAGAGCGGCGCCGAGGTGAAGAAGC CCGGCGCCAGCGTGAAGGTGAGCTGCAAGGCCAGCG GCTACACCTTCAGCGACTACGAGATGCACTGGGTGCG GCAGGCCCCCGGCCAGGGCCTGGAGTGGATGGGCGC CATCCACCCCGGCAGCGGCGACACCGCCTACAACCA GCGGTTCAAGGGCCGGGTGACCATCACCGCCGACAA GAGCACCAGCACCGCCTACATGGAGCTGAGCAGCCT GCGGAGCGAGGACACCGCCGTGTACTACTGCGCCCG GTTCTACAGCTACGCCTACTGGGGCCAGGGCACCCTG GTGACCGTGAGCGCCGGTGGAGGCGGTTCAGGCGGA GGTGGTTCTGGCGGTGGCGGATCGGACATCGTGATGA CCCAGACCCCCCTGAGCCTGCCCGTGACCCCCGGCGA GCCCGCCAGCATCAGCTGCCGGAGCAGCCAGAGCCT GGTGCACAGCAACGGCAACACCTACCTGCAGTGGTA CCTGCAGAAGCCCGGCCAGAGCCCCCAGCTGCTGATC TACAAGGTGAGCAACCGGTTCAGCGGCGTGCCCGAC CGGTTCAGCGGCAGCGGCAGCGGCACCGACTTCACCC TGAAGATCAGCCGGGTGGAGGCCGAGGACGTGGGCG TGTACTACTGCAGCCAGAGCATCTACGTGCCCTACAC CTTCGGCCAGGGCACCAAGCTGGAGATCAAACGTAC CACGACGCCAGCGCCGCGACCACCAACACCGGCGCC |
| | | CACCATCGCGTCGCAGCCCCTGTCCCTGCGCCCAGAG GCGTGCCGGCCAGCGGCGGGGGCGCAGTGCACACG AGGGGGCTGGACTTCGCCTGTGATATCTACATCTGGG CGCCCTTGGCCGGGACTTGTGGGGTCCTTCTCCTGTC ACTGGTTATCACCCTTTACTGCAAACGGGGCAGAAAG AAACTCCTGTATATATTCAAACAACCATTTATGAGAC CAGTACAAACTACTCAAGAGGAAGATGGCTGTAGCT GCCGATTTCCAGAAGAAGAAGAAGGAGGATGTGAAC TGAGAGTGAAGTTCAGCAGGAGCGCAGACGCCCCCG CGTACCAGCAGGGCCAGAACCAGCTCTATAACGAGC TCAATCTAGGACGAAGAGAGGAGTACGATGTTTTGG ACAAGAGACGTGGCCGGGACCCTGAGATGGGGGGAA AGCCGCAGAGAAGGAAGAACCCTCAGGAAGGCCTGT ACAATGAACTGCAGAAAGATAAGATGGCGGAGGCCT ACAGTGAGATTGGGATGAAAGGCGAGCGCCGGAGGG GCAAGGGGCACGATGGCCTTTACCAGGGTCTCAGTAC AGCCACCAAGGACACCTACGACGCCCTTCACATGCAG GCCCTGCCCCCTCGCGTGAAACAGACTTTGAATTTTG ACCTTCTGA |
| 19 | Full length gene fragment hu9F2-BBZ-F2A-huRUN X3 | ATCCAGGCCTAAGCTTACGCGTCCTAGCGCTACCGGT CGCCACCATGGCCTTACCAGTGACCGCCTTGCTCCTG CCGCTGGCCTTGCTGCTCCACGCCGCCAGGCCGGAGG TGCAGCTGGTGCAGAGCGGCGCCGAGGTGAAGAAGC CCGGCGCCAGCGTGAAGGTGAGCTGCAAGGCCAGCG GCTACACCTTCAGCGACTACGAGATGCACTGGGTGCG GCAGGCCCCCGGCCAGGGCCTGGAGTGGATGGGCGC CATCCACCCCGGCAGCGGCGACACCGCCTACAACCA GCGGTTCAAGGGCCGGGTGACCATCACCGCCGACAA GAGCACCAGCACCGCCTACATGGAGCTGAGCAGCCT GCGGAGCGAGGACACCGCCGTGTACTACTGCGCCCG GTTCTACAGCTACGCCTACTGGGGCCAGGGCACCCTG GTGACCGTGAGCGCCGGTGGAGGCGGTTCAGGCGGA GGTGGTTCTGGCGGTGGCGGATCGGACATCGTGATGA CCCAGACCCCCCTGAGCCTGCCCGTGACCCCCGGCGA GCCCGCCAGCATCAGCTGCCGGAGCAGCCAGAGCCT GGTGCACAGCAACGGCAACACCTACCTGCAGTGGTA CCTGCAGAAGCCCGGCCAGAGCCCCCAGCTGCTGATC TACAAGGTGAGCAACCGGTTCAGCGGCGTGCCCGAC CGGTTCAGCGGCAGCGGCAGCGGCACCGACTTCACCC TGAAGATCAGCCGGGTGGAGGCCGAGGACGTGGGCG TGTACTACTGCAGCCAGAGCATCTACGTGCCCTACAC CTTCGGCCAGGGCACCAAGCTGGAGATCAAACGTAC CACGACGCCAGCGCCGCGACCACCAACACCGGCGCC CACCATCGCGTCGCAGCCCCTGTCCCTGCGCCCAGAG GCGTGCCGGCCAGCGGCGGGGGGCGCAGTGCACACG AGGGGGCTGGACTTCGCCTGTGATATCTACATCTGGG CGCCCTTGGCCGGGACTTGTGGGGTCCTTCTCCTGTC ACTGGTTATCACCCTTTACTGCAAACGGGGCAGAAAG AAACTCCTGTATATATTCAAACAACCATTTATGAGAC CAGTACAAACTACTCAAGAGGAAGATGGCTGTAGCT GCCGATTTCCAGAAGAAGAAGAAGGAGGATGTGAAC |
| | | TGAGAGTGAAGTTCAGCAGGAGCGCAGACGCCCCCG CGTACCAGCAGGGCCAGAACCAGCTCTATAACGAGC TCAATCTAGGACGAAGAGAGGAGTACGATGTTTTGG ACAAGAGACGTGGCCGGGACCCTGAGATGGGGGGAA AGCCGCAGAGAAGGAAGAACCCTCAGGAAGGCCTGT ACAATGAACTGCAGAAAGATAAGATGGCGGAGGCCT ACAGTGAGATTGGGATGAAAGGCGAGCGCCGGAGGG GCAAGGGGCACGATGGCCTTTACCAGGGTCTCAGTAC AGCCACCAAGGACACCTACGACGCCCTTCACATGCAG GCCCTGCCCCCTCGCGTGAAACAGACTTTGAATTTTG ACCTTCTGAAGTTGGCAGGAGACGTTGAGTCCAACCC TGGGCCCATGCGTATTCCCGTAGACCCAAGCACCAGC CGCCGCTTCACACCTCCCTCCCCGGCCTTCCCCTGCGG CGGCGGCGGCGGCAAGATGGGCGAGAACAGCGGCGC GCTGAGCGCGCAGGCGGCCGTGGGGCCCGGAGGGCG CGCCCGGCCCGAGGTGCGCTCGATGGTGGACGTGCTG GCGGACCACGCAGGCGAGCTCGTGCGCACCGACAGC CCCAACTTCCTCTGCTCCGTGCTGCCCTCGCACTGGCG CTGCAACAAGACGCTGCCCGTCGCCTTCAAGGTGGTG GCATTGGGGGACGTGCCGGATGGTACGGTGGTGACT GTGATGGCAGGCAATGACGAGAACTACTCCGCTGAG CTGCGCAATGCCTCGGCCGTCATGAAGAACCAGGTGG CCAGGTTCAACGACCTTCGCTTCGTGGGCCGCAGTGG GCGAGGGAAGAGTTTCACCCTGACCATCACTGTGTTC ACCAACCCCACCCAAGTGGCGACCTACCACCGAGCC ATCAAGGTGACCGTGGACGGACCCCGGGAGCCAGA CGGCACCGGCAGAAGCTGGAGGACCAGACCAAGCCG TTCCCTGACCGCTTTGGGGACCTGGAACGGCTGCGCA TGCGGGTGACACCGAGCACACCCAGCCCCCGAGGCT CACTCAGCACCACAAGCCACTTCAGCAGCCAGCCCCA GACCCCAATCCAAGGCACCTCGGAACTGAACCCATTC TCCGACCCCCGCCAGTTTGACCGCTCCTTCCCCACGCT GCCAACCCTCACGGAGAGCCGCTTCCCAGACCCCAGG ATGCATTATCCCGGGGCCATGTCAGCTGCCTTCCCCT ACAGCGCCACGCCCTCGGGCACGAGCATCAGCAGCC TCAGCGTGGCGGGCATGCCGGCCACCAGCCGCTTCCA CCATACCTACCTCCCGCCACCCTACCCGGGGGCCCCG CAGAACCAGAGCGGGCCCTTCCAGGCCAACCCGTCCC CCTACCACCTCTACTACGGGACATCCTCTGGCTCCTA CCAGTTCTCCATGGTGGCCGGCAGCAGCAGTGGGGGC GACCGCTCACCTACCCGCATGCTGGCCTCTTGCACCA GCAGCGCTGCCTCTGTCGCCGCCGGCAACCTCATGAA CCCCAGCCTGGGCGGCCAGAGTGATGGCGTGGAGGC CGACGGCAGCCACAGCAACTCACCCACGGCCCTGAG CACGCCAGGCCGCATGGATGAGGCCGTGTGGCGGCC CTACTGAGTCGACAATCAACCTCTGGAT |
| 20 | RUNX3 sequence | MRIPVDPSTSRRFTPPSPAFPCGGGGGKMGENSGALSAQ AAVGPGGRARPEVRSMVDVLADHAGELVRTDSPNFLC SVLPSHWRCNKTLPVAFKVVALGDVPDGTVVTVMAGN DENYSAELRNASAVMKNQVARFNDLRFVGRSGRGKSF TLTITVFTNPTQVATYHRAIKVTVDGPREPRRHRQKLED |
| | | QTKPFPDRFGDLERLRMRVTPSTPSPRGSLSTTSHFSSQP QTPIQGTSELNPFSDPRQFDRSFPTLPTLTESRFPDPRMH YPGAMSAAFPYSATPSGTSISSLSVAGMPATSRFHHTYL PPPYPGAPQNQSGPFQANPSPYHLYYGTSSGSYQFSMVA GSSSGGDRSPTRMLASCTSSAASVAAGNLMNPSLGGQS DGVEADGSHSNSPTALSTPGRMDEAVWRPY |
| 21 | Hu8E5-2I scFV | Caggtgcagctgcaggagagcggccccggcctgatcaagcccagccagaccctgag cctgacctgcaccGtgagcggcggcagcatcagcagcggctacaactggcactggat ccggcagcccccggcaagggcctggAgtggatcggctacatccactacaccggca gcaccaactacaaccccgccctgcggagccgggtgaccatCagcgtggacaccagc aagaaccagttcagcctgaagctgagcagcgtgaccgccgccgacaccgccatcTac tactgcgcccggatctacaacggcaacagcttcccctactggggccagggcaccaccg tgaccgtgaGcagcggtggaggcggttcaggcggaggtggttctggcggtggcggat cggacatcgtgatgacccagagCcccgacagcctggccgtgagcctgggcgagcgg gccaccatcaactgcaagagcagccagagcctgttCaacagcggcaaccagaagaac tacctgacctggtaccagcagaagcccggccagccccccaagctgctgAtctactggg ccagcacccgggagagcggcgtgcccgaccggttcagcggcagcggcagcggcac cgactTcaccctgaccatcagcagcctgcaggccgaggacgtggccgtgtactactgc cagaacgcctacagcttcccctacaccttcggcggcggcaccaagctggagatcaagc gg |
| 22 | Hu8E5-2I scFV | Qvqlqesgpglikpsqtlsltctvsggsissgynwhwirqppgkglewigyihytgst nynpalrsrvtiSvdtsknqfslklssvtaadtaiyycariyngnsfpywgqgttvtvss ggggsggggsggggsdivmtqsPdslavslgeratinckssqslfnsgnqknyltwy qqkpgqppklliywastresgvpdrfsgsgsgtdftltisslqaedvavyycqnaysfp ytfgggtkleikr |
| 23 | F2A | Gtgaaacagactttgaattttgaccttctgaagttggcaggagacgttgagtccaaccctg ggccc |
| 24 | F2A | Vkqtlnfdllklagdvesnpgp |
| 25 | mouse RUNX3 (isoform 1) | Atgcgtattcccgtagacccgagcaccagccgccgcttcactccccctccacggcctt cccctgCggcggcggcggcggcggcaagatggcgagaacagcggcgcgctaag cgcgcaggcaaccgcggGccccggcggccgcacccggcccgaagtgcgctcgatg gtggacgtgctggccgaccacgcggaGagctcgtgcgcaccgacagccccaacttc ctctgctccgtgctgccctcgcactggcgctgcaaCaagacgctgccggtcgccttcaa ggtggtggccctgggggatgtgccggatggaacggtggtgacCgtgatggccggcaa tgatgagaactactccgccgagctgcgcaacgcttccgctgtcatgaagaAccaagtg gccaggttcaacgaccttcgattcgtgggccgcagtgggcgagggaagagtttcacgC tcacaatcaccgtgttcaccaaccctacccaagtggctacctaccaccgagccatcaag gtcacTgtggatggaccccgggaaccccgacggcaccggcagaagatagaagacca gaccaaggccttcccCgaccgctttggagacctgcgcatgcgtgtaacaccaagcaca cccagcccccgtggctctctcagCaccacgagccacttcagcagccaggcccagacc ccaatccaaggctcctcagacctgaaccccttCtccgacccccgccagtttgaccgctc cttccctacgctgcagagcctcacagagagccgcttccCggaccccaggatgcactac ccgggagccatgtctgccgccttcccctacagcgccacaccatcgGgcaccagcctg ggcagcctgagcgtggcgggcatgccggccagcagccgcttccaccacacctAcctc cctccgccctaccccggggccccacagagccagagcgggccctttcaggccaacccc gcgCcctaccacctcttttacggcgcctcctccggctcctaccagttctccatggcagcc gcgggagGtggtgagcgctcgcccacccgcatgctgacctcctgccccagcggcgct tcggtgtcagcaggCaacctcatgaaccccagcctgggccaggctgatggcgtggaa gccgacggcagccacagcaactcgcccacggccctgagcacgccgggccgcatgga cgaggccgtgtggcggccctactga |
| 26 | mouse RUNX3 (isoform 1) | MRIPVDPSTSRRFTPPSTAFPCGGGGGGKMGENSGALSA QATAGPGGRTRPEVRSMVDVLADHAGELVRTDSPNFLC SVLPSHWRCNKTLPVAFKVVALGDVPDGTVVTVMAGN DENYSAELRNASAVMKNQVARFNDLRFVGRSGRGKSF TLTITVFTNPTQVATYHRAIKVTVDGPREPRRHRQKIED QTKAFPDRFGDLRMRVTPSTPSPRGSLSTTSHFSSQAQTP IQGSSDLNPFSDPRQFDRSFPTLQSLTESRFPDPRMHYPG AMSAAFPYSATPSGTSLGSLSVAGMPASSRFHHTYLPPP YPGAPQSQSGPFQANPAPYHLFYGASSGSYQFSMAAAG GGERSPTRMLTSCPSGASVSAGNLMNPSLGQADGVEAD GSHSNSPTALSTPGRMDEAVWRPY |
| 27 | signal peptide of mouse CD8α | Atggcctcaccgttgacccgctttctgtcgctgaacctgctgctgctgggtgagtcgatta tcctggggagtggagaagct |
| 28 | signal peptide of mouse CD8α | maspltrflslnllllgesiilgsgea |
| 29 | mouse CD8 hinge + transmembra ne domain | Actactaccaagccagtgctgcgaactccctcacctgtgcaccctaccgggacatctca gccccagaGaccagaagattgtcggccccgtggctcagtgaaggggaccggattgga cttcgcctgtgatatttaCatctgggcacccttggccggaatctgcgtggcccttctgctgt ccttgatcatcactctcatctgctaccacaggagccga |
| 30 | mouse CD8 hinge + transmembra ne domain | Tttkpvlrtpspvhptgtsqpqrpedcrprgsvkgtgldfacdiyiwaplagicvallls liitlicyhrsr |
| 31 | Intracellular domain of mouse 4-1BB | Aaatggatcaggaaaaaattcccccacatattcaagcaaccatttaagaagaccactgg agcagctcaagaggaagatgcttgtagctgccgatgtccacaggaagaagaaggagg aggaggaggctatgagctg |
| 32 | Intracellular domain of mouse 4-1BB | Kwirkkfphifkqpfkkttgaaqeedacscrcpqeeegggggyel |
| 33 | Intracellular segment CD3ξ of mouse CD3 | Agcaggagtgcagagactgctgccaacctgcaggaccccaaccagctctacaatgag ctcaatctAgggcgaagagaggaatatgacgtcttggagaagaagcgggctcgggat ccagagatgggaggcaAacagcagaggaggaggaacccccaggaaggcgtataca atgcactgcagaaagacaagatggcaGaagcctacagtgagatcggcacaaaaggc gagaggcggagaggcaaggggcacgatggcctttaccagggtctcagcactgccacc aaggacacctatgatgccctgcatatgcagaccctggcc |
| 34 | Intracellular segment CD3ξ of mouse CD3 | Srsaetaanlqdpnqlynelnlgrreeydvlekkrardpemggkqqrrrnpqegvyn alqkdkmaeayseigtkgerrrgkghdglyqglstatkdtydalhmqtla |
| 35 | Nucleotide sequence of Human IL21 | ATGAGATCCAGTCCTGGCAACATGGAGAGGATTGTCA TCTGTCTGATGGTCATCTTCTTGGGGACACTGGTCCAC AAATCAAGCTCCCAAGGTCAAGATCGCCACATGATTA GAATGCGTCAACTTATAGATATTGTTGATCAGCTGAA AAATTATGTGAATGACTTGGTCCCTGAATTTCTGCCA GCTCCAGAAGATGTAGAGACAAACTGTGAGTGGTCA GCTTTTTCCTGCTTTCAGAAGGCCCAACTAAAGTCAG CAAATACAGGAAACAATGAAAGGATAATCAATGTAT CAATTAAAAAGCTGAAGAGGAAACCACCTTCCACAA |
| | | ATGCAGGGAGAAGACAGAAACACAGACTAACATGCC CTTCATGTGATTCTTATGAGAAAAAACCACCCAAAGA ATTCCTAGAAAGATTCAAATCACTTCTCCAAAAGATG ATTCATCAGCATCTGTCCTCTAGAACACACGGAAGTG AAGATTCCTGA |
| 36 | Amino acid sequence of human IL21 | MRSSPGNMERIVICLMVIFLGTLVHKSSSQGQDRHMIRM RQLIDIVDQLKNYVNDLVPEFLPAPEDVETNCEWSAFSC FQKAQLKSANTGNNERIINVSIKKLKRKPPSTNAGRRQK HRLTCPSCDSYEKKPPKEFLERFKSLLQKMIHQHLSSRT HGSEDS |
| 37 | NFAT sequence | Ggaggaaaaactgtttcatacagaaggcgt |
| 38 | Nucleotide sequence of human IL15 | ATGAGAATTTCGAAACCACATTTGAGAAGTATTTCCA TCCAGTGCTACTTGTGTTTACTTCTAAACAGTCATTTT CTAACTGAAGCTGGCATTCATGTCTTCATTTTGGGCTG TTTCAGTGCAGGGCTTCCTAAAACAGAAGCCAACTGG GTGAATGTAATAAGTGATTTGAAAAAAATTGAAGATC TTATTCAATCTATGCATATTGATGCTACTTTATATACG GAAAGTGATGTTCACCCCAGTTGCAAAGTAACAGCA ATGAAGTGCTTTCTCTTGGAGTTACAAGTTATTTCACT TGAGTCCGGAGATGCAAGTATTCATGATACAGTAGAA AATCTGATCATCCTAGCAAACAACAGTTTGTCTTCTA ATGGGAATGTAACAGAATCTGGATGCAAAGAATGTG AGGAACTGGAGGAAAAAAATATTAAAGAATTTTTGC AGAGTTTTGTACATATTGTCCAAATGTTCATCAACAC TTCTTGA |
| 39 | Amino acid sequence of human IL15 | MRISKPHLRSISIQCYLCLLLNSHFLTEAGIHVFILGCFSA GLPKTEANWVNVISDLKKIEDLIQSMHIDATLYTESDVH PSCKVTAMKCFLLELQVISLESGDASIHDTVENLIILANN SLSSNGNVTESGCKECEELEEKNIKEFLQSFVHIVQMFIN TS |
| 40 | Nucleotide sequence of human IL18 | ATGGCTGCTGAACCAGTAGAAGACAATTGCATCAACT TTGTGGCAATGAAATTTATTGACAATACGCTTTACTTT ATAGCTGAAGATGATGAAAACCTGGAATCAGATTACT TTGGCAAGCTTGAATCTAAATTATCAGTCATAAGAAA TTTGAATGACCAAGTTCTCTTCATTGACCAAGGAAAT CGGCCTCTATTTGAAGATATGACTGATTCTGACTGTA GAGATAATGCACCCCGGACCATATTTATTATAAGTAT GTATAAAGATAGCCAGCCTAGAGGTATGGCTGTAACT ATCTCTGTGAAGTGTGAGAAAATTTCAACTCTCTCCT GTGAGAACAAAATTATTTCCTTTAAGGAAATGAATCC TCCTGATAACATCAAGGATACAAAAAGTGACATCATA TTCTTTCAGAGAAGTGTCCCAGGACATGATAATAAGA TGCAATTTGAATCTTCATCATACGAAGGATACTTTCT AGCTTGTGAAAAAGAGAGAGACCTTTTAAACTCATT TTGAAAAAGAGGATGAATTGGGGGATAGATCTATA ATGTTCACTGTTCAAAACGAAGACTAG |
| 41 | Amino acid sequence of human IL18 | MAAEPVEDNCINFVAMKFIDNTLYFIAEDDENLESDYFG KLESKLSVIRNLNDQVLFIDQGNRPLFEDMTDSDCRDNA PRTIFIISMYKDSQPRGMAVTISVKCEKISTLSCENKIISFK |
| | | EMNPPDNIKDTKSDIIFFQRSVPGHDNKMQFESSSYEGY FLACEKERDLFKLILKKEDELGDRSIMFTVQNED |
| 42 | Amino acid sequence of hu9F2 scFV | EVQLVQSGAEVKKPGASVKVSCKASGYTFSDYEMHWV RQAPGQGLEWMGAIHPGSGDTAYNQRFKGRVTITADK STSTAYMELSSLRSEDTAVYYCARFYSYAYWGQGTLVT VSAGGGGSGGGGSGGGGSDIVMTQTPLSLPVTPGEPASI SCRSSQSLVHSNGNTYLQWYLQKPGQSPQLLIYKVSNR FSGVPDRFSGSGSGTDFTLKISRVEAEDVGVYYCSQSIY VPYTFGQGTKLEIKR |
| 43 | Amino acid sequence of human CD8α signal peptide | malpvtalllplalllhaarp |
| 44 | Amino acid sequence of human CD8 hinge | tttpaprpptpaptiasqplslrpeacrpaaggavhtrgldfacd |
| 45 | Amino acid sequence of human CD28 transmembra ne domain | fwvlvvvggvlacysllvtvafiifwv |
| 46 | Amino acid sequence of human CD28 intracellular domain | Rskrsrllhsdymnmtprrpgptrkhyqpyapprdfaayrs |
| 47 | Amino acid sequence of intracellular segment CD3ξ of humanCD3 | Rvkfsrsadapayqqgqnqlynelnlgrreeydvldkrrgrdpemggkpqrrknpq eglynelqkdkmaeayseigmkgerrrgkghdglyqglstatkdtydalhmqalpp r |
| 48 | Amino acid sequence of human CD8 transmembra ne domain | Iyiwaplagtcgvlllslvit |
| 49 | Amino acid sequence of intracellular signaling domain of human CD137 | Krgrkkllyifkqpfmrpvqttqeedgcscrfpeeeeggcel |
| 50 | Nucleic acid sequence of human RUNX3 | Atgcgtattcccgtagacccaagcaccagccgccgcttcacacctccctccccggcctt cccctgcggcGgcggcggcggcaagatgggcgagaacagcggcgcgctgagcgc gcaggcggccgtggggcccggagggCgcgcccggcccgaggtgcgctcgatggtg gacgtgctggcggaccacgcaggcgagctcgtgcgcaccgAcagccccaacttcctc tgctccgtgctgccctcgcactggcgctgcaacaagacgctgcccgtcgcctTcaaggt ggtggcattgggggacgtgccggatggtacggtggtgactgtgatggcaggcaatgac gagaActactccgctgagctgcgcaatgcctcggccgtcatgaagaaccaggtggcc aggttcaacgaccttcGcttcgtgggccgcagtgggcgagggaagagtttcaccctga ccatcactgtgttcaccaaccccacccAagtggcgacctaccaccgagccatcaaggt gaccgtggacggaccccgggagcccagacggcaccggcAgaagctggaggacca gaccaagccgttccctgaccgctttggggacctggaacggctgcgcatgcgggTgac accgagcacacccagcccccgaggctcactcagcaccacaagccacttcagcagcca gccccagaCcccaatccaaggcacctcggaactgaacccattctccgacccccgcca gtttgaccgctccttccccaCgctgccaaccctcacggagagccgcttcccagacccca ggatgcattatcccggggccatgtcagctgCcttcccctacagcgccacgccctcggg cacgagcatcagcagcctcagcgtggcgggcatgccggccacCagccgcttccacca tacctacctcccgccaccctacccgggggccccgcagaaccagagcgggcccttccA ggccaacccgtcccccctaccacctctactacgggacatcctctggctcctaccagttctcc atggtggccGgcagcagcagtgggggcgaccgctcacctacccgcatgctggcctctt gcaccagcagcgctgcctctgtcGccgccggcaacctcatgaaccccagcctgggcg gccagagtgatggcgtggaggccgacggcagccacagcaactcacccacggccctg agcacgccaggccgcatggatgaggccgtgtggcggccctactga |
| 51 | Amino acid sequence of human CLD18A2 | MAVTACQGLGFVVSLIGIAGIIAATCMDQWSTQDLYNN PVTAVFNYQGLWRSCVRESSGFTECRGYFTLLGLPAML QAVRALMIVGIVLGAIGLLVSIFALKCIRIGSMEDSAKAN MTLTSGIMFIVSGLCAIAGVSVFANMLVTNFWMSTANM YTGMGGMVQTVQTRYTFGAALFVGWVAGGLTLIGGV MMCIACRGLAPEETNYKAVSYHASGHSVAYKPGGFKA STGFGSNTKNKKIYDGGARTEDEVQSYPSKHDYV |
| 52 | Amino acid sequence of human CLD18A1 | MSTTTCQVVAFLLSILGLAGCIAATGMDMWSTQDLYD NPVTSVFQYEGLWRSCVRQSSGFTECRPYFTILGLPAML QAVRALMIVGIVLGAIGLLVSIFALKCIRIGSMEDSAKAN MTLTSGIMFIVSGLCAIAGVSVFANMLVTNFWMSTANM YTGMGGMVQTVQTRYTFGAALFVGWVAGGLTLIGGV MMCIACRGLAPEETNYKAVSYHASGHSVAYKPGGFKA STGFGSNTKNKKIYDGGARTEDEVQSYPSKH |
| 53 | Full length gene fragment hu9F2-28BB Z-F2A-huRU NX3 | Gaggtgcagctggtgcagagcggcgccgaggtgaagaagcccggcgccagcgtga aggtgagctgcaaGgccagcggctacaccttcagcgactacgagatgcactgggtgc ggcaggcccccggccagggcctggaGtggatgggcgccatccaccccggcagcgg cgacaccgcctacaaccagcggttcaagggccgggtgacCatcaccgccgacaaga gcaccagcaccgcctacatggagctgagcagcctgcggagcgaggacaccgcCgtg tactactgcgcccggttctacagctacgcctactggggccagggcaccctggtgaccgt gagcgCcggtggaggcggttcaggcggaggtggttctggcggtggcggatcggaca tcgtgatgacccagaccCccctgagcctgcccgtgacccccggcgagcccgccagca tcagctgccggagcagccagagcctggtGcacagcaacggcaacacctacctgcagt ggtacctgcagaagcccggccagagcccccagctgctgatCtacaaggtgagcaacc ggttcagcggcgtgcccgaccggttcagcggcagcggcagcggcaccgactTcacc ctgaagatcagccgggtggaggccgaggacgtgggcgtgtactactgcagccagagc atctacGtgccctacaccttcggccagggcaccaagctggagatcaaacgtaccacga cgccagcgccgcgaccaCcaacaccggcgcccaccatcgcgtcgcagcccctgtcc |
| | | ctgcgcccagaggcgtgccggccagcggcgGggggcgcagtgcacacgaggggg ctggacttcgcctgtgattttgggtgctggtggtggttggtggagTcctggcttgctatag cttgctagtaacagtggcctttattatttctgggtgaggagtaagaggagcagGctcctg cacagtgactacatgaacatgactccccgccgccccgggccaacccgcaagcattacc agccctAtgccccaccacgcgacttcgcagcctatcgctccaaacggggcagaaaga aactcctgtatatattcaaAcaaccatttatgagaccagtacaaactactcaagaggaag atggctgtagctgccgatttccagaagaagAagaaggaggatgtgaactgagagtgaa gttcagcaggagcgcagacgcccccgcgtaccagcagggccagAaccagctctata acgagctcaatctaggacgaagagaggagtacgatgttttggacaagagacgtggccg Ggaccctgagatgggggaaagccgcagagaaggaagaaccctcaggaaggcctg tacaatgaactgcagaAagataagatggcggaggcctacagtgagattgggatgaaag gcgagcgccggaggggcaaggggcacgatGgcctttaccagggtctcagtacagcc accaaggacacctacgacgcccttcacatgcaggccctgccccTcgcgtgaaacag actttgaattttgaccttctgaagttggcaggagacgttgagtccaaccctgggcccaTg cgtattcccgtagacccaagcaccagccgccgcttcacacctccctccccggccttccc ctgcggcggcGgcggcggcaagatgggcgagaacagcggcgcgctgagcgcgca ggcggccgtggggcccggagggcgcgccCggcccgaggtgcgctcgatggtggac gtgctggcggaccacgcaggcgagctcgtgcgcaccgacagccccAacttcctctgc tccgtgctgccctcgcactggcgctgcaacaagacgctgcccgtcgccttcaaggtggt gGcattgggggacgtgccggatggtacggtggtgactgtgatggcaggcaatgacga gaactactccgctgaGctgcgcaatgcctcggccgtcatgaagaaccaggtggccag gttcaacgaccttcgcttcgtgggccgcAgtgggcgagggaagagtttcaccctgacc atcactgtgttcaccaaccccacccaagtggcgacctaccAccgagccatcaaggtga ccgtggacggaccccgggagcccagacggcaccggcagaagctggaggaccaGa ccaagccgttccctgaccgctttggggacctggaacggctgcgcatgcgggtgacacc gagcacacccAgcccccgaggctcactcagcaccacaagccacttcagcagccagc cccagaccccaatccaaggcacctcGgaactgaacccattctccgacccccgccagttt gaccgctccttccccacgctgccaaccctcacggagaGccgcttcccagaccccagg atgcattatcccggggccatgtcagctgccttcccctacagcgccacgcccTcgggca cgagcatcagcagcctcagcgtggcgggcatgccggccaccagccgcttccaccata cctacctCccgccaccctacccgggggccccgcagaaccagagcgggcccttccag gccaacccgtcccccctaccaccTctactacgggacatcctctggctcctaccagttctcc atggtggccggcagcagcagtgggggcgaccgctCacctacccgcatgctggcctctt gcaccagcagcgctgcctctgtcgccgccggcaacctcatgaaccccaGcctgggcg gccagagtgatggcgtggaggccgacggcagccacagcaactcacccacggccctg agcacgccaggccgcatggatgaggccgtgtggcggccctac |
| 54 | Full length gene fragment 8E5-2I-BBZ-F2A-huRUN X3 | Caggtgcagctgcaggagagcggccccggcctgatcaagcccagccagaccctgag cctgacctgCaccgtgagcggcggcagcatcagcagcggctacaactggcactggat ccggcagcccccggcaaGggcctggagtggatcggctacatccactacaccggca gcaccaactacaaccccgccctgcggagCcgggtgaccatcagcgtggacaccagc aagaaccagttcagcctgaagctgagcagcgtgaccgcCgccgacaccgccatctact actgcgcccggatctacaacggcaacagcttcccctactggggccaGggcaccaccg tgaccgtgagcagcggtggaggcggttcaggcggaggtggttctggcggtggcggaT cggacatcgtgatgacccagagccccgacagcctggccgtgagcctgggcgagcgg gccaccatcaActgcaagagcagccagagcctgttcaacagcggcaaccagaagaa ctacctgacctggtaccagcaGaagcccggccagccccccaagctgctgatctactgg gccagcacccgggagagcggcgtgcccgacCggttcagcggcagcggcagcggca ccgacttcaccctgaccatcagcagcctgcaggccgaggacgTggccgtgtactactg ccagaacgcctacagcttcccctacaccttcggcggcggcaccaagctggaGatcaag cggaccacgacgccagcgccgcgaccaccaacaccggcgcccaccatcgcgtcgca gcccCtgtccctgcgcccagaggcgtgccggccagcggcgggggcgcagtgcac |
| | | acgagggggctggactTcgcctgtgatatctacatctgggcgcccttggccgggacttg tggggtccttctcctgtcactggtTatcaccctttactgcaaacggggcagaaagaaact cctgtatatattcaaacaaccatttatgagaCcagtacaaactactcaagaggaagatgg ctgtagctgccgatttccagaagaagaagaaggaggatGtgaactgagagtgaagttc agcaggagcgcagacgcccccgcgtaccagcagggccagaaccagctcTataacga gctcaatctaggacgaagagaggagtacgatgttttggacaagagacgtggccgggac cCtgagatgggggggaaagccgcagagaaggaagaaccctcaggaaggcctgtacaa tgaactgcagaaaGataagatggcggaggcctacagtgagattgggatgaaaggcga gcgccggaggggcaaggggcacgaTggcctttaccagggtctcagtacagccacca aggacacctacgacgcccttcacatgcaggccctgccCcctcgcgtgaaacagacttt gaattttgaccttctgaagttggcaggagacgttgagtccaaccctgGgcccatgcgtatt cccgtagacccaagcaccagccgccgcttcacacctccctccccggccttcccctgCg gcggcggcggcggcaagatgggcgagaacagcggcgcgctgagcgcgcaggcgg ccgtggggcccggAgggcgcgcccggcccgaggtgcgctcgatggtggacgtgct ggcggaccacgcaggcgagctcgtgcgcAccgacagccccaacttcctctgctccgt gctgccctcgcactggcgctgcaacaagacgctgcccgtcgcCttcaaggtggtggca ttgggggacgtgccggatggtacggtggtgactgtgatggcaggcaatgacgagAact actccgctgagctgcgcaatgcctcggccgtcatgaagaaccaggtggccaggttcaa cgaccttcgCttcgtgggccgcagtgggcgagggaagagtttcaccctgaccatcact gtgttcaccaaccccacccaagTggcgacctaccaccgagccatcaaggtgaccgtg gacggaccccgggagcccagacggcaccggcagaaGctggaggaccagaccaag ccgttccctgaccgctttggggacctggaacggctgcgcatgcgggtgacaCcgagca cacccagcccccgaggctcactcagcaccacaagccacttcagcagccagccccaga ccccaatCcaaggcacctcggaactgaacccattctccgacccccgccagtttgaccg ctccttccccacgctgccaaCcctcacggagagccgcttcccagaccccaggatgcatt atcccggggccatgtcagctgccttcccctacaGcgccacgccctcgggcacgagcat cagcagcctcagcgtggcgggcatgccggccaccagccgcttccaccAtacctacct cccgccaccctacccgggggccccgcagaaccagagcgggcccttccaggccaacc cgtcccCctaccacctctactacgggacatcctctggctcctaccagttctccatggtggc cggcagcagcagtgggggCgaccgctcacctacccgcatgctggcctcttgcaccag cagcgctgcctctgtcgccgccggcaacctcatgAaccccagcctgggcggccagag tgatggcgtggaggccgacggcagccacagcaactcacccacggccctgagcacgc caggccgcatggatgaggccgtgtggcggccctac |
| 55 | Full length gene fragment 8E5-2I-28Z-F2A-huRUN X3 | Caggtgcagctgcaggagagcggccccggcctgatcaagcccagccagaccctgag cctgacctgcAccgtgagcggcggcagcatcagcagcggctacaactggcactggat ccggcagcccccggcaagGgcctggagtggatcggctacatccactacaccggca gcaccaactacaaccccgccctgcggagcCgggtgaccatcagcgtggacaccagc aagaaccagttcagcctgaagctgagcagcgtgaccgccGccgacaccgccatctact actgcgcccggatctacaacggcaacagcttcccctactggggccagGgcaccaccg tgaccgtgagcagcggtggaggcggttcaggcggaggtggttctggcggtggcggaT cggacatcgtgatgacccagagccccgacagcctggccgtgagcctgggcgagcgg gccaccatcAactgcaagagcagccagagcctgttcaacagcggcaaccagaagaa ctacctgacctggtaccagCagaagcccggccagccccccaagctgctgatctactgg gccagcacccgggagagcggcgtgcccGaccggttcagcggcagcggcagcggca ccgacttcaccctgaccatcagcagcctgcaggccgaggAcgtggccgtgtactactg ccagaacgcctacagcttcccctacaccttcggcggcggcaccaagctGgagatcaag cggaccacgacgccagcgccgcgaccaccaacaccggcgcccaccatcgcgtcgca gCccctgtccctgcgcccagaggcgtgccggccagcggcgggggcgcagtgcac acgagggggctggActtcgcctgtgatttttgggtgctggtggtggttggtggagtcctg gcttgctatagcttgctagTaacagtggcctttattattttctgggtgaggagtaagaggag caggctcctgcacagtgactacatGaacatgactccccgccgccccgggccaacccg |
| | | caagcattaccagccctatgccccaccacgcgacTtcgcagcctatcgctccagagtg aagttcagcaggagcgcagacgcccccgcgtaccagcagggccaGaaccagctctat aacgagctcaatctaggacgaagagaggagtacgatgttttggacaagagacgtggCc gggaccctgagatgggggaaagccgcagagaaggaagaaccctcaggaaggcct gtacaatgaactGcagaaagataagatggcggaggcctacagtgagattgggatgaaa ggcgagcgccggaggggcaagggGcacgatggcctttaccagggtctcagtacagc caccaaggacacctacgacgcccttcacatgcaggcCctgccccctcgcgtgaaaca gactttgaattttgaccttctgaagttggcaggagacgttgagtccaacCctgggcccatg cgtattcccgtagacccaagcaccagccgccgcttcacacctccctccccggccttCcc ctgcggcggcggcggcggcaagatgggcgagaacagcggcgcgctgagcgcgcag gcggccgtggGgcccggagggcgcgcccggcccgaggtgcgctcgatggtggacg tgctggcggaccacgcaggcgagcTcgtgcgcaccgacagccccaacttcctctgct ccgtgctgccctcgcactggcgctgcaacaagacgcTgcccgtcgccttcaaggtggt ggcattgggggacgtgccggatggtacggtggtgactgtgatggcaggCaatgacga gaactactccgctgagctgcgcaatgcctcggccgtcatgaagaaccaggtggccagg ttcAacgaccttcgcttcgtgggccgcagtgggcgagggaagagtttcaccctgaccat cactgtgttcaccaAccccacccaagtggcgacctaccaccgagccatcaaggtgacc gtggacggaccccgggagcccagacggCaccggcagaagctggaggaccagacc aagccgttccctgaccgctttggggacctggaacggctgcgcatgCgggtgacaccga gcacacccagcccccgaggctcactcagcaccacaagccacttcagcagccagcccc agAccccaatccaaggcacctcggaactgaacccattctccgacccccgccagtttga ccgctccttccccacgCtgccaaccctcacggagagccgcttcccagaccccaggatg cattatcccggggccatgtcagctgccttCcccctacagcgccacgccctcgggcacga gcatcagcagcctcagcgtggcgggcatgccggccaccagccGcttccaccatacct acctcccgccaccctacccgggggccccgcagaaccagagcgggcccttccaggcC aacccgtccccctaccacctctactacgggacatcctctggctcctaccagttctccatgg tggccggcAgcagcagtgggggcgaccgctcacctacccgcatgctggcctcttgca ccagcagcgctgcctctgtcGccgccggcaacctcatgaaccccagcctgggcggcc agagtgatggcgtggaggccgacggcagccacagcaactcacccacggccctgagc acgccaggccgcatggatgaggccgtgtggcggccctac |
| 56 | Full length gene fragment 8E5-2I-28BB Z-F2A-huRU NX3 | Caggtgcagctgcaggagagcggccccggcctgatcaagcccagccagaccctgag cctgacctgcAccgtgagcggcggcagcatcagcagcggctacaactggcactggat ccggcagcccccggcaagGgcctggagtggatcggctacatccactacaccggca gcaccaactacaaccccgccctgcggagcCgggtgaccatcagcgtggacaccagc aagaaccagttcagcctgaagctgagcagcgtgaccgccGccgacaccgccatctact actgcgcccggatctacaacggcaacagcttcccctactggggccaGggcaccaccg tgaccgtgagcagcggtggaggcggttcaggcggaggtggttctggcggtggcggAt cggacatcgtgatgacccagagccccgacagcctggccgtgagcctgggcgagcgg gccaccatCaactgcaagagcagccagagcctgttcaacagcggcaaccagaagaac tacctgacctggtaccAgcagaagcccggccagcccccaagctgctgatctactggg ccagcacccgggagagcggcgtgCccgaccggttcagcggcagcggcagcggcac cgacttcaccctgaccatcagcagcctgcaggcCgaggacgtggccgtgtactactgc cagaacgcctacagcttcccctacaccttcggcggcggcaCcaagctggagatcaagc ggaccacgacgccagcgccgcgaccaccaacaccggcgcccaccatcgCgtcgca gcccctgtccctgcgcccagaggcgtgccggccagcggcgggggcgcagtgcaca cgagGgggctggacttcgcctgtgatttttgggtgctggtggtggttggtggagtcctgg cttgctatagcTtgctagtaacagtggcctttattattttctgggtgaggagtaagaggagc aggctcctgcacagtgActacatgaacatgactccccgccgccccgggccaacccgc aagcattaccagccctatgccccaccAcgcgacttcgcagcctatcgctccaaacggg gcagaaagaaactcctgtatatattcaaacaaccaTttatgagaccagtacaaactactc aagaggaagatggctgtagctgccgatttccagaagaagaagAaggaggatgtgaac |
| | | tgagagtgaagttcagcaggagcgcagacgcccccgcgtaccagcagggccaGaac cagctctataacgagctcaatctaggacgaagagaggagtacgatgttttggacaagag acgtGgccgggaccctgagatgggggaaagccgcagagaaggaagaaccctcag gaaggcctgtacaatgAactgcagaaagataagatggcggaggcctacagtgagattg ggatgaaaggcgagcgccggaggggCaaggggcacgatggcctttaccagggtctc agtacagccaccaaggacacctacgacgcccttcaCatgcaggccctgccccctcgc gtgaaacagactttgaattttgaccttctgaagttggcaggagacgTtgagtccaaccct gggcccatgcgtattcccgtagacccaagcaccagccgccgcttcacacctccctcCc cggccttcccctgcggcggcggcggcaagatgggcgagaacagcggcgcgctg agcgcgcaggcggCcgtggggcccggagggcgcgcccggcccgaggtgcgctcg atggtggacgtgctggcggaccacgcagGcgagctcgtgcgcaccgacagccccaa cttcctctgctccgtgctgccctcgcactggcgctgcaacaAgacgctgcccgtcgcctt caaggtggtggcattgggggacgtgccggatggtacggtggtgactgtgaTggcagg caatgacgagaactactccgctgagctgcgcaatgcctcggccgtcatgaagaaccag gtgGccaggttcaacgaccttcgcttcgtgggccgcagtgggcgagggaagagtttca ccctgaccatcactGtgttcaccaaccccacccaagtggcgacctaccaccgagccatc aaggtgaccgtggacggaccccggGagcccagacggcaccggcagaagctggagg accagaccaagccgttccctgaccgctttggggacctGgaacggctgcgcatgcgggt gacaccgagcacacccagcccccgaggctcactcagcaccacaagccActtcagca gccagccccagaccccaatccaaggcacctcggaactgaacccattctccgacccccg cCagtttgaccgctccttccccacgctgccaaccctcacggagagccgcttcccagacc ccaggatgcatTatcccggggccatgtcagctgccttcccctacagcgccacgccctc gggcacgagcatcagcagcctcAgcgtggcgggcatgccggccaccagccgcttcc accatacctacctcccgccaccctacccgggggcCccgcagaaccagagcgggccct tccaggccaacccgtcccccctaccacctctactacgggacatcctcTggctcctaccag ttctccatggtggccggcagcagcagtgggggcgaccgctcacctacccgcatgctgG cctcttgcaccagcagcgctgcctctgtcgccgccggcaacctcatgaaccccagcctg ggcggccagaGtgatggcgtggaggccgacggcagccacagcaactcacccacgg ccctgagcacgccaggccgcatggatgaggccgtgtggcggccctac |
| 57 | 9F2-28Z | Evqlvqsgaevkkpgasvkvsckasgytfsdyemhwvrqapgqglewmgaihp gsgdtaynqrfkgrvtitadKststaymelsslrsedtavyycarfysyaywgqgtlvt vsaggggsggggsggggsdivmtqtplslpvtpgEpasiscrssqslvhsngntylq wylqkpgqspqlliykvsnrfsgvpdrfsgsgsgtdftlkisrveaedvgVyycsqsi yvpytfgqgtkleikrtttpaprpptpaptiasqplslrpeacrpaaggavhtrgldfacd fwvlVvvggvlacysllvtvafiifwvrskrsrllhsdymnmtprrpgptrkhyqpy apprdfaayrsrvkfsrsadApayqqgqnqlynelnlgrreeydvldkrrgrdpemg gkpqrrknpqeglynelqkdkmaeayseigmkgerrrgkghdglyqglstatkdty dalhmqalppr |
| 58 | 9F2-BBZ | EVQLVQSGAEVKKPGASVKVSCKASGYTFSDYEMHWV RQAPGQGLEWMGAIHPGSGDTAYNQRFKGRVTITADK STSTAYMELSSLRSEDTAVYYCARFYSYAYWGQGTLVT VSAGGGGSGGGGSGGGGSDIVMTQTPLSLPVTPGEPASI SCRSSQSLVHSNGNTYLQWYLQKPGQSPQLLIYKVSNR FSGVPDRFSGSGSGTDFTLKISRVEAEDVGVYYCSQSIY VPYTFGQGTKLEIKRTTTPAPRPPTPAPTIASQPLSLRPEA CRPAAGGAVHTRGLDFACDIYIWAPLAGTCGVLLLSLVI TLYCKRGRKKLLYIFKQPFMRPVQTTQEEDGCSCRFPEE EEGGCELRVKFSRSADAPAYQQGQNQLYNELNLGRREE YDVLDKRRGRDPEMGGKPQRRKNPQEGLYNELQKDK MAEAYSEIGMKGERRRGKGHDGLYQGLSTATKDTYDA |
| | | LHMQALPPR |
| 59 | 9F2-28BBZ | Evqlvqsgaevkkpgasvkvsckasgytfsdyemhwvrqapgqglewmgaihp gsgdtaynqrfkgrvTitadkststaymelsslrsedtavyycarfysyaywgqgtlvt vsaggggsggggsggggsdivmtqtPlslpvtpgepasiscrssqslvhsngntylq wylqkpgqspqlliykvsnrfsgvpdrfsgsgsgtdfTlkisrveaedvgvyycsqsi yvpytfgqgtkleikrtttpaprpptpaptiasqplslrpeacrpaagGavhtrgldfac dfwvlvvvggvlacysllvtvafiifwvrskrsrllhsdymnmtprrpgptrkhyqpy Apprdfaayrskrgrkkllyifkqpfmrpvqttqeedgcscrfpeeeeggcelrvkfs rsadapayqqgqNqlynelnlgrreeydvldkrrgrdpemggkpqrrknpqegly nelqkdkmaeayseigmkgerrrgkghdglyqglstatkdtydalhmqalppr |
| 60 | 8E5-2I-28Z | QVQLQESGPGLIKPSQTLSLTCTVSGGSISSGYNWHWIR QPPGKGLEWIGYIHYTGSTNYNPALRSRVTISVDTSKNQ FSLKLSSVTAADTAIYYCARIYNGNSFPYWGQGTTVTVS SGGGGSGGGGSGGGGSDIVMTQSPDSLAVSLGERATIN CKSSQSLFNSGNQKNYLTWYQQKPGQPPKLLIYWASTR ESGVPDRFSGSGSGTDFTLTISSLQAEDVAVYYCQNAYS FPYTFGGGTKLEIKRTTTPAPRPPTPAPTIASQPLSLRPEA CRPAAGGAVHTRGLDFACDFWVLVVVGGVLACYSLLV TVAFIIFWVRSKRSRLLHSDYMNMTPRRPGPTRKHYQP YAPPRDFAAYRSRVKFSRSADAPAYQQGQNQLYNELNL GRREEYDVLDKRRGRDPEMGGKPQRRKNPQEGLYNEL QKDKMAEAYSEIGMKGERRRGKGHDGLYQGLSTATKD TYDALHMQALPPR |
| 61 | 8E5-2I-BBZ | QVQLQESGPGLIKPSQTLSLTCTVSGGSISSGYNWHWIR QPPGKGLEWIGYIHYTGSTNYNPALRSRVTISVDTSKNQ FSLKLSSVTAADTAIYYCARIYNGNSFPYWGQGTTVTVS SGGGGSGGGGSGGGGSDIVMTQSPDSLAVSLGERATIN CKSSQSLFNSGNQKNYLTWYQQKPGQPPKLLIYWASTR ESGVPDRFSGSGSGTDFTLTISSLQAEDVAVYYCQNAYS FPYTFGGGTKLEIKRTTTPAPRPPTPAPTIASQPLSLRPEA CRPAAGGAVHTRGLDFACDIYIWAPLAGTCGVLLLSLVI TLYCKRGRKKLLYIFKQPFMRPVQTTQEEDGCSCRFPEE EEGGCELRVKFSRSADAPAYQQGQNQLYNELNLGRREE YDVLDKRRGRDPEMGGKPQRRKNPQEGLYNELQKDK MAEAYSEIGMKGERRRGKGHDGLYQGLSTATKDTYDA LHMQALPPR |
| 62 | 8E5-2I-28BB Z | QVQLQESGPGLIKPSQTLSLTCTVSGGSISSGYNWHWIR QPPGKGLEWIGYIHYTGSTNYNPALRSRVTISVDTSKNQ FSLKLSSVTAADTAIYYCARIYNGNSFPYWGQGTTVTVS SGGGGSGGGGSGGGGSDIVMTQSPDSLAVSLGERATIN CKSSQSLFNSGNQKNYLTWYQQKPGQPPKLLIYWASTR ESGVPDRFSGSGSGTDFTLTISSLQAEDVAVYYCQNAYS FPYTFGGGTKLEIKRTTTPAPRPPTPAPTIASQPLSLRPEA CRPAAGGAVHTRGLDFACDFWVLVVVGGVLACYSLLV TVAFIIFWVRSKRSRLLHSDYMNMTPRRPGPTRKHYQP YAPPRDFAAYRSKRGRKKLLYIFKQPFMRPVQTTQEED |
| | | GCSCRFPEEEEGGCELRVKFSRSADAPAYQQGQNQLYN ELNLGRREEYDVLDKRRGRDPEMGGKPQRRKNPQEGL YNELQKDKMAEAYSEIGMKGERRRGKGHDGLYQGLST ATKDTYDALHMQALPPR |

## Claims

1. A genetically engineered cell, wherein the cell expresses an exogenous receptor binding to an antigen and expresses an increased level of RUNX3 or exogenous RUNX3.

2. The cell of claim 1, wherein the cell is an immune effector cell.

3. The cell of claim 2, wherein the immune effector cell is a T cell.

4. The cell of claim 1, wherein the RUNX3 is a full-length human RUNX3 or a fragment of human RUNX3 having the same function as the full-length human RUNX3.

5. The cell of claim 4, wherein the RUNX3 is at least 90% identical to the sequence as shown in SEQ ID NO: 20.

6. The cell of claim 1, wherein the RUNX3 is constitutively expressed.

7. The cell of claim 1, wherein the RUNX3 is inducibly expressed.

8. The cell of claim 1, wherein the antigen is a tumor antigen or a pathogen antigen, preferably a tumor antigen.

9. The cell of claim 8, wherein the tumor antigen is a solid tumor antigen.

10. The cell of claim 8, wherein the tumor antigen is GPC3 or claudin 18.2.

11. The cell of any one of claims 1-10, wherein the receptor is a chimeric receptor selected from the group consisting of chimeric antigen receptor (CAR), modified T cell (antigen) receptor (TCR), T cell fusion protein (TFP), T Cell antigen coupler (TAC) or a combination thereof.

12. The cell of claim 11, wherein the receptor is a chimeric antigen receptor.

13. The cell of claim 12, wherein the intracellular domain of the chimeric antigen receptor comprises the intracellular costimulatory signaling domain of CD137.

14. The cell of any one of claims 1-13, wherein the extracellular domain of the receptor has an amino acid sequence that is at least 80%, 85%, 90%, 95%, 96%, 97%, 98%, or 99% identical to SEQ ID NO: 42 or SEQ ID NO: 22.

15. The cell of any one of claims 1-14, wherein the intracellular domain of the receptor contains has an amino acid sequence that is at least 80%, 85%, 90%, 95%, 96%, 97%, 98%, or 99% identical to SEQ ID NO: 47.

16. The cell of claim 15, wherein the intracellular domain of the receptor also has an amino acid sequence that is at least 90% identical to SEQ ID NO: 46 or SEQ ID NO: 49, or comprises an amino acid sequence that is at least 90% identical to SEQ ID NO: 46 or SEQ ID NO: 49.

17. The cell of claim 11, wherein the cell comprises a nucleic acid sequence that is at least 80%, 85%, 90%, 95%, 96%, 97%, 98%, or 99% identical to SEQ ID NO: 17, 19, 53, 54, 55, or 56.

18. The cell of any one of claims 1-17, wherein the cell also expresses an exogenous cytokine receptor-binding protein, or an exogenous cytokine or a polypeptide thereof.

19. The cell of claim 18, wherein the cell also expresses an exogenous cytokine.

20. The cell of claim 18, wherein the exogenous cytokine receptor-binding protein can specifically bind to the corresponding cytokine receptor and enhance activities of the receptor.

21. The cell of claim 18, wherein the exogenous cytokine or polypeptide thereof can specifically bind to the corresponding cytokine receptor and enhance activities of the receptor.

22. The cell of claim 19, wherein the cytokine has an amino acid sequence that is at least 80%, 85%, 90%, 95%, 96%, 97%, 98%, or 99% identical to the sequence as shown in SEQ ID NO: 39, 41 or 36.

23. The cell of claim 1, wherein the expression level of RUNX3 is increased by at least 10%, 20%, 30%, 40%, 50%, 60%, 70%, 80%, 90%, or 100% as compared with the wild type.

24. The cell of claim 1, wherein the increase in the expression level of the RUNX3 as compared with the wild type is sufficient to increase the retention of the cell in non-lymphatic sites.

25. The cell of claim 19, wherein the cytokine is constitutively expressed.

26. The cell of claim 19, wherein the cytokine is inducibly expressed.

27. The cell of any one of claims 1-26, wherein the cell expresses an exogenous RUNX3.

28. The cell of claim 27, wherein the receptor and/or RUNX3 are expressed by using a viral vector; and preferably, the viral vector includes: a lentiviral vector, retroviral vector or adenoviral vector.

29. An expression construct, wherein the expression construct comprises sequentially connected expression cassette 1 of an antigen-binding receptor and expression cassette 2 of RUNX3, wherein the expression cassettes are optionally connected by tandem fragments selected from the group consisting of F2A, PA2, T2A, and E2A.

30. The experssion construct of claim 29, wherein the expression cassette of the antigen-binding receptor comprises a nucleic acid sequence encoding the sequence as shown in SEQ ID NO: 57, 58, 59, 60, 61, or 62, and the expression cassette of RUNX3 has a nucleic acid sequence encoding the sequence as shown in SEQ ID NO: 20.

31. The experssion construct of claim 29, wherein the expression construct further comprises expression cassette 3, the expression cassette 3 comprises a nucleic acid sequence encoding a cytokine, and the sequence of the cytokine is an amino acid sequence which is at least 80%, 85%, 90%, 95%, 96%, 97%, 98%, or 99% identical to the sequence as shown in SEQ ID NO: 36, 39, or 41.

32. An expression vector, comprising the expression construct of any one of claims 29-31.

33. A virus, comprising the expression vector of claim 32.

34. A method for increasing the viability of an immune effector cell expressing a chimeric receptor in an individual by increasing the expression level of RUNX3 in the immune effector cell.

35. A method for increasing the viability of an immune effector cell expressing a chimeric receptor in an individual, wherein the expression level of RUNX3 in the immunity effector cell is increased by expressing exogenous RUNX3 in the immune effector cell.

36. The method of claim 34 or 35, wherein the RUNX3 is a full-length human RUNX3 or a fragment of human RUNX3 having the same function as the full-length human RUNX3.

37. The method of claim 36, wherein the RUNX3 has at least 90% identity with the sequence as shown in SEQ ID NO: 20.

38. The method of claim 34, wherein the RUNX3 is constitutively expressed.

39. The method of claim 34, wherein the RUNX3 is inducibly expressed.

40. The method of claim 34 or 35, wherein the immune effector cell also co-expresses a cytokine

41. The method of claim 34 or 35, wherein the individual is also administered with a cytokine.

42. The method of any one of claims 34-41, wherein the immune effector cell is a T cell.

43. The method of claim 42, wherein the chimeric receptor is a chimeric antigen receptor.

44. The method of claim 34 or 35, wherein the individual is also administered with a chemotherapeutic drug.

45. Use of the cell of any one of claims 1-27, expression construct of any one of claims 29-31, or virus of claim 33 for preparing a medicament for inhibiting a tumor or inhibiting a pathogen.

46. A pharmaceutical composition for inhibiting a tumor or inhibiting a pathogen, wherein the pharmaceutical composition comprises the cell of any one of claims 1-27 and a pharmaceutically acceptable carrier or excipient.

47. A kit for treating a tumor or pathogen infection, wherein the kit comprises the cell of any one of claims 1-27 or pharmaceutical composition of claim 46.

48. Use of a immune effector cell and a chemotherapeutic drug for preparing a medicament for treating a tumor, wherein the immune effector cell expresses an increased level of RUNX3 or exogenous RUNX3 and a chimeric antigen receptor targeting a tumor antigen.

49. The use of claim 48, wherein the chemotherapeutic agent includes sorafenib.

50. The use of claim 47, wherein the tumor antigen is GPC3 or claudin 18.2.

51. The use of claim 47 or 48, wherein the immune effector cell is a T cell.

52. A method for treating a tumor in a patient in need thereof, comprising providing the patient with the cell of any one of claims 1-27.
